# EUROPEAN PATENT APPLICATION

(11) **EP 4 446 427 A1**
(43) Date of publication of application: **16.10.2024**
(21) Application number: 23187623.6
(22) Date of filing: 25.07.2023
(51) Int. Cl.: C12Q 1/6806, C12Q 1/6853

(54) **DNA AMPLIFICATION METHODS**

(30) Priority: 14.04.2023 US 202363496277 P
(71) Applicant: Quantoom Biosciences S.A., 1400 Nivelles (BE)
(72) Inventor: RANDRIANJATOVO-GBALOU, Irina, 91058 Evry-Courcouronnes (FR); SAID, Ahmed, 91058 Evry-Courcouronnes (FR); RAHIER, Renaud, 91058 Evry-Courcouronnes (FR)
(74) Representative: Icosa Europe

(57) **Abstract**

The present invention relates to methods for amplification of DNA comprising ColE1-like origin of replication, comprising using at least one bacterial RNA polymerase and at least one DNA polymerase, optionally in the form of a replisome.

## Description

### FIELD OF INVENTION

The present invention relates to methods for amplification of DNA.

### BACKGROUND OF INVENTION

Isothermal amplification of DNA is a way to replicate a DNA template at a fixed temperature, preferentially between 25°C and 40°C. However, achieving this kind of amplification is not straightforward and requires a method to separate DNA strands during replication.

Another challenge of isothermal amplification processes arises from the initiation of the replication. Indeed, replicative DNA polymerases require the presence of a priming site, which correspond to a nucleic acid duplex harboring a free 3'OH terminus. The priming site must be suitable for DNA polymerase extension.

In contrast to PCR process, in which priming is obtained through the successive denaturation of dsDNA and primer annealing, isothermal processes require the addition of enzymes to create a priming site. Thus, among all steps required for isothermal amplification of DNA, the initiation of replication is thought to be the most critical.

Several prior arts describe the use of primers, recombinases, rep proteins (dnaA), primases or non-specific RNA polymerases for this step.

The inventors herein provide an improved solution for DNA amplification, in particular isothermal DNA amplification, enabling amplification of most DNA molecules, in particular commercially used DNA plasmids, that commonly comprise ColE1-like replication origin (Wang et al., Plasmid vol. 51,3 (2004): 149-61).

### SUMMARY

The present invention relates to a method for amplifying a DNA molecule, comprising contacting the DNA molecule with a first composition comprising at least one bacterial RNA polymerase (RNAP) or a nucleic acid encoding thereof, and a second composition comprising a strand displacement DNA polymerase or a nucleic acid encoding thereof, wherein the DNA molecule comprises at least one ColE1-like origin of replication and wherein the at least one bacterial RNAP is configured to bind the at least one origin of replication.

In some embodiments, the strand displacement DNA polymerase comprises a Phi29 DNA polymerase, and wherein optionally the Phi29 DNA polymerase has at least 75% sequence identity with SEQ ID NO: 6.

The present invention further relates to a method for amplifying a DNA molecule, comprising contacting the DNA molecule with a first composition comprising at least one bacterial RNA polymerase (RNAP) or a nucleic acid encoding thereof, and a second composition comprising at least one non-native replisome with respect to the at least one bacterial RNAP or nucleic acids encoding thereof, wherein the DNA molecule comprises at least one ColE1-like origin of replication and wherein the at least one bacterial RNAP is configured to bind the at least one origin of replication.

In some embodiments, the first composition further comprises one or more proteins selected from the group comprising DNA polymerase I having at least 75% sequence identity with SEQ ID NO:16, E. coli Single-stranded DNA-binding protein having at least 75% sequence identity with SEQ ID NO: 39, E. coli RNase H having at least 75% sequence identity with SEQ ID NO: 56, and variants thereof, or nucleic acids encoding thereof.

In some embodiments, the non-native replisome is a phage T4-based replisome, a phage T3-based replisome, or a phage T7-based replisome.

In some embodiments, the non-native replisome is a phage T4-based replisome comprising GP43 DNA polymerase having at least 75% sequence identity with SEQ ID NO: 19, and optionally one or more of the proteins selected from the group comprising gp41 Helicase having at least 75% sequence identity with SEQ ID NO: 25, gp59 Helicase loader having at least 75% sequence identity with SEQ ID NO: 26, E. coli DNA Gyrase, GP32 SSB having at least 75% sequence identity with SEQ ID NO: 40, gp61 Primase having at least 75% sequence identity with SEQ ID NO: 35, gp45 clamp having at least 75% sequence identity with SEQ ID NO: 36, gp44/62 Clamp loader having at least 75% sequence identity with SEQ ID NO: 37 or SEQ ID NO: 38, and variants thereof, or nucleic acids encoding thereof.

In some embodiments, the non-native replisome is a phage T3-based replisome comprising GP5 DNA polymerase having at least 75% sequence identity with SEQ ID NO: 20, Thioredoxin 1 having at least 75% sequence identity with SEQ ID NO: 22, and optionally one or more of the proteins selected from the group comprising phage T3 DNA helicase/primase having at least 75% sequence identity with SEQ ID NO: 28, E. coli DNA Gyrase, and phage T3 Single-stranded DNA-binding protein having at least 75% sequence identity with SEQ ID NO: 42, and variants thereof, or nucleic acids encoding thereof.

In some embodiments, the non-native replisome is a phage T7-based replisome comprising GP5 DNA polymerase having at least 75% sequence identity with SEQ ID NO: 21, Thioredoxin 1 having at least 75% sequence identity with SEQ ID NO: 22, and optionally one or more of the proteins selected from the group comprising phage T7 DNA helicase/primase having at least 75% sequence identity with SEQ ID NO: 27, E. coli DNA Gyrase, and phage T7 Single-stranded DNA-binding protein having at least 75% sequence identity with SEQ ID NO: 41, and variants thereof, or nucleic acids encoding thereof.

In some embodiments, the DNA molecule is selected from the group comprising plasmid DNA (pDNA), linear DNA, minicircle DNA, and linear covalently closed DNA (LCC DNA), optionally wherein the DNA molecule is a pDNA or linear DNA.

In some embodiments, the amplification is initiated by the binding of the at least one RNAP to the at least one ColE1-like origin of replication.

In some embodiments, the at least one bacterial RNA polymerase is Escherichia coli RNA polymerase holoenzyme.

In some embodiments, the amplification is isothermal.

The present invention further relates to a composition comprising a phage T4-based replisome comprising GP43 DNA Polymerase, and one or more of the proteins selected from the group comprising or consisting of gp41 Helicase, gp59 Helicase loader, E. coli DNA Gyrase, GP32 SSB, gp61 Primase, gp45 clamp, gp44/62 Clamp loader, phage T4 RNaseH, phage T4 Ligase, E. coli DNA topoisomerase III, E. coli DNA topoisomerase IV, E. coli Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, E. coli DNA topoisomerase I, E. coli RpoE, E. coli RpoH, E. coli RpoS, E. coli ihfA, E. coli ihfB, E. coli diaA, E. coli RecA, E. coli RecBCD, E. coli RecQ, E. coli RecF, E. coli uvrD, and E. coli HelD, or nucleic acids encoding thereof.

The present invention further relates to a composition comprising a phage T3-based replisome comprising T3 GP5 and Thioredoxin 1, and one or more of the proteins selected from the group comprising or consisting of phage T3 DNA helicase/primase, E. coli DNA Gyrase, phage T3 Single-stranded DNA-binding protein, phage T3 DNA ligase, E. coli DNA topoisomerase III, E. coli DNA topoisomerase IV, T3 endonuclease, T3 exonuclease, E. coli DNA ligase, E. coli DNA topoisomerase I, E. coli RpoE, E. coli RpoH, E. coli RpoS, E. coli ihfA, E. coli ihfB, E. coli diaA, E. coli RecA, E. coli RecBCD, E. coli RecQ, E. coli RecF, E. coli uvrD, and E. coli HelD, or nucleic acids encoding thereof.

The present invention further relates to a composition comprising a phage T7-based replisome comprising T7 GP5 and Thioredoxin 1, and one or more of the proteins selected from the group comprising or consisting of phage T7 DNA helicase/primase, E. coli DNA Gyrase, phage T7 Single-stranded DNA-binding protein, phage T7 DNA ligase, E. coli DNA topoisomerase III, E. coli DNA topoisomerase IV, T7 endonuclease, T7 exonuclease, E. coli DNA ligase, E. coli DNA topoisomerase I, E. coli RpoE, E. coli RpoH, E. coli RpoS, E. coli ihfA, E. coli ihfB, E. coli diaA, E. coli RecA, E. coli RecBCD, E. coli RecQ, E. coli RecF, E. coli uvrD, and E. coli HelD, or nucleic acids encoding thereof.

### DEFINITIONS

In the present invention, the following terms have the following meanings:

**"About",** when preceding a figure, means plus or less 10% of the value of said figure.

**"And/Or"** refers to and encompasses any and all possible combinations of one or more of the associated listed items, as well as the lack of combinations when interpreted in the alternative ("**or**").

**"At least one"** includes 1, 2, 3, 4, 5, 6, 7, 8, 9, 10, 25, 50, 75, 100, 250, 500, 750, 10³,10⁴, 10⁵, 10⁶, 10⁷, 10⁸, 10⁹, 10¹⁰, 10¹¹, 10¹², 10¹³, 10¹⁴, 10¹⁵ or more.

**"Comprising", "comprises"** and **"comprised of"** are used herein are synonymous with **"including", "includes"** or **"containing", "contains",** and are inclusive or open-ended and do not exclude additional, non-recited members, elements or method steps. These terms also encompass **"consisting of".**

**"Peptide", "Polypeptide"** or **"Protein'** refers interchangeably to any peptide or polypeptide or protein comprising two or more amino acids joined to each other by peptide bonds or modified peptide bonds, *i.e*., peptide isosteres, thereby forming polymers of amino acids of any length. The polymer may be linear or branched, it may comprise modified amino acids, and it may be interrupted by non-amino acids. The terms also encompass an amino acid polymer that has been modified by, for example, disulfide bond formation, glycosylation, lipidation, acetylation, phosphorylation, pegylation, or any other manipulation, such as conjugation with a labelling component. As used herein the term **"amino acid"** includes natural and/or unnatural or synthetic amino acids, including glycine and both the D or L optical isomers, and amino acid analogs and peptidomimetics.

**"Variant"** refers a peptide, polypeptide or protein that typically differs from a peptide, polypeptide or protein specifically disclosed herein in one or more mutation, typically comprising substitutions, deletions, additions and/or insertions. In some embodiments, one or more mutation means 1, 2, 3, 4, 5, 6, 7, 8, 9, 10 or more mutations. Such variants may be naturally occurring or may be synthetically generated, for example, by modifying one or more of the peptide, polypeptide or protein sequences and evaluating one or more biological activities of the peptide, polypeptide or protein and/or using any of a number of techniques well known in the art. Modifications may be made in the structure of the peptide, polypeptide or protein and still obtain a functional molecule with desirable characteristics. When it is desired to alter the amino acid sequence of a peptide, polypeptide or protein to create an equivalent, or even an improved, variant or portion of a peptide, polypeptide or protein, one skilled in the art will typically change one or more of the codons of the encoding DNA sequence. It is thus contemplated that various changes may be made in the peptide, polypeptide or protein sequences, or corresponding DNA sequences that encode said peptide, polypeptide or protein, without appreciable loss of their biological utility or activity. In many instances, a peptide, polypeptide or protein variant will contain one or more conservative substitutions. A variant may also, or alternatively, contain nonconservative changes.

### DETAILED DESCRIPTION

The present invention relates to a method for amplifying a DNA molecule, comprising contacting the DNA molecule with a first composition comprising at least one bacterial RNA polymerase, or a nucleic acid encoding thereof, and a second composition comprising at least one DNA polymerase, or a nucleic acid encoding thereof.

In some embodiments, the DNA molecule comprises at least one origin of replication. In some embodiments, the at least one origin of replication is selected from the group comprising or consisting of pUC, pBR322, pET, pGEX, pColE1, ColE1, pACYC, pBluescript, pGEM, pMB1, p15A, F1, pR6K, R6K, pSC101, and the like.

In some embodiments, the at least one origin of replication comprises or consists of a ColE1-like sequence. In a preferred embodiment, the at least one origin of replication comprises or consists of a ColE1-like origin of replication.

In a preferred embodiment, the at least one origin of replication is a ColE1-like origin of replication.

Thus, in a preferred embodiment, the DNA molecule comprises at least one ColE1-like origin of replication. In certain embodiments, the DNA molecule comprises a ColE1-like origin of replication.

In some embodiments, the at least one ColE1-like origin of replication is selected from the group comprising or consisting of pMB1, p15A, pBR322, and ColE1 derivatives.

Typically, ColE1-like origins of replication are found in plasmids such as pUC, pBR322, pET, pGEX, pColE1, pACYC, pBluescript, pGEM.

In some embodiments, the at least one ColE1-like origin of replication is selected from the group comprising or consisting of pUC, pBR322, pET, pGEX, pColE1, ColE1, pACYC, pBluescript, pGEM, pMB1, p15A, and F1.

In some embodiments, the at least one ColE1-like origin of replication is selected from the group comprising or consisting of pUC, pBR322, pET, pGEX, pColE1, pACYC, pBluescript, and pGEM. In some embodiments, the at least one ColE1-like origin of replication is pUC. In some embodiments, the at least one ColE1-like origin of replication is pBR322. In some embodiments, the at least one ColE1-like origin of replication is pET. In some embodiments, the at least one ColE1-like origin of replication is pGEX. In some embodiments, the at least one ColE1-like origin of replication is pColE1. In some embodiments, the at least one ColE1-like origin of replication is pACYC. In some embodiments, the at least one ColE1-like origin of replication is pBluescript. In some embodiments, the at least one ColE1-like origin of replication is pBRGEM.

In some embodiments, the at least one ColE1-like origin of replication is selected from the group comprising or consisting of pMB1, pBR322, ColE1, p15A, and F1. In some embodiments, the at least one ColE1-like origin of replication is pMB1. In some embodiments, the at least one ColE1-like origin of replication is pBR322. In some embodiments, the at least one ColE1-like origin of replication is ColE1. In some embodiments, the at least one ColE1-like origin of replication is p15A. In some embodiments, the at least one ColE1-like origin of replication is F1.

In some embodiments, the at least one origin of replication may be a derivative of the at least one origin of replication.

In some embodiments, the DNA molecule is selected from the group comprising plasmid DNA (pDNA), linear DNA, minicircle DNA and linear covalently closed DNA (LCC DNA), preferably the DNA molecule is a pDNA or linear DNA, more preferably the DNA molecule is a pDNA. In some embodiment, the pDNA is comprised in, or consists of, a plasmid, preferably the plasmid is selected from the group comprising or consisting of pUC, pBR322, pET, pGEX, pColE1, pACYC, pBluescript, and pGEM.

In some embodiments, the DNA molecule does not comprise an origin of replication that can bind to an enzyme having DnaA activity. In some embodiments, the DNA molecule does not comprise an oriC origin of replication.

In some embodiments, the origin of replication cannot bind to an enzyme having DnaA activity. In some embodiments, the origin of replication is not oriC origin of replication.

In some embodiments, the method according to the invention requires the use of a promoter (*i.e.,* a promoter sequence). In some embodiment, the promoter is the origin of replication as defined herein. In some embodiments, the method according to the invention is not a promoter-free method of DNA amplification.

In some embodiments, the DNA molecule is a template DNA molecule (*i.e.,* a template molecule for DNA replication).

In some embodiments, the DNA molecule is single stranded or double-stranded. In some embodiments, the DNA molecule comprises or consists or natural nucleotides, non-natural nucleotides (*i.e*., chemically modified nucleotides), or combination thereof. In some embodiments, the DNA molecule comprises or consists or natural nucleotides.

In some embodiments, the method for amplifying a DNA molecule according to the present invention comprises at least one step of amplifying the DNA molecule by a technique known in the art. Non-limitative examples of techniques for amplifying DNA include Polymerase Chain Reaction (PCR), Loop Mediated Isothermal Amplification (LAMP), Nucleic Acid Sequence Based Amplification (NASBA, or 3SR), Strand Displacement Amplification (SDA), Multiple displacement amplification (MDA), Rolling Circle Amplification (RCA), Theta amplification, Ligase Chain Reaction (LCR), Helicase dependent amplification (HDA), Ramification amplification method (RAM), and the like.

In a preferred embodiment, the at least one step of amplifying the DNA comprises at least one isothermal DNA amplification step. In a preferred embodiment, all DNA amplifications steps of the method according to the invention are isothermal DNA amplification step(s). In some embodiments, the method according to the invention is performed at a temperature from 15°C to 80°C, preferably from 20°C to 60°C, more preferably from 25°C to 40°C.

In some embodiments, the method for amplifying a DNA molecule according to the present invention comprises or consists of at least one step of Rolling Circle Amplification (RCA) and/or at least one step of Theta amplification (*i.e*., θ amplification).

In some embodiments, the method for amplifying a DNA molecule according to the present invention comprises or consists of at least one step of RCA. In some embodiments, RCA comprises of consists of the steps of, in a defined order, (i) initiation, (ii) strand displacement, and optionally (iii) concatemer processing. In some embodiments, steps (i), (ii) and (iii) are repeated any number of times (*i.e*., 1, 2, 3, 4, 5, 10, 50, 100 times or more), together or independently from each other. In one embodiment, steps (i), (ii) and (iii) are performed continuingly. In another embodiment, steps (i), (ii) and (iii) are separated by pauses or stopping points of any duration (*i.e.*, 10 seconds, 1 minute, 1 hour or more).

In some embodiments, the method for amplifying a DNA molecule according to the present invention comprises or consists of at least one step of Theta amplification. In some embodiments, Theta amplification comprises of consists of the steps of, in a defined order, (i) initiation, (ii) fork progression, (iii) Okazaki maturation, and optionally (iv) decatenation. In some embodiments, steps (i), (ii), (iii) and (iv) are repeated any number of times (*i.e*., 1, 2, 3, 4, 5, 10, 50, 100 times or more), together or independently from each other. In one embodiment, steps (i), (ii), (iii) and (iv) are performed continuingly. In another embodiment, steps (i), (ii), (iii) and (iv) are separated by pauses or stopping points of any duration (*i.e*., 10 seconds, 1 minute, 1 hour or more).

In some embodiments, the method for amplifying a DNA molecule according to the present invention is Theta amplification and/or RCA.

In some embodiments, the method according to the invention is performed *in vitro* and/or *ex-vivo,* preferably *in vitro.*

In some embodiments, the method according to the invention is primer-free.

In some embodiments, the method according to the invention is free of use of antibiotics and/or endotoxins.

In some embodiments, the method according to the invention enables a linear or exponential amplification.

In some embodiments, the method according to the invention enables a 10-fold, 100-fold, 1 000-fold, 10 000-fold, 100 000-fold amplification, or more, of the DNA molecule.

DNA amplification can be measured by any method known in the art. In some embodiments, the method further comprises quantifying, measuring or otherwise assessing the amplified DNA.

The inventors surprisingly found that bacterial RNA polymerase enabled initiation of replication of DNA on the ColE1-like origin of replication, in association with a phage polymerase (*e.g*., Phi29 DNA polymerase) or an artificial or non-native replisome (*e.g.*, phage replisome).

Therefore, in some embodiments, contacting the DNA molecule with the at least one bacterial RNA polymerase initiates DNA amplification. In some embodiments, step (i) of RCA or Theta amplification as defined herein (*i.e*., initiation step) is performed by contacting the DNA molecule with the at least one bacterial RNA polymerase.

In some embodiments, the at least one bacterial RNA polymerase is configured to bind to the at least one origin of replication. In some embodiments, the at least one bacterial RNA polymerase is configured to bind to the at least one ColE1-like origin of replication. In some embodiments, the at least one bacterial RNA polymerase binds to the at least one ColE1-like origin of replication on the DNA molecule.

Thus, in some embodiments, the amplification is initiated by the binding of the at least one bacterial RNA polymerase to the at least one origin of replication. In some embodiments, the amplification is initiated by the binding of the at least one bacterial RNA polymerase to the at least one ColE1-like origin of replication.

In some embodiments, in the first composition, the at least one bacterial RNA polymerase is from any bacterial or mycobacterial species. In some embodiments, the at least one bacterial RNA polymerase is from bacterial species from the genus *Escherichia.*

In some embodiments, the at least one bacterial RNA polymerase is *Escherichia coli* RNA polymerase holoenzyme (herein interchangeably referred to as RNAP, EcoRNAP, or *E. coli* Protein DNA-directed RNA polymerase), or a variant thereof, or a nucleic acid encoding thereof.

In some embodiments, *Escherichia coli* RNAP comprises or consists of at least one, preferably at least two, more preferably at least three, even more preferably at least four, even more preferably five, of the subunits selected from the group comprising or consisting of Protein DNA-directed RNA polymerase subunit alpha (rpoA), Protein DNA-directed RNA polymerase subunit beta (rpoB), Protein DNA-directed RNA polymerase subunit beta' (rpoC), Protein DNA-directed RNA polymerase subunit omega (rpoZ), and Protein RNA polymerase sigma factor 70 (RpoD).

In some embodiments, rpoA has an amino acid sequence having 75%, 85%, 90%, 95%, 96%, 97%, 98%, 99%, 99.5%, or 100% sequence identity with SEQ ID NO: 1. In some embodiments, rpoA has an amino acid sequence as set forth in SEQ ID NO: 1.

In some embodiments, rpoB has an amino acid sequence having 75%, 85%, 90%, 95%, 96%, 97%, 98%, 99%, 99.5%, or 100% sequence identity with SEQ ID NO: 2. In some embodiments, rpoB has an amino acid sequence as set forth in SEQ ID NO: 2.

In some embodiments, rpoC has an amino acid sequence having 75%, 85%, 90%, 95%, 96%, 97%, 98%, 99%, 99.5%, or 100% sequence identity with SEQ ID NO: 3. In some embodiments, rpoC has an amino acid sequence as set forth in SEQ ID NO: 3.

In some embodiments, rpoZ has an amino acid sequence having 75%, 85%, 90%, 95%, 96%, 97%, 98%, 99%, 99.5%, or 100% sequence identity with SEQ ID NO: 4. In some embodiments, rpoZ has an amino acid sequence as set forth in SEQ ID NO: 4.

In some embodiments, rpoD has an amino acid sequence having 75%, 85%, 90%, 95%, 96%, 97%, 98%, 99%, 99.5%, or 100% sequence identity with SEQ ID NO: 5. In some embodiments, rpoD has an amino acid sequence as set forth in SEQ ID NO: 5.

In some embodiments, the first composition further comprises DNA polymerase I. In some embodiments, DNA polymerase I has an amino acid sequence having 75%, 85%, 90%, 95%, 96%, 97%, 98%, 99%, 99.5%, or 100% sequence identity with SEQ ID NO: 16. In some embodiments, DNA polymerase I has an amino acid sequence as set forth in SEQ ID NO: 16.

In some embodiments, the first composition further comprises at least one RNAse. In some embodiments, the at least one RNAse is RNase H/rnhA, or a variant thereof, or a nucleic acid encoding thereof, preferably the RNase H is *E. coli* RNase H/rnhA, or a variant thereof, or a nucleic acid encoding thereof. In some embodiments, *E. coli* RNase H/rnhA has an amino acid sequence having at least 75%, 80%, 85%, 95%, 96%, 97%, 98%, 99%, 99.5%, or 100% sequence identity with SEQ ID NO: 56. In some embodiments, *E. coli* RNase H/rnhA has an amino acid sequence as set forth in SEQ ID NO: 56.

In some embodiments, the first composition further comprises at least one DNA binding proteins. In some embodiments, the at least one DNA binding protein is *E. coli* SSB, or a variant thereof, or a nucleic acid encoding thereof. In some embodiments, *E*. *coli* SSB has an amino acid sequence having at least 75%, 80%, 85%, 95%, 96%, 97%, 98%, 99%, 99.5%, or 100% sequence identity with SEQ ID NO: 39. In some embodiments, *E. coli* SSB has an amino acid sequence as set forth in SEQ ID NO: 39.

In some embodiments, the second composition of the method of the present invention comprises (i) at least one DNA polymerase and (ii) zero, one, or several (*i.e*., two or more) other components. In some embodiments, the DNA polymerase is a strand displacement DNA polymerase, or a nucleic acid encoding thereof. In some embodiments, the DNA polymerase is capable of displacing the DNA molecule.

In some embodiments, the at least one DNA polymerase is selected from the group comprising or consisting of Phi29 DNA polymerase, DNA polymerase III holoenzyme (Pol III), DNA polymerase II, DNA polymerase I, B35 DNA polymerase, Bst polymerase, GP43 DNA polymerase, GP5 DNA polymerase, and variants thereof, or nucleic acids encoding thereof.

In some embodiments, the at least one DNA polymerase is selected from the group comprising or consisting of Phi29 DNA polymerase, DNA polymerase III holoenzyme (Pol III), DNA polymerase I, B35 DNA polymerase, Bst polymerase, GP43 DNA polymerase, GP5 DNA polymerase, and variants thereof, or nucleic acids encoding thereof.

In some embodiments, the at least one DNA polymerase is Phi29 DNA polymerase, B35 DNA polymerase, and/or Bst polymerase. In some embodiments, the at least one DNA polymerase is Phi29 DNA polymerase and/or B35 DNA polymerase.

In some embodiments, the at least one DNA polymerase is Phi29 DNA polymerase (interchangeably referred to as DNA polymerase phage phi29), or a variant thereof, or a nucleic acid encoding thereof.

In some embodiments, Phi29 DNA polymerase has an amino acid sequence having 75%, 85%, 90%, 95%, 96%, 97%, 98%, 99%, 99.5%, or 100% sequence identity with SEQ ID NO: 6. In some embodiments, Phi29 DNA polymerase has an amino acid sequence as set forth in SEQ ID NO: 6.

In some embodiments, the at least one DNA polymerase is selected from the group comprising or consisting of DNA polymerase III holoenzyme (Pol III), DNA polymerase II, DNA polymerase I, B35 DNA polymerase, GP43 DNA polymerase, GP5 DNA polymerase, and variants thereof, or nucleic acids encoding thereof.

In some embodiments, the at least one DNA polymerase is selected from the group comprising or consisting of DNA polymerase III holoenzyme (Pol III), DNA polymerase I, B35 DNA polymerase, Bst polymerase, GP43 DNA polymerase, GP5 DNA polymerase, and variants thereof, or nucleic acids encoding thereof.

In some embodiments, the at least one DNA polymerase is selected from the group comprising or consisting of DNA polymerase III holoenzyme (Pol III), DNA polymerase I, B35 DNA polymerase and Bst polymerase. In some embodiments, the at least one DNA polymerase is DNA polymerase III holoenzyme (Pol III) and/or DNA polymerase I.

In some embodiments, the at least one DNA polymerase is DNA polymerase III holoenzyme, or a variant thereof, or a nucleic acid encoding thereof. In some embodiments, DNA polymerase III holoenzyme comprises or consists of at least 1, at least 2, at least 3, at least 4, at least 5, at least 6, at least 7, at least 8, or 9, of the subunits selected from the group comprising or consisting of Beta sliding clamp (dnaN, or DPO3B), DNA polymerase III subunit delta (holA), DNA polymerase III subunit delta' (holB), DNA polymerase III subunit chi (holC), DNA polymerase III subunit psi (holD), DNA polymerase III subunit theta (holE), DNA polymerase III subunit alpha (dnaE, or DPO3A), DNA polymerase III subunit epsilon (dnaQ, or DPO3E), and DNA polymerase III subunit tau (dnaX, or DPO3X).

In some embodiments, dnaN/DPO3B has an amino acid sequence having 75%, 85%, 90%, 95%, 96%, 97%, 98%, 99%, 99.5%, or 100% sequence identity with SEQ ID NO: 7. In some embodiments, dnaN/DPO3B has an amino acid sequence as set forth in SEQ ID NO: 7.

In some embodiments, holA has an amino acid sequence having 75%, 85%, 90%, 95%, 96%, 97%, 98%, 99%, 99.5%, or 100% sequence identity with SEQ ID NO: 8. In some embodiments, holA has an amino acid sequence as set forth in SEQ ID NO: 8.

In some embodiments, holB has an amino acid sequence having 75%, 85%, 90%, 95%, 96%, 97%, 98%, 99%, 99.5%, or 100% sequence identity with SEQ ID NO: 9. In some embodiments, holB has an amino acid sequence as set forth in SEQ ID NO: 9.

In some embodiments, holC has an amino acid sequence having 75%, 85%, 90%, 95%, 96%, 97%, 98%, 99%, 99.5%, or 100% sequence identity with SEQ ID NO: 10. In some embodiments, holC has an amino acid sequence as set forth in SEQ ID NO: 10.

In some embodiments, holD has an amino acid sequence having 75%, 85%, 90%, 95%, 96%, 97%, 98%, 99%, 99.5%, or 100% sequence identity with SEQ ID NO: 11. In some embodiments, holD has an amino acid sequence as set forth in SEQ ID NO: 11.

In some embodiments, holE has an amino acid sequence having 75%, 85%, 90%, 95%, 96%, 97%, 98%, 99%, 99.5%, or 100% sequence identity with SEQ ID NO: 12. In some embodiments, holE has an amino acid sequence as set forth in SEQ ID NO: 12.

In some embodiments, dnaE/DPO3A has an amino acid sequence having 75%, 85%, 90%, 95%, 96%, 97%, 98%, 99%, 99.5%, or 100% sequence identity with SEQ ID NO: 13. In some embodiments, dnaE/DPO3A has an amino acid sequence as set forth in SEQ ID NO: 13.

In some embodiments, dnaQ/DPO3E has an amino acid sequence having 75%, 85%, 90%, 95%, 96%, 97%, 98%, 99%, 99.5%, or 100% sequence identity with SEQ ID NO: 14. In some embodiments, dnaQ/DPO3E has an amino acid sequence as set forth in SEQ ID NO: 14.

In some embodiments, dnaX/DPO3X has an amino acid sequence having 75%, 85%, 90%, 95%, 96%, 97%, 98%, 99%, 99.5%, or 100% sequence identity with SEQ ID NO: 15. In some embodiments, dnaX/DPO3X has an amino acid sequence as set forth in SEQ ID NO: 15.

In some embodiments, the at least one DNA polymerase is DNA polymerase I (interchangeably referred to as polA or DPO1), or a variant thereof, or a nucleic acid encoding thereof.

In some embodiments, DNA polymerase I has an amino acid sequence having 75%, 85%, 90%, 95%, 96%, 97%, 98%, 99%, 99.5%, or 100% sequence identity with SEQ ID NO: 16. In some embodiments, DNA polymerase I has an amino acid sequence as set forth in SEQ ID NO: 16.

In some embodiments, the at least one DNA polymerase is B35 DNA polymerase, or a variant thereof, or a nucleic acid encoding thereof. In some embodiments, B35 DNA polymerase is DNA-directed DNA polymerase Bam35c, or B35-HhH DNA polymerase.

In some embodiments, DNA-directed DNA polymerase Bam35c has an amino acid sequence having 75%, 85%, 90%, 95%, 96%, 97%, 98%, 99%, 99.5%, or 100% sequence identity with SEQ ID NO: 17. In some embodiments, DNA-directed DNA polymerase Bam35c has an amino acid sequence as set forth in SEQ ID NO: 17.

In some embodiments, B35-HhH DNA polymerase has an amino acid sequence having 75%, 85%, 90%, 95%, 96%, 97%, 98%, 99%, 99.5%, or 100% sequence identity with SEQ ID NO: 18. In some embodiments, B35-HhH DNA polymerase has an amino acid sequence as set forth in SEQ ID NO: 18.

In some embodiments, the at least one DNA polymerase is selected from the group comprising or consisting of GP43 DNA polymerase, GP5 DNA polymerase, DNA polymerase I, B35 DNA polymerase, and variants thereof, or nucleic acids encoding thereof.

In some embodiments, the at least one DNA polymerase is selected from the group comprising or consisting of GP43 DNA polymerase, DNA polymerase I, B35 DNA polymerase, and variants thereof, or nucleic acids encoding thereof. In some embodiments, the at least one DNA polymerase is GP43 DNA polymerase and/or DNA polymerase I.

In some embodiments, the at least one DNA polymerase is GP43 DNA polymerase (interchangeably referred to as SHEE T4 43, or phage T4 DNA polymerase), or a variant thereof, or a nucleic acid encoding thereof.

In some embodiments, GP43 DNA polymerase has an amino acid sequence having 75%, 85%, 90%, 95%, 96%, 97%, 98%, 99%, 99.5%, or 100% sequence identity with SEQ ID NO: 19. In some embodiments, GP43 DNA polymerase has an amino acid sequence as set forth in SEQ ID NO: 19.

In some embodiments, the at least one DNA polymerase is selected from the group comprising or consisting of GP5 DNA polymerase, DNA polymerase I, B35 DNA polymerase, and variants thereof, or nucleic acids encoding thereof. In some embodiments, the at least one DNA polymerase is GP5 DNA polymerase and/or DNA polymerase I.

In some embodiments, the at least one DNA polymerase is GP5 DNA polymerase, or a variant thereof, or a nucleic acid encoding thereof. In some embodiments, GP5 DNA polymerase comprises or consists of the subunits GP5 and/or Thioredoxin 1. In a preferred embodiment, Thioredoxin 1 is *E. coli* Thioredoxin 1. In certain embodiments, GP5 is phage T7 GP5 (T7 GP5) or phage T3 GP5 (T3 GP5).

In some embodiments, T7 GP5 has an amino acid sequence having 75%, 85%, 90%, 95%, 96%, 97%, 98%, 99%, 99.5%, or 100% sequence identity with SEQ ID NO: 20. In some embodiments, T7 GP5 has an amino acid sequence as set forth in SEQ ID NO: 20.

In some embodiments, T3 GP5 has an amino acid sequence having 75%, 85%, 90%, 95%, 96%, 97%, 98%, 99%, 99.5%, or 100% sequence identity with SEQ ID NO: 21. In some embodiments, T3 GP5 has an amino acid sequence as set forth in SEQ ID NO: 21.

In some embodiments, Thioredoxin 1 has an amino acid sequence having 75%, 85%, 90%, 95%, 96%, 97%, 98%, 99%, 99.5%, or 100% sequence identity with SEQ ID NO: 22. In some embodiments, Thioredoxin 1has an amino acid sequence as set forth in SEQ ID NO: 22.

In some embodiments, the second composition further comprises one or more components selected from the group comprising or consisting of DNA helicases and helicase associated proteins, sliding clamps, DNA binding proteins, DNA ligases, gyrases, topoisomerases, primases, RNAses, RNA polymerases, integration host factors, DNA maintenance proteins, regulators of initiation, repressors, nucleases, recombinases, polynucleotide kinases, and methyl transferases.

In some embodiments, the one or more further components are variants thereof.

In some embodiments, the second composition further comprises one or more components selected from the group comprising or consisting of DNA helicases and helicase associated proteins, sliding clamps, DNA binding proteins, DNA ligases, gyrases, topoisomerases, primases, RNAses.

In some embodiments, the one or more DNA helicase or helicase associated protein is selected from the group comprising or consisting of DnaB Helicase, DnaC Helicase loader, gp41 Helicase, gp59 Helicase loader, gp4 Helicase/Primase, phage T3 Helicase/Primase, ATP-dependent DNA helicase RecQ (RecQ), DNA helicase II uvrD (uvrD), DNA helicase IV helD (helD), ATP-dependent DNA helicase dda (Dda), usvW helicase, or variants thereof, or nucleic acids encoding thereof.

In some embodiments, the one or more DNA helicase or helicase associated protein is selected from the group comprising or consisting of DnaB Helicase, DnaC Helicase loader, ATP-dependent DNA helicase RecQ (RecQ), DNA helicase II uvrD (uvrD), DNA helicase IV helD (helD), ATP-dependent DNA helicase dda (Dda), usvW helicase, or variants thereof, or nucleic acids encoding thereof.

In some embodiments, the one or more DNA helicase or helicase associated protein is DnaB Helicase and/or DnaC Helicase loader, or variants thereof, or nucleic acids encoding thereof.

In some embodiments, the one or more DNA helicase or helicase associated protein is DnaB Helicase, or a variant thereof, or a nucleic acid encoding thereof, preferably *E. coli* DnaB Helicase, or a variant thereof, or a nucleic acid encoding thereof.

In some embodiments, *E*. *coli* DnaB Helicase has an amino acid sequence having at least 75%, 80%, 85%, 95%, 96%, 97%, 98%, 99%, 99.5%, or 100% sequence identity with SEQ ID NO: 23. In some embodiments, *E. coli* DnaB Helicase has an amino acid sequence as set forth in SEQ ID NO: 23.

In some embodiments, the one or more DNA helicase or helicase associated protein is DnaC Helicase loader, or a variant thereof, or a nucleic acid encoding thereof, preferably *E. coli* DnaC Helicase loader, or a variant thereof, or a nucleic acid encoding thereof.

In some embodiments, *E. coli* DnaC Helicase loader has an amino acid sequence having at least 75%, 80%, 85%, 95%, 96%, 97%, 98%, 99%, 99.5% or 100% sequence identity with SEQ ID NO: 24. In some embodiments, *E. coli* DnaC Helicase loader has an amino acid sequence as set forth in SEQ ID NO: 24.

In some embodiments, the one or more DNA helicase or helicase associated protein is selected from the group comprising or consisting of gp41 Helicase, gp59 Helicase loader, gp4 Helicase/Primase, phage T3 Helicase/Primase, ATP-dependent DNA helicase RecQ (RecQ), DNA helicase II uvrD (uvrD), DNA helicase IV helD (helD), ATP-dependent DNA helicase dda (Dda), usvW helicase, or variants thereof, or nucleic acids encoding thereof.

In some embodiments, the one or more DNA helicase or helicase associated protein is selected from the group comprising or consisting of gp41 Helicase, gp59 Helicase loader, ATP-dependent DNA helicase RecQ (RecQ), DNA helicase II uvrD (uvrD), DNA helicase IV helD (helD), ATP-dependent DNA helicase dda (Dda), usvW helicase, or variants thereof, or nucleic acids encoding thereof.

In some embodiments, the one or more DNA helicase or helicase associated protein is gp41 Helicase and/or gp59 Helicase loader, or variants thereof, or nucleic acids encoding thereof.

In some embodiments, the one or more DNA helicase or helicase associated protein is gp41 Helicase (interchangeably referred to as SHEE T4 41), or a variant thereof, or a nucleic acid encoding thereof.

In some embodiments, gp41 Helicase has an amino acid sequence having at least 75%, 80%, 85%, 95%, 96%, 97%, 98%, 99% or 100% sequence identity with SEQ ID NO: 25. In some embodiments, gp41 Helicase has an amino acid sequence as set forth in SEQ ID NO: 25.

In some embodiments, the one or more DNA helicase or helicase associated protein is gp59 Helicase loader (interchangeably referred to as SHEE T4 59), or a variant thereof, or a nucleic acid encoding thereof.

In some embodiments, gp59 Helicase loader has an amino acid sequence having at least 75%, 80%, 85%, 95%, 96%, 97%, 98%, 99% or 100% sequence identity with SEQ ID NO: 26. In some embodiments, gp59 Helicase loader has an amino acid sequence as set forth in SEQ ID NO: 26.

In some embodiments, the one or more DNA helicase or helicase associated protein is selected from the group comprising or consisting of gp4 Helicase/Primase, phage T3 Helicase/Primase, ATP-dependent DNA helicase RecQ (RecQ), DNA helicase II uvrD (uvrD), DNA helicase IV helD (helD), ATP-dependent DNA helicase dda (Dda), usvW helicase, or variants thereof, or nucleic acids encoding thereof.

In some embodiments, the one or more DNA helicase or helicase associated protein is gp4 Helicase/Primase and/or phage T3 Helicase/Primase, or variants thereof, or nucleic acids encoding thereof.

In some embodiments, the one or more DNA helicase or helicase associated protein is gp4 Helicase/Primase, herein interchangeably referred to as phage T7 DNA helicase/primase, or a variant thereof, or a nucleic acid encoding thereof.

In some embodiments, gp4 Helicase/Primase has an amino acid sequence having at least 75%, 80%, 85%, 95%, 96%, 97%, 98%, 99%, 99.5%, or 100% sequence identity with SEQ ID NO: 27. In some embodiments, gp4 Helicase/Primase has an amino acid sequence as set forth in SEQ ID NO: 27.

In some embodiments, the one or more DNA helicase or helicase associated protein is phage T3 Helicase/Primase, herein interchangeably referred to as phage T7 DNA helicase/primase, or a variant thereof, or a nucleic acid encoding thereof.

In some embodiments, phage T3 Helicase/Primase has an amino acid sequence having at least 75%, 80%, 85%, 95%, 96%, 97%, 98%, 99%, 99.5%, or 100% sequence identity with SEQ ID NO: 28. In some embodiments, phage T3 Helicase/Primase has an amino acid sequence as set forth in SEQ ID NO: 28.

In some embodiments, the one or more DNA helicase or helicase associated protein is RecQ, or a variant thereof, or a nucleic acid encoding thereof, preferably *E. coli* RecQ, or a variant thereof, or a nucleic acid encoding thereof.

In some embodiments, *E. coli* RecQ has an amino acid sequence having at least 75%, 80%, 85%, 95%, 96%, 97%, 98%, 99%, 99.5%, or 100% sequence identity with SEQ ID NO: 29. In some embodiments, *E. coli* RecQ has an amino acid sequence as set forth in SEQ ID NO: 29.

In some embodiments, the one or more DNA helicase or helicase associated protein is uvrD, or a variant thereof, or a nucleic acid encoding thereof, preferably *E. coli* uvrD, or a variant thereof, or a nucleic acid encoding thereof.

In some embodiments, *E. coli* uvrD has an amino acid sequence having at least 75%, 80%, 85%, 95%, 96%, 97%, 98%, 99%, 99.5%, or 100% sequence identity with SEQ ID NO: 30. In some embodiments, *E. coli* uvrD has an amino acid sequence as set forth in SEQ ID NO: 30.

In some embodiments, the one or more DNA helicase or helicase associated protein is helD, or a variant thereof, or a nucleic acid encoding thereof, preferably *E. coli* helD, or a variant thereof, or a nucleic acid encoding thereof.

In some embodiments, *E. coli* helD has an amino acid sequence having at least 75%, 80%, 85%, 95%, 96%, 97%, 98%, 99%, 99.5%, or 100% sequence identity with SEQ ID NO: 31 . In some embodiments, *E. coli* helD has an amino acid sequence as set forth in SEQ ID NO: 31.

In some embodiments, the one or more DNA helicase or helicase associated protein is ATP-dependent DNA helicase dda (interchangeably referred to as SHEE T4 Dda, or Dda), or a variant thereof, or a nucleic acid encoding thereof.

In some embodiments, Dda has an amino acid sequence having at least 75%, 80%, 85%, 95%, 96%, 97%, 98%, 99%, 99.5%, or 100% sequence identity with SEQ ID NO: 32. In some embodiments, Dda has an amino acid sequence as set forth in SEQ ID NO: 32.

In some embodiments, the one or more DNA helicase or helicase associated protein is usvW, or a variant thereof, or a nucleic acid encoding thereof.

In some embodiments, usvW has an amino acid sequence having at least 75%, 80%, 85%, 95%, 96%, 97%, 98%, 99%, 99.5%, or 100% sequence identity with SEQ ID NO: 33. In some embodiments, usvW has an amino acid sequence as set forth in SEQ ID NO: 33.

In some embodiments, the one or more primase is selected from the group comprising or consisting of DnaG primase, gp61 Primase, gp4 Helicase/Primase, phage T3 Helicase/Primase, or variants thereof, or nucleic acids encoding thereof.

In some embodiments, the one or more primase is DnaG primase, or a variant thereof, or a nucleic acid encoding thereof, preferably *E. coli* DnaG primase, or a variant thereof, or a nucleic acid encoding thereof.

In some embodiments, *E. coli* DnaG primase has an amino acid sequence having at least 75%, 80%, 85%, 95%, 96%, 97%, 98%, 99%, 99.5%, or 100% sequence identity with SEQ ID NO: 34. In some embodiments, *E. coli* DnaG primase has an amino acid sequence as set forth in SEQ ID NO: 34.

In some embodiments, the one or more primase is selected from the group comprising or consisting of gp61 Primase, gp4 Helicase/Primase, phage T3 Helicase/Primase, or variants thereof, or nucleic acids encoding thereof.

In some embodiments, the one or more primase is gp61 Primase (interchangeably referred to as SHEE T4 61), or a variant thereof, or a nucleic acid encoding thereof.

In some embodiments, gp61 Primase has an amino acid sequence having at least 75%, 80%, 85%, 95%, 96%, 97%, 98%, 99% or 100% sequence identity with SEQ ID NO: 35. In some embodiments, gp61 Primase has an amino acid sequence as set forth in SEQ ID NO: 35.

In some embodiments, the one or more primase is gp4 Helicase/Primase and/or phage T3 Helicase/Primase, or variants thereof, or nucleic acids encoding thereof.

In some embodiments, the one or more primase is gp4 Helicase/Primase, or a variant thereof, or a nucleic acid encoding thereof.

In some embodiments, gp4 Helicase/Primase has an amino acid sequence having at least 75%, 80%, 85%, 95%, 96%, 97%, 98%, 99% or 100% sequence identity with SEQ ID NO: 27. In some embodiments, gp4 Helicase/Primase has an amino acid sequence as set forth in SEQ ID NO: 28.

In some embodiments, the one or more primase is phage T3 Helicase/Primase, herein interchangeably referred to as phage T7 DNA helicase/primase, or a variant thereof, or a nucleic acid encoding thereof.

In some embodiments, phage T3 Helicase/Primase has an amino acid sequence having at least 75%, 80%, 85%, 95%, 96%, 97%, 98%, 99%, 99.5%, or 100% sequence identity with SEQ ID NO: 28. In some embodiments, phage T3 Helicase/Primase has an amino acid sequence as set forth in SEQ ID NO: 28.

In some embodiments, the one or more sliding clamp is selected from the group comprising or consisting of gp45 sliding clamp, gp44/62 clamp loader, Beta sliding clamp (dnaN, or DPO3B), or variants thereof, or nucleic acids encoding thereof.

In some embodiments, the one or more sliding clamp is gp45 sliding clamp (interchangeably referred to as SHEE T4 45), or a variant thereof, or a nucleic acid encoding thereof.

In some embodiments, gp45 sliding clamp has an amino acid sequence having at least 75%, 80%, 85%, 95%, 96%, 97%, 98%, 99% or 100% sequence identity with SEQ ID NO: 36. In some embodiments, gp45 sliding clamp has an amino acid sequence as set forth in SEQ ID NO: 36.

In some embodiments, the one or more sliding clamp is gp44/62 clamp loader, or a variant thereof, or a nucleic acid encoding thereof. In some embodiments, gp44/62 clamp loader comprises or consists of the subunits gp44 (interchangeably referred to as SHEE T4 44 large subunit) and/or gp62 (interchangeably referred to as SHEE T4 62 small subunit).

In some embodiments, gp44 has an amino acid sequence having at least 75%, 80%, 85%, 95%, 96%, 97%, 98%, 99% or 100% sequence identity with SEQ ID NO: 37. In some embodiments, gp44 has an amino acid sequence as set forth in SEQ ID NO: 37.

In some embodiments, gp62 has an amino acid sequence having at least 75%, 80%, 85%, 95%, 96%, 97%, 98%, 99% or 100% sequence identity with SEQ ID NO: 38. In some embodiments, gp62 has an amino acid sequence as set forth in SEQ ID NO: 38.

In some embodiments, the one or more sliding clamp is dnaN, or a variant thereof, or a nucleic acid encoding thereof.

In some embodiments, dnaN has an amino acid sequence having 75%, 85%, 90%, 95%, 96%, 97%, 98%, 99%, 99.5%, or 100% sequence identity with SEQ ID NO: 7. In some embodiments, dnaN has an amino acid sequence as set forth in SEQ ID NO: 7.

In some embodiments, the one or more DNA binding protein is selected from the group comprising or consisting of *E. coli* SSB (single stranded DNA-binding protein), gp32 SSB, gp2.5 SSB, T3 Single-stranded DNA-binding protein, or variants thereof, or nucleic acids encoding thereof.

In some embodiments, the one or more DNA binding protein is *E*. *coli* SSB, or a variant thereof, or a nucleic acid encoding thereof.

In some embodiments, *E. coli* SSB has an amino acid sequence having at least 75%, 80%, 85%, 95%, 96%, 97%, 98%, 99%, 99.5%, or 100% sequence identity with SEQ ID NO: 39. In some embodiments, *E. coli* SSB has an amino acid sequence as set forth in SEQ ID NO: 39.

In some embodiments, the one or more DNA binding protein is selected from the group comprising or consisting of gp32 SSB, gp2.5 SSB, T3 Single-stranded DNA-binding protein, or variants thereof, or nucleic acids encoding thereof.

In some embodiments, the one or more DNA binding protein is gp32 SSB (interchangeably referred to as SHEE T4 32), or a variant thereof, or a nucleic acid encoding thereof.

In some embodiments, gp32 SSB has an amino acid sequence having at least 75%, 80%, 85%, 95%, 96%, 97%, 98%, 99% or 100% sequence identity with SEQ ID NO: 40. In some embodiments, gp32 SSB has an amino acid sequence as set forth in SEQ ID NO: 40.

In some embodiments, the one or more DNA binding protein is gp2.5 SSB and/or T3 Single-stranded DNA-binding protein, or variants thereof, or nucleic acids encoding thereof.

In some embodiments, the one or more DNA binding protein is gp2.5 SSB, herein interchangeably referred to as phage T7 Single-stranded DNA-binding protein, or a variant thereof, or a nucleic acid encoding thereof.

In some embodiments, gp2.5 SSB has an amino acid sequence having at least 75%, 80%, 85%, 95%, 96%, 97%, 98%, 99%, 99.5%, or 100% sequence identity with SEQ ID NO: 41. In some embodiments, gp2.5 SSB has an amino acid sequence as set forth in SEQ ID NO: 41.

In some embodiments, the one or more DNA binding protein is T3 Single-stranded DNA-binding protein, or a variant thereof, or a nucleic acid encoding thereof.

In some embodiments, T3 Single-stranded DNA-binding protein has an amino acid sequence having at least 75%, 80%, 85%, 95%, 96%, 97%, 98%, 99%, 99.5%, or 100% sequence identity with SEQ ID NO: 42. In some embodiments, T3 Single-stranded DNA-binding protein has an amino acid sequence as set forth in SEQ ID NO: 42.

In some embodiments, the one or more DNA ligase is selected from the group comprising or consisting of *E. coli* Protein DNA ligase (interchangeably referred to as ligA, DNLJ, or "*E*. *coli* ligase"), T7 DNA ligase, T3 DNA ligase, T4 DNA ligase, and variants thereof, or nucleic acids encoding thereof.

In some embodiments, the one or more DNA ligase is *E*. *coli* ligase, or a variant thereof, or a nucleic acid encoding thereof.

In some embodiments, *E. coli* ligase has an amino acid sequence having at least 75%, 80%, 85%, 95%, 96%, 97%, 98%, 99%, 99.5%, or 100% sequence identity with SEQ ID NO: 43. In some embodiments, *E. coli* ligase has an amino acid sequence as set forth in SEQ ID NO: 43.

In some embodiments, the one or more DNA ligase is selected from the group comprising or consisting of T7 DNA ligase, T3 DNA ligase, T4 DNA ligase, dumbbell ligase, and variants thereof, or nucleic acids encoding thereof.

In some embodiments, the one or more DNA ligase is T7 DNA ligase and/or T3 DNA ligase, and variants thereof, or nucleic acids encoding thereof.

In some embodiments, the one or more DNA ligase is T7 DNA ligase, or a variant thereof, or a nucleic acid encoding thereof.

In some embodiments, T7 DNA ligase has an amino acid sequence having at least 75%, 80%, 85%, 95%, 96%, 97%, 98%, 99%, 99.5%, or 100% sequence identity with SEQ ID NO: 44. In some embodiments, T7 DNA ligase has an amino acid sequence as set forth in SEQ ID NO: 44.

In some embodiments, the one or more DNA ligase is T3 DNA ligase, or a variant thereof, or a nucleic acid encoding thereof.

In some embodiments, T3 DNA ligase has an amino acid sequence having at least 75%, 80%, 85%, 95%, 96%, 97%, 98%, 99%, 99.5%, or 100% sequence identity with SEQ ID NO: 45. In some embodiments, T3 DNA ligase has an amino acid sequence as set forth in SEQ ID NO: 45.

In some embodiments, the one or more DNA ligase is T4 DNA ligase (interchangeably referred to as SHEE T4 30), or a variant thereof, or a nucleic acid encoding thereof.

In some embodiments, T4 DNA ligase has an amino acid sequence having at least 75%, 80%, 85%, 95%, 96%, 97%, 98%, 99%, 99.5%, or 100% sequence identity with SEQ ID NO: 46. In some embodiments, T4 DNA ligase has an amino acid sequence as set forth in SEQ ID NO: 46.

In some embodiments, the one or more gyrase is *E. coli* gyrase comprising the subunits DNA gyrase subunit A (GyrA) and DNA gyrase subunit B (GyrB), or variants thereof, or nucleic acids encoding thereof.

In some embodiments, GyrA has an amino acid sequence having at least 75%, 80%, 85%, 95%, 96%, 97%, 98%, 99%, 99.5%, or 100% sequence identity with SEQ ID NO: 47. In some embodiments, GyrA has an amino acid sequence as set forth in SEQ ID NO: 47.

In some embodiments, GyrB has an amino acid sequence having at least 75%, 80%, 85%, 95%, 96%, 97%, 98%, 99%, 99.5%, or 100% sequence identity with SEQ ID NO: 48. In some embodiments, GyrB has an amino acid sequence as set forth in SEQ ID NO: 48.

In some embodiments, the one or more topoisomerase is selected from the group comprising or consisting of Topoisomerase III (Topo III, or topB), Topoisomerase IV (Topo IV), phage T4 DNA topoisomerase, Topoisomerase I (Topo I, or topA) or variants thereof, or nucleic acids encoding thereof.

In some embodiments, the one or more topoisomerase is selected from the group comprising or consisting of Topoisomerase III (Topo III, or topB), Topoisomerase IV (Topo IV), Topoisomerase I (Topo I, or topA) or variants thereof, or nucleic acids encoding thereof.

In some embodiments, the one or more topoisomerase is Topo III, preferably *E*. *coli* Topo III, or a variant thereof, or a nucleic acid encoding thereof.

In some embodiments, *E. coli* Topo III has an amino acid sequence having at least 75%, 80%, 85%, 95%, 96%, 97%, 98%, 99%, 99.5%, or 100% sequence identity with SEQ ID NO: 49. In some embodiments, *E. coli* Topo III has an amino acid sequence as set forth in SEQ ID NO: 49.

In some embodiments, the one or more topoisomerase is Topo IV, preferably *E*. *coli* Topo IV, or a variant thereof, or a nucleic acid encoding thereof. In some embodiments, *E. coli* Topo IV comprises or consists of the subunits DNA topoisomerase IV subunit B (ParE) and/or DNA topoisomerase IV subunit A (ParC).

In some embodiments, ParE has an amino acid sequence having at least 75%, 80%, 85%, 95%, 96%, 97%, 98%, 99%, 99.5%, or 100% sequence identity with SEQ ID NO: 50. In some embodiments, ParE has an amino acid sequence as set forth in SEQ ID NO: 50.

In some embodiments, ParC has an amino acid sequence having at least 75%, 80%, 85%, 95%, 96%, 97%, 98%, 99%, 99.5%, or 100% sequence identity with SEQ ID NO: 51. In some embodiments, ParC has an amino acid sequence as set forth in SEQ ID NO: 51.

In some embodiments, the one or more topoisomerase is selected from the group comprising or consisting of phage T4 DNA topoisomerase, Topoisomerase I (Topo I, or topA) or variants thereof, or nucleic acids encoding thereof.

In some embodiments, the one or more topoisomerase is phage T4 DNA topoisomerase, or a variant thereof, or a nucleic acid encoding thereof. In some embodiments, phage T4 DNA topoisomerase comprises or consists of 1, 2, or 3, preferably 3, of the subunits selected from the group comprising or consisting of DNA topoisomerase large subunit (SHEE T4 39), DNA topoisomerase medium subunit (SHEE T4 52), and DNA topoisomerase small subunit (SHEE T4 60).

In some embodiments, SHEE T4 39 has an amino acid sequence having at least 75%, 80%, 85%, 95%, 96%, 97%, 98%, 99%, 99.5%, or 100% sequence identity with SEQ ID NO: 52. In some embodiments, SHEE T4 39 has an amino acid sequence as set forth in SEQ ID NO: 52.

In some embodiments, SHEE T4 52 has an amino acid sequence having at least 75%, 80%, 85%, 95%, 96%, 97%, 98%, 99%, 99.5%, or 100% sequence identity with SEQ ID NO: 53. In some embodiments, SHEE T4 52 has an amino acid sequence as set forth in SEQ ID NO: 53.

In some embodiments, SHEE T4 60 has an amino acid sequence having at least 75%, 80%, 85%, 95%, 96%, 97%, 98%, 99%, 99.5%, or 100% sequence identity with SEQ ID NO: 54. In some embodiments, SHEE T4 60 has an amino acid sequence as set forth in SEQ ID NO: 54.

In some embodiments, the one or more topoisomerase is Topo I, preferably *E*. *coli* Topo I, or a variant thereof, or a nucleic acid encoding thereof.

In some embodiments, Topo I has an amino acid sequence having at least 75%, 80%, 85%, 95%, 96%, 97%, 98%, 99%, 99.5%, or 100% sequence identity with SEQ ID NO: 55. In some embodiments, Topo I has an amino acid sequence as set forth in SEQ ID NO: 55.

In some embodiments, the one or more RNAse is selected from the group comprising or consisting of Ribonuclease HI (RNase H, or rnhA), phage T4 RNaseH, or variants thereof, or nucleic acids encoding thereof.

In some embodiments, the one or more RNAse is RNase H/rnhA, or a variant thereof, or a nucleic acid encoding thereof. In some embodiments, the RNase H is *E. coli* RNase H/rnhA, or a variant thereof, or a nucleic acid encoding thereof.

In some embodiments, *E. coli* RNase H/rnhA has an amino acid sequence having at least 75%, 80%, 85%, 95%, 96%, 97%, 98%, 99%, 99.5%, or 100% sequence identity with SEQ ID NO: 56. In some embodiments, *E. coli* RNase H/rnhA has an amino acid sequence as set forth in SEQ ID NO: 56.

In some embodiments, the one or more RNAse is phage T4 RNaseH, or a variant thereof, or a nucleic acid encoding thereof.

In some embodiments, phage T4 RNaseH has an amino acid sequence having at least 75%, 80%, 85%, 95%, 96%, 97%, 98%, 99%, 99.5%, or 100% sequence identity with SEQ ID NO: 57. In some embodiments, phage T4 RNaseH has an amino acid sequence as set forth in SEQ ID NO: 57.

In some embodiments, the one or more RNA polymerase is selected from the group comprising or consisting of sigma-E factor rpoE Sigma-24 (rpoE, or σ24, interchangeably referred to as "RNA polymerase extracytoplasmic E"), sigma factor RpoH sigma-32 (rpoH, or σ32), sigma factor RpoS Sigma-38 (rpoS, or σ38), and variants thereof, or nucleic acids encoding thereof. In some embodiments, the one or more RNA polymerase is selected from the group comprising or consisting of *E. coli* rpoE, *E. coli* rpoH, *E. coli* rpoS, and variants thereof, or nucleic acids encoding thereof.

In some embodiments, rpoE has an amino acid sequence having at least 75%, 80%, 85%, 95%, 96%, 97%, 98%, 99%, 99.5%, or 100% sequence identity with SEQ ID NO: 58. In some embodiments, rpoE has an amino acid sequence as set forth in SEQ ID NO: 58.

In some embodiments, rpoH has an amino acid sequence having at least 75%, 80%, 85%, 95%, 96%, 97%, 98%, 99%, 99.5%, or 100% sequence identity with SEQ ID NO: 59. In some embodiments, rpoH has an amino acid sequence as set forth in SEQ ID NO: 59.

In some embodiments, rpoS has an amino acid sequence having at least 75%, 80%, 85%, 95%, 96%, 97%, 98%, 99%, 99.5%, or 100% sequence identity with SEQ ID NO: 60. In some embodiments, rpoS has an amino acid sequence as set forth in SEQ ID NO: 60.

In some embodiments, the one or more integration host factor is integration host factor, preferably *E. coli* integration host factor, comprising the subunits integration host factor subunit alpha (ihfA) and integration host factor subunit beta (ihfB), or variants thereof, or nucleic acids encoding thereof.

In some embodiments, ihfA has an amino acid sequence having at least 75%, 80%, 85%, 95%, 96%, 97%, 98%, 99%, 99.5%, or 100% sequence identity with SEQ ID NO: 61. In some embodiments, ihfA has an amino acid sequence as set forth in SEQ ID NO: 61.

In some embodiments, ihfB has an amino acid sequence having at least 75%, 80%, 85%, 95%, 96%, 97%, 98%, 99%, 99.5%, or 100% sequence identity with SEQ ID NO: 62. In some embodiments, ihfB has an amino acid sequence as set forth in SEQ ID NO: 62.

In some embodiments, the one or more DNA maintenance proteins is selected from the group comprising or consisting of RecA, RecBCD, RecF, and variants thereof, or nucleic acids encoding thereof. In some embodiments, the one or more DNA maintenance proteins is selected from the group comprising or consisting of *E. coli* RecA, *E. coli* RecBCD, *E. coli* RecF, and variants thereof, or nucleic acids encoding thereof.

In some embodiments, RecA has an amino acid sequence having at least 75%, 80%, 85%, 95%, 96%, 97%, 98%, 99%, 99.5%, or 100% sequence identity with SEQ ID NO: 63. In some embodiments, RecA has an amino acid sequence as set forth in SEQ ID NO: 63.

In some embodiments, RecBCD comprises or consists of at least one, preferably at least 2, more preferably 3, of the subunits selected from the group comprising or consisting of RecB, RecC, and RecD.

In some embodiments, RecB has an amino acid sequence having at least 75%, 80%, 85%, 95%, 96%, 97%, 98%, 99%, 99.5%, or 100% sequence identity with SEQ ID NO: 64. In some embodiments, RecB has an amino acid sequence as set forth in SEQ ID NO: 64.

In some embodiments, RecC has an amino acid sequence having at least 75%, 80%, 85%, 95%, 96%, 97%, 98%, 99%, 99.5%, or 100% sequence identity with SEQ ID NO: 65. In some embodiments, RecC has an amino acid sequence as set forth in SEQ ID NO: 65.

In some embodiments, RecD has an amino acid sequence having at least 75%, 80%, 85%, 95%, 96%, 97%, 98%, 99%, 99.5%, or 100% sequence identity with SEQ ID NO: 66. In some embodiments, RecD has an amino acid sequence as set forth in SEQ ID NO: 66.

In some embodiments, RecF has an amino acid sequence having at least 75%, 80%, 85%, 95%, 96%, 97%, 98%, 99%, 99.5%, or 100% sequence identity with SEQ ID NO: 67. In some embodiments, RecF has an amino acid sequence as set forth in SEQ ID NO: 67.

In some embodiments, the one or more regulator of initiation is DnaA initiator-associating protein (DiaA), preferably *E. coli* DiaA, or variants thereof, or nucleic acids encoding thereof.

In some embodiments, *E. coli* DiaA has an amino acid sequence having at least 75%, 80%, 85%, 95%, 96%, 97%, 98%, 99%, 99.5%, or 100% sequence identity with SEQ ID NO: 68. In some embodiments, *E. coli* DiaA has an amino acid sequence as set forth in SEQ ID NO: 68.

In some embodiments, the one or more repressor is repressor of primer (Rop), preferably *E. coli* Rop, or variants thereof, or nucleic acids encoding thereof.

In some embodiments, *E. coli* Rop has an amino acid sequence having at least 75%, 80%, 85%, 95%, 96%, 97%, 98%, 99%, 99.5%, or 100% sequence identity with SEQ ID NO: 69. In some embodiments, *E. coli* Rop has an amino acid sequence as set forth in SEQ ID NO: 69.

In some embodiments, the one or more nuclease is selected from the group comprising or consisting of T7 endonuclease, T7 exonuclease, T3 endonuclease, T3 exonuclease, T4 endonuclease V, T4 endonuclease VII, T4 endonuclease SEGA, or variants thereof, or nucleic acids encoding thereof.

In some embodiments, the one or more nuclease is T7 endonuclease, or a variant thereof, or a nucleic acid encoding thereof.

In some embodiments, T7 endonuclease has an amino acid sequence having at least 75%, 80%, 85%, 95%, 96%, 97%, 98%, 99%, 99.5%, or 100% sequence identity with SEQ ID NO: 70. In some embodiments, T7 endonuclease has an amino acid sequence as set forth in SEQ ID NO: 70.

In some embodiments, the one or more nuclease is T7 exonuclease, or a variant thereof, or a nucleic acid encoding thereof.

In some embodiments, T7 exonuclease has an amino acid sequence having at least 75%, 80%, 85%, 95%, 96%, 97%, 98%, 99%, 99.5%, or 100% sequence identity with SEQ ID NO: 71. In some embodiments, T7 exonuclease has an amino acid sequence as set forth in SEQ ID NO: 71.

In some embodiments, the one or more nuclease is T3 endonuclease, or a variant thereof, or a nucleic acid encoding thereof.

In some embodiments, T3 endonuclease has an amino acid sequence having at least 75%, 80%, 85%, 95%, 96%, 97%, 98%, 99%, 99.5%, or 100% sequence identity with SEQ ID NO: 72. In some embodiments, T3 endonuclease has an amino acid sequence as set forth in SEQ ID NO: 72.

In some embodiments, the one or more nuclease is T3 exonuclease, or a variant thereof, or a nucleic acid encoding thereof.

In some embodiments, T3 exonuclease has an amino acid sequence having at least 75%, 80%, 85%, 95%, 96%, 97%, 98%, 99%, 99.5%, or 100% sequence identity with SEQ ID NO: 73. In some embodiments, T3 exonuclease has an amino acid sequence as set forth in SEQ ID NO: 73.

In some embodiments, the one or more nuclease is T4 endonuclease V (EndoV), or a variant thereof, or a nucleic acid encoding thereof.

In some embodiments, EndoV has an amino acid sequence having at least 75%, 80%, 85%, 95%, 96%, 97%, 98%, 99%, 99.5%, or 100% sequence identity with SEQ ID NO: 74. In some embodiments, EndoV has an amino acid sequence as set forth in SEQ ID NO: 74.

In some embodiments, the one or more nuclease is T4 endonuclease VII, or a variant thereof, or a nucleic acid encoding thereof.

In some embodiments, T4 endonuclease VII has an amino acid sequence having at least 75%, 80%, 85%, 95%, 96%, 97%, 98%, 99%, 99.5%, or 100% sequence identity with SEQ ID NO: 75. In some embodiments, T4 endonuclease VII has an amino acid sequence as set forth in SEQ ID NO: 75.

In some embodiments, the one or more nuclease is T4 endonuclease SEGA, or a variant thereof, or a nucleic acid encoding thereof.

In some embodiments, T4 endonuclease SEGA has an amino acid sequence having at least 75%, 80%, 85%, 95%, 96%, 97%, 98%, 99%, 99.5%, or 100% sequence identity with SEQ ID NO: 76. In some embodiments, T4 endonuclease SEGA has an amino acid sequence as set forth in SEQ ID NO: 76.

In some embodiments, the one or more recombinase is uvsX or uvsY, or variants thereof, or nucleic acids encoding thereof.

In some embodiments, the one or more recombinase is uvsX, or a variant thereof, or a nucleic acid encoding thereof.

In some embodiments, uvsX has an amino acid sequence having at least 75%, 80%, 85%, 95%, 96%, 97%, 98%, 99%, 99.5%, or 100% sequence identity with SEQ ID NO: 77. In some embodiments, uvsX has an amino acid sequence as set forth in SEQ ID NO: 77.

In some embodiments, the one or more recombinase is uvsY, or a variant thereof, or a nucleic acid encoding thereof.

In some embodiments, uvsY has an amino acid sequence having at least 75%, 80%, 85%, 95%, 96%, 97%, 98%, 99%, 99.5%, or 100% sequence identity with SEQ ID NO: 78. In some embodiments, uvsY has an amino acid sequence as set forth in SEQ ID NO: 78.

In some embodiments, the one or more polynucleotide kinase is phage T4 polynucleotide kinase (PNK), or a variant thereof, or a nucleic acid encoding thereof.

In some embodiments, PNK has an amino acid sequence having at least 75%, 80%, 85%, 95%, 96%, 97%, 98%, 99%, 99.5%, or 100% sequence identity with SEQ ID NO: 79. In some embodiments, PNK has an amino acid sequence as set forth in SEQ ID NO: 79.

In some embodiments, the one or more methyl transferase is DNA-[N6-adenine] methyltransferase (DMA), or a variant thereof, or a nucleic acid encoding thereof.

In some embodiments, DMA has an amino acid sequence having at least 75%, 80%, 85%, 95%, 96%, 97%, 98%, 99%, 99.5%, or 100% sequence identity with SEQ ID NO: 80. In some embodiments, DMA has an amino acid sequence as set forth in SEQ ID NO: 80.

In some embodiments, the second composition further comprises one or more components selected from the group comprising or consisting of protelomerases, integrases, restriction enzymes, and the like.

The present invention further relates to a method for amplifying a DNA molecule, comprising contacting the DNA molecule with a first composition comprising at least one bacterial RNA polymerase or a nucleic acid encoding thereof, and a second composition comprising a strand displacement DNA polymerase or a nucleic acid encoding thereof, wherein the DNA molecule comprises at least one origin of replication and wherein the at least one bacterial RNA polymerase is configured to bind the at least one origin of replication.

In some embodiments, the method comprises at least one step of RCA. In some embodiments, the method is a RCA method.

In a preferred embodiment, the at least one origin of replication comprises or consists of a ColE1-like origin of replication. Thus, in some embodiments, the at least one bacterial RNA polymerase is configured to bind to ColE1-like origin of replication. In some embodiments, the amplification is initiated by the binding of the at least one bacterial RNA polymerase to the at least one origin of replication. In a preferred embodiment, the amplification is initiated by the binding of the at least one bacterial RNA polymerase to the at least one ColE1-like origin of replication.

RNaseH and/or Pol I are particularly useful for produce a DNA that correspond to the supercoiled form I, with the amplification method of the invention.

In a preferred embodiment, the strand displacement DNA polymerase is Phi29 DNA polymerase. Phi29 DNA polymerase is described hereinabove. Phi 29 DNA polymerase is particularly useful for implementing the method according to the invention, wherein the method comprises at least one step of RCA.

In some embodiments, the second composition further comprises *E. coli* Ribonuclease HI (rnhA), *E. coli* DNA polymerase I (polA) and/or B35 DNA polymerase. In some embodiments, the second composition further comprises *E. coli* Ribonuclease HI (rnhA) and/or B35 DNA polymerase. rnhA, polA and B35 DNA polymerase are described hereinabove.

In some embodiments, the second composition further comprises one or more protelomerase, integrase, recombinase and/or restriction enzyme. Alternatively, in some embodiments, the method further comprises contacting the DNA molecule with a third composition comprising one or more protelomerase, integrase, recombinase, dumbbell ligase and/or restriction enzyme.

In some embodiments, the first composition further comprises one or more proteins selected from the group comprising DNA polymerase I having at least 75% sequence identity with SEQ ID NO:16, and variants thereof, or nucleic acids encoding thereof.

In some embodiments, the second composition further comprises one or more proteins selected from the group comprising B35 DNA polymerase having at least 75% sequence identity with SEQ ID NO: 17 or SEQ ID NO: 18, *E. coli* Ribonuclease HI having at least 75% sequence identity with SEQ ID NO: 56, and variants thereof, or nucleic acids encoding thereof.

In some embodiments, the method for amplifying a DNA molecule according to the present invention is a RCA method, preferably a RCA method using Phi29 DNA polymerase. In some embodiments, the method for amplifying a DNA molecule according to the present invention comprises the following steps:
(i) initiation step comprising contacting the DNA molecule with composition comprising at least one bacterial RNA polymerase, preferably RNAP;
(ii) strand displacement step comprising contacting the product of step (i) with a composition comprising Phi29 DNA polymerase; and
(iii) optionally, concatemer processing step comprising contacting the product of step (ii) with at least one integrase, restriction enzyme , dumbbell ligase, and/or protelomerase.

In some embodiments, steps (i), (ii) and (iii) are repeated any number of times, concomitantly or independently from each other.

In some embodiments, the composition of step (ii) further comprises *E. coli* Ribonuclease HI (rnhA), *E. coli* DNA polymerase I (polA), and/or B35 DNA polymerase. In some embodiments, the composition of step (ii) further comprises *E*. *coli* Ribonuclease HI (rnhA) and *E. coli* DNA polymerase I (polA). In some embodiments, the composition of step (ii) further comprises *E. coli* Ribonuclease HI (rnhA). In some embodiments, the composition of step (ii) further comprises *E. coli* DNA polymerase I (polA).

In some embodiments, the composition of step (ii) consists of Phi29 DNA polymerase, *E. coli* Ribonuclease HI (rnhA) and *E. coli* DNA polymerase I (polA). In some embodiments, the composition of step (ii) consists of Phi29 DNA polymerase and *E. coli* Ribonuclease HI (rnhA). In some embodiments, the composition of step (ii) consists of Phi29 DNA polymerase, *E. coli* DNA polymerase I (polA).

In some embodiments, at step (iii), integrases or recombinases will be preferred for circularization of the DNA molecule, restriction enzymes will be preferred for linear DNA synthesis, and protelomerases will be preferred for linear covalently closed DNA synthesis. Recombinases are described hereinabove.

RCA amplification steps may be performed according to well-established protocols known in the art.

In some embodiments, the RCA amplification comprises contacting the DNA molecule with the first and second compositions according to the invention, and optionally one or more components selected from the group comprising or consisting of a buffer, rNTPs and dNTPs. In a preferred embodiment, the enzymatic components are added extemporaneously.

In some embodiments, the RCA is performed for a duration from 4 to 64 hours, preferably for a duration from 8 to 32 hours, more preferably for a duration of about 16 hours. In some embodiments, the RCA is performed at a temperature ranging from 15°C to 50°C, preferably from 20°C to 42°C, more preferably from 30°C to 37°C. In a preferred embodiment, the RCA is performed in isothermal conditions. In some embodiments, the RCA is performed under centrifugation at a speed from 100 rpm to 600 rpm, preferably at a speed from 300 rpm to 400 rpm, more preferably at a speed of about 350 rpm.

It is needless to mention that the RCA yields amplification products of the DNA molecule, or "amplicons".

In some embodiments, the RCA is followed by one or more steps of digestion of the amplification products, preferably one step of digestion of the amplification products. In some embodiments, the one or more steps of digestion of the amplification products comprise using at least one restriction enzyme. Restriction enzymes are well described in the art, and the person skilled in the art is capable of picking a suitable restriction enzyme depending on the application and/or the nucleic acid sequence to be digested. In some embodiments, the one or more steps of digestion of the amplification products comprise incubating the amplification products and the at least one restriction enzyme for a duration from 30 minutes to 3 hours, preferably for a duration from 1 hour to 2 hours, more preferably for a duration of about 1 hour and 30 minutes. In some embodiments, the one or more steps of digestion of the amplification products comprise incubating the amplification products and the at least one restriction enzyme at a temperature ranging from 30°C to 50°C, preferably from 32°C to 42°C, more preferably at about 37°C.

In some embodiments, the RCA is followed by one or more steps of purification of the amplification products and/or of the digested amplification products. In some embodiments, the purification is performed by any suitable mean known in the art, preferably the purification is performed by silica beads, filtration or Fast Protein Liquid Chromatography (FPLC). In some embodiments, the purified amplification products and/or purified digested amplification are resuspended in a purification buffer.

In some embodiments, the RCA is followed by a first step of digestion of the amplification products, and a second step of purification of the digested amplification products.

In some embodiments, the RCA is followed by a first step of purification of the amplification products, a second step of digestion of the purified amplification products, and a third step of purification of the digested amplification products.

The present invention further relates to a method for amplifying a DNA molecule, comprising contacting the DNA molecule with a first composition comprising at least one bacterial RNA polymerase or a nucleic acid encoding thereof, and a second composition comprising at least one non-native or artificial replisome with respect to the at least one bacterial RNA polymerase or nucleic acids encoding thereof, wherein the DNA molecule comprises at least one origin of replication and wherein the at least one bacterial RNA polymerase is configured to bind the at least one origin of replication.

In some embodiments, the method comprises at least one step of theta amplification. In some embodiments, the method is a theta amplification method. In certain embodiments, the method is a theta amplification method further comprising one or more steps of RCA.

In a preferred embodiment, the at least one origin of replication comprises or consists of a ColE1-like origin of replication. Thus, in some embodiments, the at least one bacterial RNA polymerase is configured to bind to ColE1-like origin of replication. In some embodiments, the amplification is initiated by the binding of the at least one bacterial RNA polymerase to the at least one origin of replication. In a preferred embodiment, the amplification is initiated by the binding of the at least one bacterial RNA polymerase to the at least one ColE1-like origin of replication.

In a preferred embodiment, the at least one non-native or artificial replisome comprises at least one DNA polymerase.

The general term "replisome", as used herein, refers to a plurality of proteins (*e.g.,* a complex) that cooperate in order to achieve DNA replication. A replisome may comprise, non-imitatively, one or more enzymes that are directly involved in DNA strand synthesis and protein factors that coordinate and accelerate the process. In some embodiments, a replisome comprises one or more of the following: DNA polymerases, DNA helicases, primases, sliding clamps, DNA binding proteins, DNA ligases, and the like.

In some embodiments, the replisome is a bacterial replisome or an artificial or non-native replisome.

In a less preferred embodiment, the replisome is a bacterial replisome. In some embodiments, the bacterial replisome is *E*. *coli-based* replisome.

In a preferred embodiment, the replisome is an artificial or non-native replisome.

As used herein, the expressions "artificial replisome" and "non-native replisome" are used interchangeably. In some embodiments, at least one non-native replisome is from a different species than the at least one bacterial RNA polymerase, i.e., in some embodiments, the at least one non-native replisome shares at most 95%, 90%, 80%, 70%, 60%, 50%, 40%, 30%, 20%, 10% or less of its components with the native replisome from the species of origin of the at least one bacterial RNA polymerase. In some embodiments, at least one artificial replisome does not exist in nature.

In some embodiments, the artificial or non-native replisome is a phage T4-based replisome, a phage T3-based replisome, or a phage T7-based replisome.

Replisomes, as described herein, are particularly useful for implementing the method according to the invention, wherein the method comprises at least one step of Theta amplification, and, optionally, at least one step of RCA.

In some embodiments, the first composition further comprises one or more proteins selected from the group comprising DNA polymerase I having at least 75% sequence identity with SEQ ID NO: 16, *E. coli* Single-stranded DNA-binding protein having at least 75% sequence identity with SEQ ID NO: 39, *E. coli* RNase H having at least 75% sequence identity with SEQ ID NO: 56, and variants thereof, or nucleic acids encoding thereof

In some embodiments, the method for amplifying a DNA molecule according to the present invention comprises contacting the DNA molecule with a first composition comprising at least one bacterial RNA polymerase, preferably RNAP, and a second composition comprising at least one *E*. *coli*-based replisome.

In some embodiments, the *E*. *coli-based* replisome comprises or consists of one or more of the proteins selected from the group comprising or consisting of *E. coli* DNA polymerase I (polA), *E. coli* Ribonuclease HI, *E. coli* Single-stranded DNA-binding protein, *E. coli* Replicative DNA helicase (DnaB), *E. coli* DNA replication protein (DnaC), *E. coli* DNA primase (DnaG), *E. coli* DNA Gyrase, *E. coli* DNA polymerase III holoenzyme, *E. coli* Protein DNA ligase, *E. coli* DNA topoisomerase III, *E. coli* DNA topoisomerase IV, *E*. *coli* DNA topoisomerase I, *E*. *coli* RpoE, *E. coli* RpoH, *E. coli* RpoS*, E. coli* ihfA, *E. coli* ihfB, *E. coli* diaA, *E. coli* RecA, *E. coli* RecBCD, *E. coli* RecQ, *E*. *coli* RecF, *E. coli* uvrD, *E. coli* HelD, and Rop.

In some embodiments, the *E*. *coli-based* replisome comprises or consists of one or more of the proteins selected from the group comprising or consisting of *E. coli* DNA polymerase I (polA), *E. coli* Ribonuclease HI, *E. coli* Single-stranded DNA-binding protein, *E. coli* Replicative DNA helicase (DnaB), *E. coli* DNA replication protein (DnaC), *E. coli* DNA primase (DnaG), *E. coli* DNA Gyrase, *E. coli* DNA polymerase III holoenzyme, *E. coli* Protein DNA ligase, *E. coli* DNA topoisomerase III, and *E. coli* DNA topoisomerase IV.

In some embodiments, the *E*. *coli-based* replisome comprises or consists of one or more of the proteins selected from the group comprising or consisting of *E. coli* DNA polymerase I (polA), *E. coli* Ribonuclease HI, *E. coli* Single-stranded DNA-binding protein, *E. coli* Replicative DNA helicase (DnaB), *E. coli* DNA replication protein (DnaC), *E. coli* DNA primase (DnaG), *E. coli* DNA Gyrase, and *E. coli* DNA polymerase III holoenzyme.

In some embodiments, the *E*. *coli-based* replisome comprises or consists of one or more of the proteins selected from the group comprising or consisting of *E. coli* Replicative DNA helicase (DnaB), *E. coli* DNA polymerase III holoenzyme, *E. coli* DNA primase (DnaG), and *E. coli* Single-stranded DNA-binding protein.

In some embodiments, the method for amplifying a DNA molecule according to the present invention is a Theta amplification method or a RCA method, preferably a Theta amplification method using *E*. *coli-based* replisome or a RCA method using *E. coli-*based replisome, more preferably a Theta amplification method using *E*. *coli*-based replisome. In some embodiments, the method for amplifying a DNA molecule according to the present invention comprises the following steps:
(i) initiation step comprising contacting the DNA molecule with a composition comprising at least one bacterial RNA polymerase, preferably RNAP, and optionally 1, 2 or 3 components selected from the group comprising or consisting of *E. coli* Ribonuclease HI, *E. coli* DNA polymerase I (polA) and *E. coli* Single-stranded DNA-binding protein;
(ii) fork progression step comprising contacting the product of step (i) with a composition comprising one or more components selected from the group comprising or consisting of *E. coli* DNA polymerase III holoenzyme, *E. coli* Replicative DNA helicase (DnaB), *E. coli* DNA replication protein (DnaC), *E. coli* DNA Gyrase, *E. coli* Single-stranded DNA-binding protein, and A. *coli* DNA primase (DnaG);
(iii) optionally, Okazaki maturation step comprising contacting the product of step (ii) with a composition comprising one or more components selected from the group comprising or consisting of *E. coli* Ribonuclease HI, *E. coli* DNA polymerase I (polA), and *E. coli* Protein DNA ligase; and
(iv) optionally, decatenation step comprising contacting the product of step (iii) with a composition comprising *E. coli* DNA topoisomerase III and/or *E. coli* DNA topoisomerase IV.

In some embodiments, steps (i), (ii), (iii) and (iv) are repeated any number of times, concomitantly or independently from each other.

In some embodiments, any of steps (i), (ii), (iii) and/or (iv) further comprise one or more component selected from the group comprising or consisting of *E. coli* DNA topoisomerase I, *E. coli* RpoE, *E. coli* RpoH, *E. coli* RpoS, *E. coli* ihfA, *E. coli* ihfB, *E*. *coli* diaA, *E. coli* RecA, *E. coli* RecBCD, *E. coli* RecQ, *E. coli* RecF, *E. coli* uvrD, *E. coli* HelD, and Rop.

In some embodiments, the method for amplifying a DNA molecule according to the present invention comprises contacting the DNA molecule with a first composition comprising at least one bacterial RNA polymerase, preferably RNAP, and a second composition comprising at least one phage T4-based replisome.

A phage T4-based replisome, or T4 replisome, as used herein, is an artificial or non-native replisome that replaces part of endogenous bacterial enzymes and/or proteins with components from phage T4. It is advantageous in that T4 replisome comprises less proteins and/or enzymes than natural bacterial replisomes, and is thus easier to manufacture and/or to use. Thus, T4 replisome is hereby interchangeably referred to as "simplified replisome".

In some embodiments, the phage T4-based replisome comprises or consists of one or more of the proteins selected from the group comprising or consisting of gp41 Helicase, gp59 Helicase loader, *E. coli* DNA Gyrase, GP32 SSB, gp61 Primase, gp45 clamp, gp44/62 Clamp loader, GP43 DNA Polymerase, phage T4 RNaseH, phage T4 Ligase, *E. coli* DNA topoisomerase III, *E. coli* DNA topoisomerase IV, *E. coli* Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the phage T4-based replisome comprises or consists of one or more of the proteins selected from the group comprising or consisting of gp41 Helicase, gp59 Helicase loader, *E. coli* DNA Gyrase, GP32 SSB, gp61 Primase, gp45 clamp, gp44/62 Clamp loader, GP43 DNA Polymerase, phage T4 RNaseH, phage T4 Ligase, *E. coli* DNA topoisomerase III, *E. coli* DNA topoisomerase IV, *E. coli* Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, and phage T4 DMA.

In some embodiments, the phage T4-based replisome comprises or consists of one or more of the proteins selected from the group comprising or consisting of gp41 Helicase, gp59 Helicase loader, *E. coli* DNA Gyrase, GP32 SSB, gp61 Primase, gp45 clamp, gp44/62 Clamp loader, GP43 DNA Polymerase, phage T4 RNaseH, phage T4 Ligase, *E. coli* DNA topoisomerase III, and *E. coli* DNA topoisomerase IV.

In some embodiments, the phage T4-based replisome comprises or consists of one or more of the proteins selected from the group comprising or consisting of gp41 Helicase, gp59 Helicase loader, *E. coli* DNA Gyrase, GP32 SSB, gp61 Primase, gp45 clamp, gp44/62 Clamp loader, and GP43 DNA Polymerase.

In some embodiments, the phage T4-based replisome comprises or consists of GP43 DNA polymerase having at least 75% sequence identity with SEQ ID NO: 19, and optionally one or more of the proteins selected from the group comprising gp41 Helicase having at least 75% sequence identity with SEQ ID NO: 25, gp59 Helicase loader having at least 75% sequence identity with SEQ ID NO: 26, *E. coli* DNA Gyrase, GP32 SSB having at least 75% sequence identity with SEQ ID NO: 40, gp61 Primase having at least 75% sequence identity with SEQ ID NO: 35, gp45 clamp having at least 75% sequence identity with SEQ ID NO: 36, gp44/62 Clamp loader having at least 75% sequence identity with SEQ ID NO: 37 or SEQ ID NO: 38, and variants thereof, or nucleic acids encoding thereof.

In some embodiments, the phage T4-based replisome comprises or consists of GP43 DNA polymerase having the sequence of SEQ ID NO: 19, and optionally one or more of the proteins selected from the group comprising gp41 Helicase having the sequence of SEQ ID NO: 25, gp59 Helicase loader having the sequence of SEQ ID NO: 26, *E. coli* DNA Gyrase, GP32 SSB having the sequence of SEQ ID NO: 40, gp61 Primase having the sequence of SEQ ID NO: 35, gp45 clamp having the sequence of SEQ ID NO: 36, gp44/62 Clamp loader having the sequence of SEQ ID NO: 37 or SEQ ID NO: 38, and variants thereof, or nucleic acids encoding thereof.

In a preferred embodiment, the T4-based replisome comprises or consists of replicase proteins, primosomal proteins, and Okazaki fragment-repair proteins. In some embodiments, the replicase proteins include the gp43 DNA polymerase, responsible for leading and lagging strand synthesis, the gp45 clamp, the ring shaped processivity factor involved in polymerase fidelity, and gp44/62 clamp loader, an AAA + ATPase responsible for opening gp45 for placement and removal on duplex DNA. In some embodiments, the primosomal proteins include the gp41 helicase, a hexameric 5' to 3' ATP dependent DNA helicase, the gp61 primase, a DNA dependent RNA polymerase responsible for synthesis of primers for lagging strand synthesis, the gp32 single stranded DNA binding protein, responsible for protection of single stranded DNA created by gp41 helicase activity, and the gp59 helicase loading protein, responsible for the loading of gp41 helicase onto gp32 protected ssDNA. In some embodiments, the repair of Okazaki fragments is accomplished by the RNase H, a 5' to 3' exonuclease, and gp30 ligase, the ATP dependent DNA ligase.

In some embodiments, the method for amplifying a DNA molecule according to the present invention is a Theta amplification method or a RCA method, preferably a Theta amplification method using phage T4-based replisome or a RCA method using phage T4-based replisome, more preferably a Theta amplification method using phage T4-based replisome. In some embodiments, the method for amplifying a DNA molecule according to the present invention comprises the following steps:
(i) initiation step comprising contacting the DNA molecule with a composition comprising at least one bacterial RNA polymerase, preferably RNAP, and optionally 1, 2 or 3 components selected from the group comprising or consisting of *E. coli* Ribonuclease HI, *E. coli* DNA polymerase I (polA) and *E. coli* Single-stranded DNA-binding protein;
(ii) fork progression step comprising contacting the product of step (i) with a composition comprising one or more components selected from the group comprising or consisting of gp41 Helicase, gp59 Helicase loader, *E. coli* DNA Gyrase, GP32 SSB, gp61 Primase, gp45 clamp, gp44/62 Clamp loader, and GP43 DNA Polymerase;
(iii) optionally, Okazaki maturation step comprising contacting the product of step (ii) with a composition comprising phage T4 RNaseH and/or phage T4 Ligase; and
(iv) optionally, decatenation step comprising contacting the product of step (iii) with a composition comprising *E. coli* DNA topoisomerase III and/or *E. coli* DNA topoisomerase IV.

In some embodiments, steps (i), (ii), (iii) and (iv) are repeated any number of times, concomitantly or independently from each other.

In some embodiments, any of steps (i), (ii), (iii) and/or (iv) further comprise one or more component selected from the group comprising or consisting of *E. coli* Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the method for amplifying a DNA molecule according to the present invention comprises contacting the DNA molecule with a first composition comprising at least one bacterial RNA polymerase, preferably RNAP, and a second composition comprising at least one phage T3-based replisome.

A phage T3-based replisome, or T3 replisome, as used herein, is an artificial or non-native replisome that replaces most of endogenous bacterial enzymes and/or proteins with components from phage T3. It is advantageous in that T3 replisome comprises even less proteins and/or enzymes than natural bacterial replisomes, and is thus easier to manufacture and/or to use. Thus, T3 replisome is hereby interchangeably referred to as "minimal replisome".

In some embodiments, the phage T3-based replisome comprises or consists of one or more of the proteins selected from the group comprising or consisting of phage T3 DNA helicase/primase, *E. coli* DNA Gyrase, phage T3 Single-stranded DNA-binding protein, phage T3 DNA polymerase (*i.e*., comprising T3 GP5 and Thioredoxin 1), phage T3 DNA ligase, *E. coli* DNA topoisomerase III, *E. coli* DNA topoisomerase IV, T3 endonuclease, T3 exonuclease, *E. coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the phage T3-based replisome comprises or consists of one or more of the proteins selected from the group comprising or consisting of phage T3 DNA helicase/primase, *E. coli* DNA Gyrase, phage T3 Single-stranded DNA-binding protein, phage T3 DNA polymerase (*i.e*., comprising T3 GP5 and Thioredoxin 1), phage T3 DNA ligase, *E. coli* DNA topoisomerase III, *E. coli* DNA topoisomerase IV, T3 endonuclease, and T3 exonuclease.

In some embodiments, the phage T3-based replisome comprises or consists of one or more of the proteins selected from the group comprising or consisting of phage T3 DNA helicase/primase, *E. coli* DNA Gyrase, phage T3 Single-stranded DNA-binding protein, phage T3 DNA polymerase (*i.e*., comprising T3 GP5 and Thioredoxin 1), phage T3 DNA ligase, *E. coli* DNA topoisomerase III, and *E. coli* DNA topoisomerase IV.

In some embodiments, the phage T3-based replisome comprises or consists of one or more of the proteins selected from the group comprising or consisting of phage T3 DNA helicase/primase, *E. coli* DNA Gyrase, phage T3 Single-stranded DNA-binding protein, phage T3 DNA polymerase (*i.e*., comprising T3 GP5 and Thioredoxin 1), and phage T3 DNA ligase.

In some embodiments, the phage T3-based replisome comprises or consists of one or more of the proteins selected from the group comprising or consisting of phage T3 DNA helicase/primase, *E. coli* DNA Gyrase, phage T3 Single-stranded DNA-binding protein, and phage T3 DNA polymerase (*i.e*., comprising T3 GP5 and Thioredoxin 1).

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, and optionally one or more of the proteins selected from the group comprising phage T3 DNA helicase/primase having at least 75% sequence identity with SEQ ID NO: 28, *E. coli* DNA Gyrase, Topoisomerase III, and phage T3 Single-stranded DNA-binding protein having at least 75% sequence identity with SEQ ID NO: 42, and variants thereof, or nucleic acids encoding thereof.

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, phage T3 Single-stranded DNA-binding protein having at least 75% sequence identity with SEQ ID NO: 42, and optionally one or more of the proteins selected from the group comprising phage T3 DNA helicase/primase having at least 75% sequence identity with SEQ ID NO: 28, Topoisomerase III and *E. coli* DNA Gyrase, and variants thereof, or nucleic acids encoding thereof.

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, phage T3 Single-stranded DNA-binding protein having at least 75% sequence identity with SEQ ID NO: 42, phage T3 DNA helicase/primase having at least 75% sequence identity with SEQ ID NO: 28, and optionally Topoisomerase III and *E*. *coli* DNA Gyrase, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, and optionally one or more of the proteins selected from the group comprising phage T3 DNA helicase/primase having the sequence of SEQ ID NO: 28, *E. coli* DNA Gyrase, Topoisomerase III and phage T3 Single-stranded DNA-binding protein having the sequence of SEQ ID NO: 42, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, phage T3 Single-stranded DNA-binding protein having the sequence of SEQ ID NO: 42, and optionally one or more of the proteins selected from the group comprising phage T3 DNA helicase/primase having the sequence of SEQ ID NO: 28, Topoisomerase III and *E. coli* DNA Gyrase, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, phage T3 Single-stranded DNA-binding protein having the sequence of SEQ ID NO: 42, phage T3 DNA helicase/primase having the sequence of SEQ ID NO: 28, and optionally Topoisomerase III and *E. coli* DNA Gyrase, and variants thereof, or nucleic acids encoding thereof.

In some embodiments, the method for amplifying a DNA molecule according to the present invention is a Theta amplification method or a RCA method, preferably a Theta amplification method using phage T3-based replisome or a RCA method using phage T3-based replisome, more preferably a Theta amplification method using phage T3-based replisome. In some embodiments, the method for amplifying a DNA molecule according to the present invention comprises the following steps:
(i) initiation step comprising contacting the DNA molecule with a composition comprising at least one bacterial RNA polymerase, preferably RNAP, and optionally 1, 2 or 3 components selected from the group comprising or consisting of *E. coli* Ribonuclease HI, *E. coli* DNA polymerase I (polA) and *E. coli* Single-stranded DNA-binding protein;
(ii) fork progression step comprising contacting the product of step (i) with a composition comprising one or more components selected from the group comprising or consisting of phage T3 DNA helicase/primase, *E. coli* DNA Gyrase, phage T3 Single-stranded DNA-binding protein, and phage T3 DNA polymerase (*i.e.*, comprising T3 GP5 and Thioredoxin 1);
(iii) optionally, Okazaki maturation step comprising contacting the product of step (ii) with a composition comprising phage T3 DNA ligase; and
(iv) optionally, decatenation step comprising contacting the product of step (iii) with a composition comprising *E. coli* DNA topoisomerase III and/or *E. coli* DNA topoisomerase IV.

In some embodiments, steps (i), (ii), (iii) and (iv) are repeated any number of times, concomitantly or independently from each other.

In some embodiments, any of steps (i), (ii), (iii) and/or (iv) further comprise one or more component selected from the group comprising or consisting of T3 endonuclease, T3 exonuclease, *E. coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the method for amplifying a DNA molecule according to the present invention comprises contacting the DNA molecule with a first composition comprising at least one bacterial RNA polymerase, preferably RNAP, and a second composition comprising at least one phage T7-based replisome.

A phage T7-based replisome, or T7 replisome, as used herein, is an artificial or non-native replisome that replaces most of endogenous bacterial enzymes and/or proteins with components from phage T7. It is advantageous in that T7 replisome comprises even less proteins and/or enzymes than natural bacterial replisomes, and is thus easier to manufacture and/or to use. Thus, T7 replisome is hereby interchangeably referred to as "minimal replisome".

In some embodiments, the phage T7-based replisome comprises or consists of one or more of the proteins selected from the group comprising or consisting of phage T7 DNA helicase/primase, *E. coli* DNA Gyrase, phage T7 Single-stranded DNA-binding protein, phage T7 DNA polymerase (*i.e*., comprising T7 GP5 and Thioredoxin 1), T7 DNA ligase, *E. coli* DNA topoisomerase III, *E. coli* DNA topoisomerase IV, T7 endonuclease, T7 exonuclease, *E. coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the phage T7-based replisome comprises or consists of one or more of the proteins selected from the group comprising or consisting of phage T7 DNA helicase/primase, *E. coli* DNA Gyrase, phage T7 Single-stranded DNA-binding protein, phage T7 DNA polymerase (*i.e*., comprising T7 GP5 and Thioredoxin 1), T7 DNA ligase, *E. coli* DNA topoisomerase III, *E. coli* DNA topoisomerase IV, T7 endonuclease, and T7 exonuclease.

In some embodiments, the phage T7-based replisome comprises or consists of one or more of the proteins selected from the group comprising or consisting of phage T7 DNA helicase/primase, *E. coli* DNA Gyrase, phage T7 Single-stranded DNA-binding protein, phage T7 DNA polymerase (*i.e.*, comprising T7 GP5 and Thioredoxin 1), T7 DNA ligase, *E. coli* DNA topoisomerase III, and *E. coli* DNA topoisomerase IV.

In some embodiments, the phage T7-based replisome comprises or consists of one or more of the proteins selected from the group comprising or consisting of phage T7 DNA helicase/primase, *E. coli* DNA Gyrase, phage T7 Single-stranded DNA-binding protein, phage T7 DNA polymerase (*i.e.,* comprising T7 GP5 and Thioredoxin 1), and T7 DNA ligase.

In some embodiments, the phage T7-based replisome comprises or consists of one or more of the proteins selected from the group comprising or consisting of phage T7 DNA helicase/primase, *E. coli* DNA Gyrase, phage T7 Single-stranded DNA-binding protein, and phage T7 DNA polymerase (*i.e*., comprising T7 GP5 and Thioredoxin 1).

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, and optionally one or more of the proteins selected from the group comprising phage T7 DNA helicase/primase having at least 75% sequence identity with SEQ ID NO: 27, *E. coli* DNA Gyrase, Topoisomerase III and phage T7 Single-stranded DNA-binding protein having at least 75% sequence identity with SEQ ID NO: 41, and variants thereof, or nucleic acids encoding thereof.

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, phage T7 Single-stranded DNA-binding protein having at least 75% sequence identity with SEQ ID NO: 41, and optionally one or more of the proteins selected from the group comprising phage T7 DNA helicase/primase having at least 75% sequence identity with SEQ ID NO: 27, Topoisomerase III, *E. coli* DNA Gyrase, and variants thereof, or nucleic acids encoding thereof.

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, phage T7 Single-stranded DNA-binding protein having at least 75% sequence identity with SEQ ID NO: 41, phage T7 DNA helicase/primase having at least 75% sequence identity with SEQ ID NO: 27, Topoisomerase III and optionally *E. coli* DNA Gyrase, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, and optionally one or more of the proteins selected from the group comprising phage T7 DNA helicase/primase having the sequence of SEQ ID NO: 27, *E. coli* DNA Gyrase, Topoisomerase III and phage T7 Single-stranded DNA-binding protein having the sequence of SEQ ID NO: 41, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, phage T7 Single-stranded DNA-binding protein having the sequence of SEQ ID NO: 41, and optionally one or more of the proteins selected from the group comprising phage T7 DNA helicase/primase having the sequence of SEQ ID NO: 27, Topoisomerase III, *E. coli* DNA Gyrase, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, phage T7 Single-stranded DNA-binding protein having the sequence of SEQ ID NO: 41, phage T7 DNA helicase/primase having the sequence of SEQ ID NO: 27, and optionally Topoisomerase III, *E. coli* DNA Gyrase, and variants thereof, or nucleic acids encoding thereof.

In some embodiments, the method for amplifying a DNA molecule according to the present invention is a Theta amplification method or a RCA method, preferably a Theta amplification method using phage T7-based replisome or a RCA method using phage T7-based replisome, more preferably a Theta amplification method using phage T7-based replisome. In some embodiments, the method for amplifying a DNA molecule according to the present invention comprises the following steps:
(i) initiation step comprising contacting the DNA molecule with a composition comprising at least one bacterial RNA polymerase, preferably RNAP, and optionally 1, 2 or 3 components selected from the group comprising or consisting of *E. coli* Ribonuclease HI, *E. coli* DNA polymerase I (polA) and *E. coli* Single-stranded DNA-binding protein;
(ii) fork progression step comprising contacting the product of step (i) with a composition comprising one or more components selected from the group comprising or consisting of phage T7 DNA helicase/primase, *E. coli* DNA Gyrase, phage T7 Single-stranded DNA-binding protein, and phage T7 DNA polymerase (*i.e*., comprising T7 GP5 and Thioredoxin 1);
(iii) optionally, Okazaki maturation step comprising contacting the product of step (ii) with a composition comprising T7 DNA ligase; and
(iv) optionally, decatenation step comprising contacting the product of step (iii) with a composition comprising *E. coli* DNA topoisomerase III and/or *E. coli* DNA topoisomerase IV.

In some embodiments, steps (i), (ii), (iii) and (iv) are repeated any number of times, concomitantly or independently from each other.

In some embodiments, any of steps (i), (ii), (iii) and/or (iv) further comprise one or more component selected from the group comprising or consisting of T7 endonuclease, T7 exonuclease, *E. coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In certain embodiments, the replisome may be a T3/T7-based replisome, thus in some embodiments, the replisome may comprise one or more components from T3 replisome and T7 replisome. T3/T7-based replisome is also a minimal replisome.

The present invention relates to a method for amplifying a DNA molecule, comprising contacting the DNA molecule with a composition comprising at least one bacterial RNA polymerase and at least one DNA polymerase, as defined herein.

In some embodiments, the composition comprises at least one bacterial RNA polymerase, at least one DNA polymerase, and one or more component selected from the group comprising or consisting of DNA helicases and helicase associated proteins, sliding clamps, DNA binding proteins, DNA ligases, gyrases, topoisomerases, primases, RNAses, RNA polymerases, integration host factors, DNA maintenance proteins, regulators of initiation, repressors, nucleases, recombinases, polynucleotide kinases, and methyl transferases.

In some embodiments, the at least one bacterial RNA polymerase is RNAP. In some embodiments, the at least one DNA polymerase is Phi29 DNA polymerase. In some embodiments, the at least one bacterial RNA polymerase is RNAP and the at least one DNA polymerase is Phi29 DNA polymerase.

In some embodiments, the method comprises contacting the DNA molecule with RNAP and Phi29 DNA polymerase.

In some embodiments, the at least one bacterial RNA polymerase is RNAP. In some embodiments, the at least one DNA polymerase is *E. coli* DNA polymerase III holoenzyme. In some embodiments, the one or more components are selected from the group comprising or consisting of *E. coli* DNA polymerase I (polA), *E. coli* Ribonuclease HI, *E. coli* Single-stranded DNA-binding protein, *E. coli* Replicative DNA helicase (DnaB), *E. coli* DNA replication protein (DnaC), *E. coli* DNA primase (DnaG), *E. coli* DNA Gyrase, *E. coli* Protein DNA ligase, *E. coli* DNA topoisomerase III, *E. coli* DNA topoisomerase IV, *E*. *coli* DNA topoisomerase I, *E*. *coli* RpoE, *E. coli* RpoH, *E. coli* RpoS*, E. coli* ihfA, *E. coli* ihfB, *E. coli* diaA, *E. coli* RecA, *E. coli* RecBCD, *E. coli* RecQ, *E*. *coli* RecF, *E. coli* uvrD, *E. coli* HelD, and Rop.

In some embodiments, the method comprises contacting the DNA molecule with a composition comprising RNAP, *E. coli* DNA polymerase III and one or more of *E. coli* Ribonuclease HI, *E. coli* DNA polymerase I (polA), *E. coli* Single-stranded DNA-binding protein, *E. coli* Replicative DNA helicase (DnaB), *E. coli* DNA replication protein (DnaC), *E. coli* DNA Gyrase, *E. coli* Single-stranded DNA-binding protein, *E. coli* DNA primase (DnaG), *E. coli* Ribonuclease HI, *E. coli* DNA polymerase I (polA), *E. coli* Protein DNA ligase, *E. coli* DNA topoisomerase III and/or *E. coli* DNA topoisomerase IV.

In some embodiments, the at least one bacterial RNA polymerase is RNAP. In some embodiments, the at least one DNA polymerase is GP43 DNA Polymerase. In some embodiments, the one or more components are selected from the group comprising or consisting of gp41 Helicase, gp59 Helicase loader, *E. coli* DNA Gyrase, GP32 SSB, gp61 Primase, gp45 clamp, gp44/62 Clamp loader, phage T4 RNaseH, phage T4 Ligase, *E. coli* DNA topoisomerase III, *E. coli* DNA topoisomerase IV, *E. coli* Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, *E. coli* DNA topoisomerase I, *E. coli* RpoE, *E*. *coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E. coli* HelD.

In some embodiments, the method comprises contacting the DNA molecule with a composition comprising RNAP, GP43 DNA Polymerase, and one or more of *E. coli* Ribonuclease HI, *E. coli* DNA polymerase I (polA), *E. coli* Single-stranded DNA-binding protein, gp41 Helicase, gp59 Helicase loader, *E. coli* DNA Gyrase, GP32 SSB, gp61 Primase, gp45 clamp, gp44/62 Clamp loader, phage T4 RNaseH, phage T4 Ligase, *E. coli* DNA topoisomerase III and/or *E. coli* DNA topoisomerase IV.

In some embodiments, the at least one bacterial RNA polymerase is RNAP. In some embodiments, the at least one DNA polymerase is phage T3 DNA polymerase. In some embodiments, the one or more components are selected from the group comprising or consisting of phage T3 DNA helicase/primase, *E. coli* DNA Gyrase, phage T3 Single-stranded DNA-binding protein, phage T3 DNA ligase, *E. coli* DNA topoisomerase III, *E. coli* DNA topoisomerase IV, T3 endonuclease, T3 exonuclease, *E. coli* DNA ligase, *E*. *coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and *E*. *coli* HelD.

In some embodiments, the method comprises contacting the DNA molecule with a composition comprising RNAP, phage T3 DNA polymerase, and one or more of *E. coli* Ribonuclease HI, *E. coli* DNA polymerase I (polA), *E. coli* Single-stranded DNA-binding protein, phage T3 DNA helicase/primase, *E. coli* DNA Gyrase, phage T3 Single-stranded DNA-binding protein, phage T3 DNA ligase, *E. coli* DNA topoisomerase III and/or *E*. *coli* DNA topoisomerase IV.

In some embodiments, the at least one bacterial RNA polymerase is RNAP. In some embodiments, the at least one DNA polymerase is phage T7 DNA polymerase. In some embodiments, the one or more components are selected from the group comprising or consisting of phage T7 DNA helicase/primase, *E. coli* DNA Gyrase, phage T7 Single-stranded DNA-binding protein, phage T7 DNA ligase, *E. coli* DNA topoisomerase III, *E. coli* DNA topoisomerase IV, T7 endonuclease, T7 exonuclease, *E. coli* DNA ligase, *E*. *coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E. coli* RecF, *E. coli* uvrD, and A. *coli* HelD.

In some embodiments, the method comprises contacting the DNA molecule with a composition comprising RNAP, phage T7 DNA polymerase, and one or more of *E. coli* Ribonuclease HI, *E. coli* DNA polymerase I (polA), *E. coli* Single-stranded DNA-binding protein, phage T7 DNA helicase/primase, *E. coli* DNA Gyrase, phage T7 Single-stranded DNA-binding protein, phage T7 DNA ligase, *E. coli* DNA topoisomerase III and/or *E*. *coli* DNA topoisomerase IV.

The present invention further relates to a phage T4-based replisome comprising GP43 DNA Polymerase, and one or more of the proteins selected from the group comprising or consisting of gp41 Helicase, gp59 Helicase loader, *E. coli* DNA Gyrase, GP32 SSB, gp61 Primase, gp45 clamp, gp44/62 Clamp loader, phage T4 RNaseH, phage T4 Ligase, *E. coli* DNA topoisomerase III, E. coli DNA topoisomerase IV, *E. coli* Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, *E. coli* DNA topoisomerase I, *E. coli* RpoE, *E. coli* RpoH, E. coli RpoS, *E. coli* ihfA, *E. coli* ihfB, *E. coli* diaA, *E. coli* RecA, *E. coli* RecBCD, E. coli RecQ, *E. coli* RecF, *E. coli* uvrD, and *E. coli* HelD, or nucleic acids encoding thereof.

In some embodiments, the phage T4-based replisome comprises or consists of GP43 DNA polymerase having at least 75% sequence identity with SEQ ID NO: 19, and optionally one or more of the proteins selected from the group comprising gp41 Helicase having at least 75% sequence identity with SEQ ID NO: 25, gp59 Helicase loader having at least 75% sequence identity with SEQ ID NO: 26, *E. coli* DNA Gyrase, GP32 SSB having at least 75% sequence identity with SEQ ID NO: 40, gp61 Primase having at least 75% sequence identity with SEQ ID NO: 35, gp45 clamp having at least 75% sequence identity with SEQ ID NO: 36, gp44/62 Clamp loader having at least 75% sequence identity with SEQ ID NO: 37 or SEQ ID NO: 38, and variants thereof, or nucleic acids encoding thereof.

In some embodiments, the phage T4-based replisome comprises or consists of GP43 DNA polymerase having the sequence of SEQ ID NO: 19, and optionally one or more of the proteins selected from the group comprising gp41 Helicase having the sequence of SEQ ID NO: 25, gp59 Helicase loader having the sequence of SEQ ID NO: 26, *E. coli* DNA Gyrase, GP32 SSB having the sequence of SEQ ID NO: 40, gp61 Primase having the sequence of SEQ ID NO: 35, gp45 clamp having the sequence of SEQ ID NO: 36, gp44/62 Clamp loader having the sequence of SEQ ID NO: 37 or SEQ ID NO: 38, and variants thereof, or nucleic acids encoding thereof.

In a preferred embodiment, the T4-based replisome comprises or consists of replicase proteins, primosomal proteins, and Okazaki fragment-repair proteins. In some embodiments, the replicase proteins include the gp43 DNA polymerase, responsible for leading and lagging strand synthesis, the gp45 clamp, the ring shaped processivity factor involved in polymerase fidelity, and gp44/62 clamp loader, an AAA + ATPase responsible for opening gp45 for placement and removal on duplex DNA. In some embodiments, the primosomal proteins include the gp41 helicase, a hexameric 5' to 3' ATP dependent DNA helicase, the gp61 primase, a DNA dependent RNA polymerase responsible for synthesis of primers for lagging strand synthesis, the gp32 single stranded DNA binding protein, responsible for protection of single stranded DNA created by gp41 helicase activity, and the gp59 helicase loading protein, responsible for the loading of gp41 helicase onto gp32 protected ssDNA. In some embodiments, the repair of Okazaki fragments is accomplished by the RNase H, a 5' to 3' exonuclease, and gp30 ligase, the ATP dependent DNA ligase.

The present invention further relates to a phage T3-based replisome comprising T3 GP5 and Thioredoxin 1, and one or more of the proteins selected from the group comprising or consisting of phage T3 DNA helicase/primase, *E. coli* DNA Gyrase, phage T3 Single-stranded DNA-binding protein, phage T3 DNA ligase, *E. coli* DNA topoisomerase III, *E. coli* DNA topoisomerase IV, T3 endonuclease, T3 exonuclease, *E*. *coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E*. *coli* RecF, *E. coli* uvrD, and *E. coli* HelD, or nucleic acids encoding thereof.

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, and optionally one or more of the proteins selected from the group comprising phage T3 DNA helicase/primase having at least 75% sequence identity with SEQ ID NO: 28, *E. coli* DNA Gyrase, *E. coli* DNA topoisomerase III, and phage T3 Single-stranded DNA-binding protein having at least 75% sequence identity with SEQ ID NO: 42, and variants thereof, or nucleic acids encoding thereof.

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, phage T3 Single-stranded DNA-binding protein having at least 75% sequence identity with SEQ ID NO: 42, and optionally one or more of the proteins selected from the group comprising phage T3 DNA helicase/primase having at least 75% sequence identity with SEQ ID NO: 28, *E. coli* DNA topoisomerase III, and *E. coli* DNA Gyrase, and variants thereof, or nucleic acids encoding thereof.

In some embodiments, the phage T3-based replisome comprises or consists of GP5 DNA polymerase having at least 75% sequence identity with SEQ ID NO: 20, Thioredoxin 1 having at least 75% sequence identity with SEQ ID NO: 22, phage T3 Single-stranded DNA-binding protein having at least 75% sequence identity with SEQ ID NO: 42, phage T3 DNA helicase/primase having at least 75% sequence identity with SEQ ID NO: 28, and optionally *E. coli* DNA topoisomerase III, *E. coli* DNA Gyrase, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, and optionally one or more of the proteins selected from the group comprising phage T3 DNA helicase/primase having the sequence of SEQ ID NO: 28, *E. coli* DNA Gyrase, *E. coli* DNA topoisomerase III, and phage T3 Single-stranded DNA-binding protein having the sequence of SEQ ID NO: 42, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, phage T3 Single-stranded DNA-binding protein having the sequence of SEQ ID NO: 42, and optionally one or more of the proteins selected from the group comprising phage T3 DNA helicase/primase having the sequence of SEQ ID NO: 28, *E. coli* DNA topoisomerase III, and *E. coli* DNA Gyrase, and variants thereof, or nucleic acids encoding thereof.

In some embodiments, the phage T3-based replisome comprises or consists of GP5 DNA polymerase having the sequence of SEQ ID NO: 20, Thioredoxin 1 having the sequence of SEQ ID NO: 22, phage T3 Single-stranded DNA-binding protein having the sequence of SEQ ID NO: 42, phage T3 DNA helicase/primase having the sequence of SEQ ID NO: 28, and optionally *E. coli* DNA topoisomerase III, *E. coli* DNA Gyrase, and variants thereof, or nucleic acids encoding thereof.

The present invention further relates to a phage T7-based replisome comprising T7 GP5 and Thioredoxin 1, and one or more of the proteins selected from the group comprising or consisting of phage T7 DNA helicase/primase, *E. coli* DNA Gyrase, phage T7 Single-stranded DNA-binding protein, phage T7 DNA ligase, *E. coli* DNA topoisomerase III, *E. coli* DNA topoisomerase IV, T7 endonuclease, T7 exonuclease, *E*. *coli* DNA ligase, *E. coli* DNA topoisomerase I, *E. coli* RpoE, *E. coli* RpoH, *E. coli* RpoS, *E. coli* ihfA, *E. coli* ihfB, *E. coli* diaA, *E. coli* RecA, *E. coli* RecBCD, *E. coli* RecQ, *E*. *coli* RecF, *E. coli* uvrD, and *E. coli* HelD, or nucleic acids encoding thereof.

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, and optionally one or more of the proteins selected from the group comprising phage T7 DNA helicase/primase having at least 75% sequence identity with SEQ ID NO: 27, *E. coli* DNA topoisomerase III, *E. coli* DNA Gyrase, and phage T7 Single-stranded DNA-binding protein having at least 75% sequence identity with SEQ ID NO: 41, and variants thereof, or nucleic acids encoding thereof.

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, phage T7 Single-stranded DNA-binding protein having at least 75% sequence identity with SEQ ID NO: 41, and optionally one or more of the proteins selected from the group comprising phage T7 DNA helicase/primase having at least 75% sequence identity with SEQ ID NO: 27, *E. coli* DNA topoisomerase III, *E. coli* DNA Gyrase, and variants thereof, or nucleic acids encoding thereof.

In some embodiments, the phage T7-based replisome comprises or consists of GP5 DNA polymerase having at least 75% sequence identity with SEQ ID NO: 21, Thioredoxin 1 having at least 75% sequence identity with SEQ ID NO: 22, phage T7 Single-stranded DNA-binding protein having at least 75% sequence identity with SEQ ID NO: 41, phage T7 DNA helicase/primase having at least 75% sequence identity with SEQ ID NO: 27, and optionally *E. coli* DNA topoisomerase III, *E. coli* DNA Gyrase, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, and optionally one or more of the proteins selected from the group comprising phage T7 DNA helicase/primase having the sequence of SEQ ID NO: 27, *E. coli* DNA Gyrase, *E. coli* DNA topoisomerase III, and phage T7 Single-stranded DNA-binding protein having the sequence of SEQ ID NO: 41, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, phage T7 Single-stranded DNA-binding protein having the sequence of SEQ ID NO: 41, and optionally one or more of the proteins selected from the group comprising phage T7 DNA helicase/primase having the sequence of SEQ ID NO: 27, *E. coli* DNA topoisomerase III, *E. coli* DNA Gyrase, and variants thereof, or nucleic acids encoding thereof.

In some embodiments, the phage T7-based replisome comprises or consists of GP5 DNA polymerase having the sequence of SEQ ID NO: 21, Thioredoxin 1 having the sequence of SEQ ID NO: 22, phage T7 Single-stranded DNA-binding protein having the sequence of SEQ ID NO: 41, phage T7 DNA helicase/primase having the sequence of SEQ ID NO: 27, and optionally *E. coli* DNA topoisomerase III, *E. coli* DNA Gyrase, and variants thereof, or nucleic acids encoding thereof.

The present invention further relates to a method for initiating replication of a DNA molecule, comprising contacting the DNA molecule with a composition comprising at least one bacterial RNA polymerase as described herein; wherein the DNA molecule comprises at least one ColE1-like origin of replication.

In some embodiments, the replication process is isothermal.

The present invention further relates to a method for amplifying a DNA molecule after initiation of DNA replication, preferably immediately after initiation of DNA replication, comprising the DNA molecule with a composition comprising at least one DNA polymerase as described herein; wherein the DNA molecule comprises at least one ColE1-like origin of replication.

In some embodiments, the amplification process is isothermal.

The present invention further relates to a composition comprising a Phi29 DNA polymerase as defined herein, or nucleic acids encoding thereof.

The present invention further relates to a composition comprising a *E. coli*-based replisome as defined herein, or nucleic acids encoding thereof.

The present invention further relates to a composition comprising a phage T4-based replisome as defined herein, or nucleic acids encoding thereof. The present invention further relates to a composition comprising a phage T4-based replisome as described herein, and one or more components selected from the group comprising or consisting of a buffer, rNTPs and dNTPs.

The present invention further relates to a composition comprising a phage T3-based replisome as defined herein, or nucleic acids encoding thereof. The present invention further relates to a composition comprising a phage T3-based replisome as described herein, and one or more components selected from the group comprising or consisting of a buffer, rNTPs and dNTPs.

The present invention further relates to a composition comprising a phage T7-based replisome as defined herein, or nucleic acids encoding thereof. The present invention further relates to a composition comprising a phage T7-based replisome as described herein, and one or more components selected from the group comprising or consisting of a buffer, rNTPs and dNTPs.

The present invention further relates to a composition comprising a phage T3/T7-based replisome as defined herein, or nucleic acids encoding thereof.

The present invention further relates to a composition comprising at least one Phi29 DNA polymerase as defined herein and/or at least one replisome as defined herein, and at least one bacterial RNA polymerase as defined herein, or nucleic acids encoding thereof.

The present invention further relates to the use of one or more composition according to the invention.

The present invention further relates to the use of a composition comprising at least one Phi29 DNA polymerase according to the invention, preferably at least one Phi29 DNA polymerase according to the invention, in combination with a composition comprising at least one bacterial RNA polymerase as described herein, for use for amplifying a DNA molecule *in vitro,* wherein the DNA molecule comprises at least one ColE1-like origin of replication. In some embodiments, amplifying the DNA molecule comprises at least one step of RCA.

The present invention further relates to the use of a composition comprising at least one replisome according to the invention, preferably at least one replisome according to the invention, in combination with a composition comprising at least one bacterial RNA polymerase as described herein, for use for amplifying a DNA molecule *in vitro,* wherein the DNA molecule comprises at least one ColE1-like origin of replication. In some embodiments, amplifying the DNA molecule comprises at least one step of Theta amplification.

The present invention further relates to a kit comprising or consisting of:
- a composition comprising at least one DNA polymerase as defined herein, or nucleic acids encoding thereof;
- a composition comprising at least one bacterial RNA polymerase as defined herein, or a nucleic acid encoding thereof; and
- instructions for use.

In some embodiments, the kit further comprises one or more protein or enzyme useful for DNA amplification as described herein.

The present invention further relates to a kit comprising or consisting of:
- a composition comprising at least one replisome as defined herein, or nucleic acids encoding thereof;
- a composition comprising at least one bacterial RNA polymerase as defined herein, or a nucleic acid encoding thereof; and
- instructions for use.

In some embodiments, the kit further comprises one or more protein or enzyme useful for DNA amplification as described herein.

The present invention further relates to a kit comprising or consisting of:
- a composition comprising at least one Phi29 DNA polymerase as defined herein, or nucleic acids encoding thereof;
- a composition comprising at least one bacterial RNA polymerase as defined herein, or a nucleic acid encoding thereof; and
- instructions for use.

In some embodiments, the kit further comprises one or more protein or enzyme useful for DNA amplification as described herein.

The present invention further relates to the one or more DNA molecule obtained by the method according to the invention.

In some embodiments, the one or more DNA molecule obtained by the method according to the invention is linearized. In some embodiments, the one or more DNA molecule obtained by the method according to the invention is linearized by a restriction enzyme.

In some embodiments, the one or more DNA molecule obtained by the method according to the invention, preferably the linear one or more DNA molecule obtained by the method according to the invention, is covalently closed, *i.e.,* the one or more DNA molecule obtained by the method according to the invention is a LCC DNA molecule. In some embodiments, the one or more DNA molecule obtained by the method according to the invention, preferably the linear one or more DNA molecule obtained by the method according to the invention, is covalently closed by a protelomerase, preferably TelN.

The sequences disclosed herein are recapitulated in **Table 1:**

| **ID** | **SEQ ID NO** | **Sequence (amino acids)** |
|---|---|---|
| rpoA | 1 | |
| | | |
| rpoB | **2** | |
| | | |
| rpoC | **3** | |
| | | |
| rpoZ | 4 | |
| rpoD | 5 | MEQNPQSQLKLLVTRGKEQGYLTYAEVNDHLPEDIVDSDQI EDIIQMINDMGIQVMEEAPDADDLMLAENTADEDAAEAAA QVLSSVESEIGRTTDPVRMYMREMGTVELLTREGEIDIAKRI EDGINQVQCSVAEYPEAITYLLEQYDRVEAEEARLSDLITGF VDPNAEEDLAPTATHVGSELSQEDLDDDEDEDEEDGDDDSA DDDNSIDPELAREKFAELRAQYVVTRDTIKAKGRSHATAQE EILKLSEVFKQFRLVPKQFDYLVNSMRVMMDRVRTQERLIM KLCVEQCKMPKKNFITLFTGNETSDTWFNAAIAMNKPWSEK LHDVSEEVHRALQKLQQIEEETGLTIEQVKDINRRMSIGEAK ARRAKKEMVEANLRLVISIAKKYTNRGLQFLDLIQEGNIGLM KAVDKFEYRRGYKFSTYATWWIRQAITRSIADQARTIRIPVH MIETINKLNRISRQMLQEMGREPTPEELAERMLMPEDKIRKV LKIAKEPISMETPIGDDEDSHLGDFIEDTTLELPLDSATTESLR AATHDVLAGLTAREAKVLRMRFGIDMNTDYTLEEVGKQFD VTRERIRQIEAKALRKLRHPSRSEVLRSFLDD |
| Phi29 DNA polyme rase | 6 | |
| | | |
| dnaN/D PO3B | 7 | MKFTVEREHLLKPLQQVSGPLGGRPTLPILGNLLLQVADGTL SLTGTDLEMEMVARVALVQPHEPGATTVPARKFFDICRGLP EGAEIAVQLEGERMLVRSGRSRFSLSTLPAADFPNLDDWQSE VEFTLPQATMKRLIEATQFSMAHQDVRYYLNGMLFETEGEE LRTVATDGHRLAVCSMPIGQSLPSHSVIVPRKGVIELMRMLD GGDNPLRVQIGSNNIRAHVGDFIFTSKLVDGRFPDYRRVLPK NPDKHLEAGCDLLKQAFARAAILSNEKFRGVRLYVSENQLK ITANNPEQEEAEEILDVTYSGAEMEIGFNVSYVLDVLNALKC ENVRMMLTDSVSSVQIEDAASQSAAYVVMPMRL |
| holA | 8 | MIRLYPEQLRAQLNEGLRAAYLLLGNDPLLLQESQDAVRQV AAAQGFEEHHTFSIDPNTDWNAIFSLCQAMSLFASRQTLLLL LPENGPNAAINEQLLTLTGLLHDDLLLIVRGNKLSKAQENAA WFTALANRSVQVTCQTPEQAQLPRWVAARAKQLNLELDDA ANQVLCYCYEGNLLALAQALERLSLLWPDGKLTLPRVEQA VNDAAHFTPFHWVDALLMGKSKRALHILQQLRLEGSEPVIL LRTLQRELLLLVNLKRQSAHTPLRALFDKHRVWQNRRGMM GEALNRLSQTQLRQAVQLLTRTELTLKQDYGQSVWAELEGL SLLLCHKPLADVFIDG |
| holB | 9 | MRWYPWLRPDFEKLVASYQAGRGHHALLIQALPGMGDDA LIYALSRYLLCQQPQGHKSCGHCRGCQLMQAGTHPDYYTL APEKGKNTLGVDAVREVTEKLNEHARLGGAKVVWVTDAA LLTDAAANALLKTLEEPPAETWFFLATREPERLLATLRSRCR LHYLAPPPEQYAVTWLSREVTMSQDALLAALRLSAGSPGAA LALFQGDNWQARETLCQALAYSVPSGDWYSLLAALNHEQA PARLHWLATLLMDALKRHHGAAQVTNVDVPGLVAELANH LSPSRLQAILGDVCHIREQLMSVTGINRELLITDLLLRIEHYLQ PGVVLPVPHL |
| holC | 10 | MKNATFYLLDNDTTVDGLSAVEQLVCEIAAERWRSGKRVLI ACEDEKQAYRLDEALWARPAESFVPHNLAGEGPRGGAPVEI AWPQKRSSSRRDILISLRTSFADFATAFTEVVDFVPYEDSLKQ LARERYKAYRVAGFNLNTATWK |
| holD | 11 | MTSRRDWQLQQLGITQWSLRRPGALQGEIAIAIPAHVRLVM VANDLPALTDPLVSDVLRALTVSPDQVLQLTPEKIAMLPQGS HCNSWRLGTDEPLSLEGAQVASPALTDLRANPTARAALWQ QICTYEHDFFPRND |
| holE | 12 | MLKNLAKLDQTEMDKVNVDLAAAGVAFKERYNMPVIAEA VEREQPEHLRSWFRERLIAHRLASVNLSRLPYEPKLK |
| dnaE/D PO3A | 13 | MSEPRFVHLRVHSDYSMIDGLAKTAPLVKKAAALGMPALAI TDFTNLCGLVKFYGAGHGAGIKPIVGADFNVQCDLLGDELT HLTVLAANNTGYQNLTLLISKAYQRGYGAAGPIIDRDWLIEL NEGLILLSGGRMGDVGRSLLRGNSALVDECVAFYEEHFPDR YFLELIRTGRPDEESYLHAAVELAEARGLPVVATNDVRFIDS SDFDAHEIRVAIHDGFTLDDPKRPRNYSPQQYMRSEEEMCEL FADIPEALANTVEIAKRCNVTVRLGEYFLPQFPTGDMSTEDY LVKRAKEGLEERLAFLFPDEEERLKRRPEYDERLETELQVIN QMGFPGYFLIVMEFIQWSKDNGVPVGPGRGSGAGSLVAYAL |
| | | KITDLDPLEFDLLFERFLNPERVSMPDFDVDFCMEKRDQVIE HVADMYGRDAVSQIITFGTMAAKAVIRDVGRVLGHPYGFV DRISKLIPPDPGMTLAKAFEAEPQLPEIYEADEEVKALIDMAR KLEGVTRNAGKHAGGVVIAPTKITDFAPLYCDEEGKHPVTQ FDKSDVEYAGLVKFDFLGLRTLTIINWALEMINKRRAKNGEP PLDIAAIPLDDKKSFDMLQRSETTAVFQLESRGMKDLIKRLQ PDCFEDMIALVALFRPGPLQSGMVDNFIDRKHGREEISYPDV QWQHESLKPVLEPTYGIILYQEQVMQIAQVLSGYTLGGADM LRRAMGKKKPEEMAKQRSVFAEGAEKNGINAELAMKIFDL VEKFAGYGFNKSHSAAYALVSYQTLWLKAHYPAEFMAAV MTADMDNTEKVVGLVDECWRMGLKILPPDINSGLYHFHVN DDGEIVYGIGAIKGVGEGPIEAIIEARNKGGYFRELFDLCART DTKKLNRRVLEKLIMSGAFDRLGPHRAALMNSLGDALKAA DQHAKAEAIGQADMFGVLAEEPEQIEQSYASCQPWPEQVVL DGERETLGLYLTGHPINQYLKEIERYVGGVRLKDMHPTERG KVITAAGLVVAARVMVTKRGNRIGICTLDDRSGRLEVMLFT DALDKYQQLLEKDRILIVSGQVSFDDFSGGLKMTAREVMDI DEAREKYARGLAISLTDRQIDDQLLNRLRQSLEPHRSGTIPV HLYYQRADARARLRFGATWRVSPSDRLLNDLRGLIGSEQVE LEFD |
| dnaQ/D POSE | 14 | MSTAITRQIVLDTETTGMNQIGAHYEGHKIIEIGAVEVVNRR LTGNNFHVYLKPDRLVDPEAFGVHGIADEFLLDKPTFAEVA DEFMDYIRGAELVIHNAAFDIGFMDYEFSLLKRDIPKTNTFC KVTDSLAVARKMFPGKRNSLDALCARYEIDNSKRTLHGALL DAQILAEVYLAMTGGQTSMAFAMEGETQQQQGEATIQRIVR QASKLRVVFATDEEIAAHEARLDLVQKKGGSCLWRA |
| dnaX/D PO3X | 15 | MSYQVLARKWRPQTFADVVGQEHVLTALANGLSLGRIHHA YLFSGTRGVGKTSIARLLAKGLNCETGITATPCGVCDNCREI EQGRFVDLIEIDAASRTKVEDTRDLLDNVQYAPARGRFKVY LIDEVHMLSRHSFNALLKTLEEPPEHVKFLLATTDPQKLPVTI |
| | | LSRCLQFHLKALDVEQIRHQLEHILNEEHIAHEPRALQLLAR AAEGSLRDALSLTDQAIASGDGQVSTQAVSAMLGTLDDDQ ALSLVEAMVEANGERVMALINEAAARGIEWEALLVEMLGL LHRIAMVQLSPAALGNDMAAIELRMRELARTIPPTDIQLYYQ TLLIGRKELPYAPDRRMGVEMTLLRALAFHPRMPLPEPEVPR QSFAPVAPTAVMTPTQVPPQPQSAPQQAPTVPLPETTSQVLA ARQQLQRVQGATKAKKSEPAAATRARPVNNAALERLASVT DRVQARPVPSALEKAPAKKEAYRWKATTPVMQQKEVVATP KALKKALEHEKTPELAAKLAAEAIERDPWAAQVSQLSLPKL VEQVALNAWKEESDNAVCLHLRSSQRHLNNRGAQQKLAEA LSMLKGSTVELTIVEDDNPAVRTPLEWRQAIYEEKLAQARES IIADNNIQTLRRFFDAELDEESIRPI |
| polA/D PO1 | 16 | |
| | | |
| Bam35 c | 17 | MMTTTNRKKRREIKLFTLDTETRGLDGDVFRIGLFDGKQYY TGYTFADVLPVFEKYKAYDCHVYIHNLDFDLSKIIAELRDYA EPTFNNSLFINGNIVTFTASHIILHDSFRLLPSSLENLCRDFDLL EGGKMDIVDYMEENNYGIYNVKNRKLNKRLTKGNFFTTVD KDDPVLCEYMEYDCRSLYKILEIVIGLSKLEVEQFINCPTTAS LAKTVYKEQYKKDYKVAISTKQYNHKQIGKGLEAFIRKGYY GGRTEVFTPRIENGYHYDKNSLYPYVMKMAEMPVGYPNVL DNEEAELSFDLWKRRKYGAGFIHAKVHVPEDMYIPILPKKD YTGKLIFPVGKIEGVWTFPELALAEAEGCRIEKIESGVVFEKT APVFREFISYFEEIKNTSKGAKRAFSKLMQNALYGKFAMQR ERIMYADISERDKLEAEGHTVSEIIYDMNGIRMEFLEYDGYA KAEYIQPHISAYITSIARILLFKGLKYAHEKGILAYCDTDSCAT TTKFPDKMVHDKEYGKWKLEGYVIEGLYFQPKMYAEKAIN TDGEYEEVLRMKGVPKWVVEEQLDYNTFKKWYLQVKRGK AEIPIYKGGERVQKFLTKSKNNIEMNELAEMHKTINFAREQK RNIDLNKNTTSPLVRNDYGENKDEKSEYEFDEWYERLEEFN DDMNAVEELCMKFGKIQIPEKKQRKLYGLYKEYSSKAKAM CFSNEGLPIQDWCKKTGWDMKELLGELSFL |
| B35-HhH DNA polyme rase | 18 | MMTTTNRKKRREIKLFTLDTETRGLDGDVFRIGLFDGKQYY TGYTFADVLPVFEKYKAYDCHVYIHNLDFDLSKIIAELRDYA EPTFNNSLFINGNIVTFTASHIILHDSFRLLPSSLENLCRDFDLL EGGKMDIVDYMEENNYGIYNVKNRKLNKRLTKGNFFTTVD KDDPVLCEYMEYDCRSLYKILEIVIGLSKLEVEQFINCPTTAS LAKTVYKEQYKKDYKVAISTKQYNHKQIGKGLEAFIRKGYY GGRTEVFTPRIENGYHYDKNSLYPYVMKMAEMPVGYPNVL DNEEAELSFDLWKRRKYGAGFIHAKVHVPEDMYIPILPKKD |
| | | YTGKLIFPVGKIEGVWTFPELALAEAEGCRIEKIESGVVFEKT APVFREFISYFEEIKNTSKGAKRAFSKLMQNALYGKFAMQR ERIMYADISERDKLEAEGHTVSEIIYDMNGIRMEFLEYDGYA KAEYIQPHISAYITSIARILLFKGLKYAHEKGILAYCDTDSCAT TTKFPDKMVHDKEYGKWKLEGYVIEGLYFQPKMYAEKAIN TDGEYEEVLRMKGVPKWVVEEQLDYNTFKKWYLQVKRGK AEIPIYKGGERVQKFLTKSKNNIEMNELAEMHKTINFAREQK RNIDLNKNTTSPLVRNDYGENKDEKSEYEFDEWYERLEEFN DDMNAVEELCMKFGKIQIPEKKQRKLYGLYKEYSSKAKAM CFSNEGLPIQDWCKKTGWDMKELLGELSFLGTGSGAWKEW LERKVGEGRARRLIEYFGSAGEVGKLVENAEVSKLLEVPGIG DEAVARLVPGYKTLRDAGLTPAEAERVLKRYGSVSKVQEG ATPDELRELGLGDAKIARILGLEHHHHHH |
| GP43 DNA polyme rase | 19 | MKEFYISIETVGNNIVERYIDENGKERTREVEYLPTMFRHCK EESKYKDIYGKNCAPQKFPSMKDARDWMKRMEDIGLEALG MNDFKLAYISDTYGSEIVYDRKFVRVANCDIEVTGDKFPDP MKAEYEIDAITHYDSIDDRFYVFDLLNSMYGSVSKWDAKLA AKLDCEGGDEVPQEILDRVIYMPFDNERDMLMEYINLWEQK RPAIFTGWNIEGFDVPYIMNRVKMILGERSMKRFSPIGRVKS KLIQNMYGSKEIYSIDGVSILDYLDLYKKFAFTNLPSFSLESV AQHETKKGKLPYDGPINKLRETNHQRYISYNIIDVESVQAID KIRGFIDLVLSMSYYAKMPFSGVMSPIKTWDAIIFNSLKGEH KVIPQQGSHVKQSFPGAFVFEPKPIARRYIMSFDLTSLYPSIIR QVNISPETIRGQFKVHPIHEYIAGTAPKPSDEYSCSPNGWMY DKHQEGIIPKEIAKVFFQRKDWKKKMFAEEMNAEAIKKIIMK GAGSCSTKPEVERYVKFSDDFLNELSNYTESVLNSLIEECEK AATLANTNQLNRKILINSLYGALGNIHFRYYDLRNATAITIFG QVGIQWIARKINEYLNKVCGTNDEDFIAAGDTDSVYVCVDK VIEKVGLDRFKEQNDLVEFMNQFGKKKMEPMIDVAYRELC DYMNNREHLMHMDREAISCPPLGSKGVGGFWKAKKRYAL NVYDMEDKRFAEPHLKIMGMETQQSSTPKAVQEALEESIRRI |
| | | LQEGEESVQEYYKNFEKEYRQLDYKVIAEVKTANDIAKYDD KGWPGFKCPFHIRGVLTYRRAVSGLGVAPILDGNKVMVLPL REGNPFGDKCIAWPSGTELPKEIRSDVLSWIDHSTLFQKSFV KPLAGMCESAGMDYEEKASLDFLFG |
| T7 GP5 | 20 | MIVSDIEANALLESVTKFHCGVIYDYSTAEYVSYRPSDFGAY LDALEAEVARGGLIVFHNGHKYDVPALTKLAKLQLNREFHL PRENCIDTLVLSRLIHSNLKDTDMGLLRSGKLPGKRFGSHAL EAWGYRLGEMKGEYKDDFKRMLEEQGEEYVDGMEWWNF NEEMMDYNVQDVVVTKALLEKLLSDKHYFPPEIDFTDVGY TTFWSESLEAVDIEHRAAWLLAKQERNGFPFDTKAIEELYVE LAARRSELLRKLTETFGSWYQPKGGTEMFCHPRTGKPLPKY PRIKTPKVGGIFKKPKNKAQREGREPCELDTREYVAGAPYTP VEHVVFNPSSRDHIQKKLQEAGWVPTKYTDKGAPVVDDEV LEGVRVDDPEKQAAIDLIKEYLMIQKRIGQSAEGDKAWLRY VAEDGKIHGSVNPNGAVTGRATHAFPNLAQIPGVRSPYGEQ CRAAFGAEHHLDGITGKPWVQAGIDASGLELRCLAHFMARF DNGEYAHEILNGDIHTKNQIAAELPTRDNAKTFIYGFLYGAG DEKIGQIVGAGKERGKELKKKFLENTPAIAALRESIQQTLVES SQWVAGEQQVKWKRRWIKGLDGRKVHVRSPHAALNTLLQ SAGALICKLWIIKTEEMLVEKGLKHGWDGDFAYMAWVHDE IQVGCRTEEIAQVVIETAQEAMRWVGDHWNFRCLLDTEGK MGPNWAICH |
| T3 GP5 | 21 | MLVSDIEANNLLEKVTKFHCGVIYDYRDGEYHSYRPGDFGA YLDALEAEVKRGGLIVFHNGHKYDVPALTKLAKLQLNREFH LPRENCIDTLVLSRLIHSNLKDTDMGLLRSGKLPGKRFGSHA LEAWGYRLGEMKGEYKDDFKRMLEEQGEEYVDGMEWWN FNEEMMDYNVQDVVVTKALLEKLLSDKHYFPPEIDFTDVGY TTFWSESLEAVDVEHRAAWLLAKQERNGFPFDTKAIEELYV ELAARRSELLRNLTETFGSWYQPKGGTEMFCHPRTGKPLPK YPRIKIPKVGGIFKKPKNKAQREGREPCELDTREYVAGAPYT |
| | | PVEHVVFNPSSRDHIQKKLQEAGWVPTKFTDKGAPVVDDEV LEGVRVDDPEKQAAIDLIKEYLMIQKRIGQSAEGDKAWLRY VAEDGKIHGSVNPNGAVTGRATHAFPNLAQIPGVRSPYGEQ CRAAFGAEHHLDGITGKPWVQAGIDASGLELRCLAHFMARF DNGEYAHEILNGDIHTKNQMAAELPTRDNAKTFIYGFLYGA GDEKIGQIVGAGKERGKELKKKFLENTPAIAALRESIQQTLV ESSQWVAGEQQVKWKRRWIKGLDGRKVHVRSPHAALNTL LQSAGALICKLWIIKTEEMLVEKGLKHGWDGDFAYMAWIH DEIQVACRTEEIAKTVIEVAQEAMRWVGEHWNFRCLLDTEG KMGANWKECH |
| Thiored oxin 1 | 22 | MSDKIIHLTDDSFDTDVLKADGAILVDFWAEWCGPCKMIAPI LDEIADEYQGKLTVAKLNIDQNPGTAPKYGIRGIPTLLLFKN GEVAATKVGALSKGQLKEFLDANLA |
| DnaB Helicas e | 23 | MAGNKPFNKQQAEPRERDPQVAGLKVPPHSIEAEQSVLGGL MLDNERWDDVAERVVADDFYTRPHRHIFTEMARLQESGSPI DLITLAESLERQGQLDSVGGFAYLAELSKNTPSAANISAYADI VRERAVVREMISVANEIAEAGFDPQGRTSEDLLDLAESRVFK IAESRANKDEGPKNIADVLDATVARIEQLFQQPHDGVTGVN TGYDDLNKKTAGLQPSDLIIVAARPSMGKTTFAMNLVENAA MLQDKPVLIFSLEMPSEQIMMRSLASLSRVDQTKIRTGQLDD EDWARISGTMGILLEKRNIYIDDSSGLTPTEVRSRARRIAREH GGIGLIMIDYLQLMRVPALSDNRTLEIAEISRSLKALAKELNV PVVALSQLNRSLEQRADKRPVNSDLRESGSIEQDADLIMFIY RDEVYHENSDLKGIAEIIIGKQRNGPIGTVRLTFNGQWSRFD NYAGPQYDDE |
| DnaC Helicas e loader | 24 | MKNVGDLMQRLQKMMPAHIKPAFKTGEELLAWQKEQGAI RSAALERENRAMKMQRTFNRSGIRPLHQNCSFENYRVECEG QMNALSKARQYVEEFDGNIASFIFSGKPGTGKNHLAAAICNE LLLRGKSVLIITVADIMSAMKDTFRNSGTSEEQLLNDLSNVD |
| | | LLVIDEIGVQTESKYEKVIINQIVDRRSSSKRPTGMLTNSNME EMTKLLGERVMDRMRLGNSLWVIFNWDSYRSRVTGKEY |
| gp41 Helicas e | 25 | MVEIILSHLIFDQAYFSKVWPYMDSEYFESGPAKNTFKLIKS HVNEYHSVPSINALNVALENSSFTETEYSGVKTLISKLADSPE DHSWLVKETEKYVQQRAMFNATSKIIEIQTNAELPPEKRNK KMPDVGAIPDIMRQALSISFDSYVGHDWMDDYEARWLSYM NKARKVPFKLRILNKITKGGAETGTLNVLMAGVNVGKSLGL CSLAADYLQLGHNVLYISMEMAEEVCAKRIDANMLDVSLD DIDDGHISYAEYKGKMEKWREKSTLGRLIVKQYPTGGADAN TFRSLLNELKLKKNFVPTIIIVDYLGICKSCRIRVYSENSYTTV KAIAEELRALAVETETVLWTAAQVGKQAWDSSDVNMSDIA ESAGLPATADFMLAVIETEELAAAEQQLIKQIKSRYGDKNK WNKFLMGVQKGNQKWVEIEQDSTPTEVNEVAGSQQIQAEQ NRYQRNESTRAQLDALANELKF |
| gp59 Helicas e loader | 26 | MIKLRMPAGGERYIDGKSVYKLYLMIKQHMNGKYDVIKYN WCMRVSDAAYQKRRDKYFFQKLSEKYKLKELALIFISNLVA NQDAWIGDISDADALVFYREYIGRLKQIKFKFEEDIRNIYYFS KKVEVSAFKEIFEYNPKVQSSYIFKLLQSNIISFETFILLDSFLN IIDKHDEQTDNLVWNNYSIKLKAYRKILNIDSQKAKNVFIET VKSCKY |
| gp4 Helicas e/Prima se | 27 | MDNSHDSDSVFLYHIPCDNCGSSDGNSLFSDGHTFCYVCEK WTAGNEDTKERASKRKPSGGKPMTYNVWNFGESNGRYSAL TARGISKETCQKAGYWIAKVDGVMYQVADYRDQNGNIVSQ KVRDKDKNFKTTGSHKSDALFGKHLWNGGKKIVVTEGEID MLTVMELQDCKYPVVSLGHGASAAKKTCAANYEYFDQFEQ IILMFDMDEAGRKAVEEAAQVLPAGKVRVAVLPCKDANEC HLNGHDREIMEQVWNAGPWIPDGVVSALSLRERIREHLSSEE SVGLLFSGCTGINDKTLGARGGEVIMVTSGSGMGKSTFVRQ QALQWGTAMGKKVGLAMLEESVEETAEDLIGLHNRVRLRQ |
| | | SDSLKREIIIENGKFDQWFDELFGNDTFHLYDSFAEAETDRLL AKLAYMRSGLGCDVIILDHISIVVSAGESDERKMIDNLMTK LKGFAKSTGVVLVVICHLKNPDKGKAHEEGRPVSITDLRGS GALRQLSDTIIALERNQQGDMPNLVLVRILKCRFTGDTGIAG YMEYNKETGWLEPSSYSGEEESHSESTDWSNDTDF |
| phage T3 Helicas e/Prima se | 28 | MEREDDSIFLFHAPCENCGSSDGNSVYSDGHEWCFVCEHRV PANEEREAKLSTRRRTGGSKPMSYDVWNFGDSNGRYSDLT ARGISKETCQKAGYWLAKVDNRMYQVADYRDQNGSIVSQK VRDKDKNFKTTGSHKSDALFLKHLWSGGKKIVVTEGEIDAL TVMELQDCKYPVVSLGHGASAAKKTCAANYEYFDQFEQIIL MFDMDDAGRKAVEEAAQVLPAGKVRVAVLPCKDANECHI MGEDKAILEQVWNANPWVPDGVVSALSLKDRVKEAMTSE DAVGLLFDGCQGLNDRTLGARGGEVVMVTSGSGMGKSTFV RQQALAWGKRMGKRVGLAMLEESVEDTIQDMMGLNNKVR LRQSDEVKKAIAEDGRFDEWYDELFGDDTFHLYDSFAEAEA DRLLAKLAYMRTGLGCDVIVLDHISIVVSASEESDERKMIDR LMTKLKGFAKSTGVVLVVICHLKNPEKGKAHEEGRAVSITD LRGSGALRQLSDTIIALERNQQGDMPNLVLVRLLKCRFTGDT GIAGYMEYNRETGWLEPSSYTGGEGEGDTGWTEQDGQSDF |
| RecQ | 29 | MAQAEVLNLESGAKQVLQETFGYQQFRPGQEEIIDTVLSGR DCLVVMPTGGGKSLCYQIPALLLNGLTVVVSPLISLMKDQV DQLQANGVAAACLNSTQTREQQLEVMTGCRTGQIRLLYIAP ERLMLDNFLEHLAHWNPVLLAVDEAHCISQWGHDFRPEYA ALGQLRQRFPTLPFMALTATADDTTRQDIVRLLGLNDPLIQIS SFDRPNIRYMLMEKFKPLDQLMRYVQEQRGKSGIIYCNSRA KVEDTAARLQSKGISAAAYHAGLENNVRADVQEKFQRDDL QIVVATVAFGMGINKPNVRFVVHFDIPRNIESYYQETGRAGR DGLPAEAMLFYDPADMAWLRRCLEEKPQGQLQDIERHKLN AMGAFAEAQTCRRLVLLNYFGEGRQEPCGNCDICLDPPKQY DGSTDAQIALSTIGRVNQRFGMGYVVEVIRGANNQRIRDYG |
| | | HDKLKVYGMGRDKSHEHWVSVIRQLIHLGLVTQNIAQHSAL QLTEAARPVLRGESSLQLAVPRIVALKPKAMQKSFGGNYDR KLFAKLRKLRKSIADESNVPPYVVFNDATLIEMAEQMPITAS EMLSVNGVGMRKLERFGKPFMALIRAHVDGDDEE |
| uvrD | 30 | MDVSYLLDSLNDKQREAVAAPRSNLLVLAGAGSGKTRVLV HRIAWLMSVENCSPYSIMAVTFTNKAAAEMRHRIGQLMGTS QGGMWVGTFHGLAHRLLRAHHMDANLPQDFQILDSEDQLR LLKRLIKAMNLDEKQWPPRQAMWYINSQKDEGLRPHHIQSY GNPVEQTWQKVYQAYQEACDRAGLVDFAELLLRAHELWL NKPHILQHYRERFTNILVDEFQDTNNIQYAWIRLLAGDTGKV MIVGDDDQSIYGWRGAQVENIQRFLNDFPGAETIRLEQNYRS TSNILSAANALIENNNGRLGKKLWTDGADGEPISLYCAFNEL DEARFVVNRIKTWQDNGGALAECAILYRSNAQSRVLEEALL QASMPYRIYGGMRFFERQEIKDALSYLRLIANRNDDAAFER VVNTPTRGIGDRTLDVVRQTSRDRQLTLWQACRELLQEKAL AGRAASALQRFMELIDALAQETADMPLHVQTDRVIKDSGLR TMYEQEKGEKGQTRIENLEELVTATRQFSYNEEDEDLMPLQ AFLSHAALEAGEGQADTWQDAVQLMTLHSAKGLEFPQVFI VGMEEGMFPSQMSLDEGGRLEEERRLAYVGVTRAMQKLTL TYAETRRLYGKEVYHRPSRFIGELPEECVEEVRLRATVSRPV SHQRMGTPMVENDSGYKLGQRVRHAKFGEGTIVNMEGSGE HSRLQVAFQGQGIKWLVAAYARLESV |
| helD | 31 | MELKATTLGKRLAQHPYDRAVILNAGIKVSGDRHEYLIPFN QLLAIHCKRGLVWGELEFVLPDEKVVRLHGTEWGETQRFY HHLDAHWRRWSGEMSEIASGVLRQQLDLIATRTGENKWLT REQTSGVQQQIRQALSALPLPVNRLEEFDNCREAWRKCQAW LKDIESARLQHNQAYTEAMLTEYADFFRQVESSPLNPAQAR AVVNGEHSLLVLAGAGSGKTSVLVARAGWLLARGEASPEQI LLLAFGRKAAEEMDERIRERLHTEDITARTFHALALHIIQQGS KKVPIVSKLENDTAARHELFIAEWRKQCSEKKAQAKGWRQ |
| | | WLTEEMQWSVPEGNFWDDEKLQRRLASRLDRWVSLMRMH GGAQAEMIASAPEEIRDLFSKRIKLMAPLLKAWKGALKAEN AVDFSGLIHQAIVILEKGRFISPWKHILVDEFQDISPQRAALLA ALRKQNSQTTLFAVGDDWQAIYRFSGAQMSLTTAFHENFGE GERCDLDTTYRFNSRIGEVANRFIQQNPGQLKKPLNSLTNGD KKAVTLLDESQLDALLDKLSGYAKPEERILILARYHHMRPAS LEKAATRWPKLQIDFMTIHASKGQQADYVIIVGLQEGSDGFP AAARESIMEEALLPPVEDFPDAEERRLMYVALTRARHRVWA LFNKENPSPFVEILKNLDVPVARKP |
| Dda | 32 | MTFDDLTEGQKNAFNIVMKAIKEKKHHVTINGPAGTGKTTL TKFIIEALISTGGTGIILAAPTHAAKKILSKLSGKEASTIHSILKI NPVTYEENVLFEQKEVPDLAKCRVLICDEVSMYDRKLFKILL STIPPWCTIIGIGDNKQIRPVEPGENTAYISPFFTHKDFYQCEL TEVKRSNAPIIDVATDVRNGKWNYDKVVDGHGVRGFTGDT ALRDFMVNYFSIVKSLDDLFENRVMAFTNKSVDKLNSIIRKK IFETDKDFIVGEIIVMQEPLFKTYKIDGKPVSEIIFNNGQLVRII EAEYTSTFVKARGVPGEYLIRHWDLTVETYGDDEYYREKIKI ISSDEELYKFNLFLAKTAETYKNWNKGGKAPWSDFWDAKS QFSKVKALPASTFHKAQGMSVDRAFIYTPCIHYADVELAQQ LLYVGVTRGRYDVFYV |
| usvW | 33 | MDIKVHFHDFSHVRIDCEESTFHELRDFFSFEADGYRFNPRF RYGNWDGRIRLLDYNRLLPFGLVGQIKKFCDNFGYKAWIDP QINEKEELSRKDFDEWLSKLEIYSGNKRIEPHWYQKDAVFEG LVNRRRILNLPTSAGKSLIQALLARYYLENYEGKILIIVPTTAL TTQMADDFVDYRLFSHAMIKKIGGGASKDDKYKNDAPVVV GTWQTVVKQPKEWFSQFGMMMNDECHLATGKSISSIISGLN NCMFKFGLSGSLRDGKANIMQYVGMFGEIFKPVTTSKLMED GQVTELKINSIFLRYPDEFTTKLKGKTYQEEIKIITGLSKRNK WIAKLAIKLAQKDENAFVMFKHVSHGKAIFDLIKNEYDKVY YVSGEVDTETRNIMKTLAENGKGIIIVASYGVFSTGISVKNLH |
| | | HVVLAHGVKSKIIVLQTIGRVLRKHGSKTIATVWDLIDSAGV KPKSANTKKKYVHLNYLLKHGIDRIQRYADEKFNYVMKTV NLISFGPLEKKMLLEFKQFLYEASIDEFMGKIASCQTLEGLEE LEAYYKKRVKETELKDTDDISVRDALAGKRAELEDSDDEVE ESF |
| DnaG primase | 34 | MAGRIPRVFINDLLARTDIVDLIDARVKLKKQGKNFHACCPF HNEKTPSFTVNGEKQFYHCFGCGAHGNAIDFLMNYDKLEFV ETVEELAAMHNLEVPFEAGSGPSQIERHQRQTLYQLMDGLN TFYQQSLQQPVATSARQYLEKRGLSHEVIARFAIGFAPPGWD NVLKRFGGNPENRQSLIDAGMLVTNDQGRSYDRFRERVMFP IRDKRGRVIGFGGRVLGNDTPKYLNSPETDIFHKGRQLYGLY EAQQDNAEPNRLLVVEGYMDVVALAQYGINYAVASLGTST TADHIQLLFRATNNVICCYDGDRAGRDAAWRALETALPYM TDGRQLRFMFLPDGEDPDTLVRKEGKEAFEARMEQAMPLS AFLFNSLMPQVDLSTPDGRARLSTLALPLISQVPGETLRIYLR QELGNKLGILDDSQLERLMPKAAESGVSRPVPQLKRTTMRIL IGLLVQNPELATLVPPLENLDENKLPGLGLFRELVNTCLSQP GLTTGQLLEHYRGTNNAATLEKLSMWDDIADKNIAEQTFTD SLNHMFDSLLELRQEELIARERTHGLSNEERLELWTLNQELA KK |
| gp61 Primas e | 35 | MSSIPWIDNEFAYRALAHLPKFTQVNNSSTFKLRFRCPVCGD SKTDQNKARGWYYGDNNEGNIHCYNCNYHAPIGIYLKEFEP DLYREYIFEIRKEKGKSRPIEKPKELPKQPEKKIIKSLPSCVRL DKLAEDHPIIKYVKARCIPKDKWKYLWFTTEWPKLVNSIAP GTYKKEISEPRLVIPIYNANGKAESFQGRALKKDAPQKYITIE AYPEATKIYGVERVKDGDVYVLEGPIDSLFIENGIAITGGQLD LEVVPFKDRRVWVLDNEPRHPDTIKRMTKLVDAGERVMFW DKSPWKSKDVNDMIRKEGATPEQIMEYMKNNIAQGLMAK MRLSKYAKI |
| gp45 sliding clamp | 36 | MKLSKDTTALLKNFATINSGIMLKSGQFIMTRAVNGTTYAE ANISDVIDFDVAIYDLNGFLGILSLVNDDAEISQSEDGNIKIAD ARSTIFWPAADPSTVVAPNKPIPFPVASAVTEIKAEDLQQLLR VSRGLQIDTIAITVKEGKIVINGFNKVEDSALTRVKYSLTLGD YDGENTFNFIINMANMKMQPGNYKLLLWAKGKQGAAKFE GEHANYVVALEADSTHDF |
| gp44 | 37 | MITVNEKEHILEQKYRPSTIDECILPAFDKETFKSITSKGKIPHI ILHSPSPGTGKTTVAKALCHDVNADMMFVNGSDCKIDFVRG PLTNFASAASFDGRQKVIVIDEFDRSGLAESQRHLRSFMEAY SSNCSIIITANNIDGIIKPLQSRCRVITFGQPTDEDKIEMMKQMI RRLTEICKHEGIAIADMKVVAALVKKNFPDFRKTIGELDSYS SKGVLDAGILSLVTNDRGAIDDVLESLKNKDVKQLRALAPK YAADYSWFVGKLAEEIYSRVTPQSIIRMYEIVGENNQYHGIA ANTELHLAYLFIQLACEMQWK |
| gp62 | 38 | MSLFKDDIQLNEHQVAWYSKDWTAVQSAADSFKEKAENEF FEIIGAINNKTKCSIAQKDYSKFMVENALSQFPECMPAVYAM NLIGSGLSDEAHFNYLMAAVPRGKRYGKWAKLVEDSTEVLI IKLLAKRYQVNTNDAINYKSILTKNGKLPLVLKELKGLVTDD FLKEVTKNVKEQKQLKKLALEW |
| E. coli SSB | 39 | MASRGVNKVILVGNLGQDPEVRYMPNGGAVANITLATSES WRDKATGEMKEQTEWHRVVLFGKLAEVASEYLRKGSQVYI EGQLRTRKWTDQSGQDRYTTEVVVNVGGTMQMLGGRQGG GAPAGGNIGGGQPQGGWGQPQQPQGGNQFSGGAQSRPQQS APAAPSNEPPMDFDDDIPF |
| gp32 SSB | 40 | MFKRKSTAELAAQMAKLNGNKGFSSEDKGEWKLKLDNAG NGQAVIRFLPSKNDEQAPFAILVNHGFKKNGKWYIETCSSTH GDYDSCPVCQYISKNDLYNTDNKEYSLVKRKTSYWANILVV KDPAAPENEGKVFKYRFGKKIWDKINAMIAVDVEMGETPV |
| | | DVTCPWEGANFVLKVKQVSGFSNYDESKFLNQSAIPNIDDES FQKELFEQMVDLSEMTSKDKFKSFEELNTKFGQVMGTAVM GGAAATAAKKADKVADDLDAFNVDDFNTKTEDDFMSSSSG SSSSADDTDLDDLLNDL |
| gp2.5 SSB | 41 | MAKKIFTSALGTAEPYAYIAKPDYGNEERGFGNPRGVYKVD LTIPNKDPRCQRMVDEIVKCHEEAYAAAVEEYEANPPAVAR GKKPLKPYEGDMPFFDNGDGTTTFKFKCYASFQDKKTKETK HINLVVVDSKGKKMEDVPIIGGGSKLKVKYSLVPYKWNTAV GASVKLQLESVMLVELATFGGGEDDWADEVEENGYVASGS AKASKPRDEESWDEDDEESEEADEDGDF |
| T3 Singlestrande d DNA-binding protein | 42 | MAGFKKKVYTSGLGTAEPYAYLSKPDYGNEERGFGNPRGV YKVDLTLSNKDPRCQAMVDEIVKTHEEAYAAAVEEFEANPP QVQRGKKPLTPYEGDMPFFDNGDGTTTFKFKCYASFQDKKT KETKHINLVVVDSKGKKIQEVPIIGGGSKLKVKYSLVPYKW NTAVGASVKLQLESVMLVELATFGGGGEDEWADEVEDGGY TASESRQSRDEQEWQEDEHEETPDDDEDF |
| E. coli ligase | 43 | MESIEQQLTELRTTLRHHEYLYHVMDAPEIPDAEYDRLMRE LRELETKHPELITPDSPTQRVGAAPLAAFSQIRHEVPMLSLDN VFDEESFLAFNKRVQDRLKNNEKVTWCCELKLDGLAVSILY ENGVLVSAATRGDGTTGEDITSNVRTIRAIPLKLHGENIPARL EVRGEVFLPQAGFEKINEDARRTGGKVFANPRNAAAGSLRQ LDPRITAKRPLTFFCYGVGVLEGGELPDTHLGRLLQFKKWG LPVSDRVTLCESAEEVLAFYHKVEEDRPTLGFDIDGVVIKVN SLAQQEQLGFVARAPRWAVAFKFPAQEQMTFVRDVEFQVG RTGAITPVARLEPVHVAGVLVSNATLHNADEIERLGLRIGDK VVIRRAGDVIPQVVNVVLSERPEDTREVVFPTHCPVCGSDVE RVEGEAVARCTGGLICGAQRKESLKHFVSRRAMDVDGMGD KIIDQLVEKEYVHTPADLFKLTAGKLTGLERMGPKSAQNVV NALEKAKETTFARFLYALGIREVGEATAAGLAAYFGTLEAL |
| | | EAASIEELQKVPDVGIVVASHVHNFFAEESNRNVISELLAEG VHWPAPIVINAEEIDSPFAGKTVVLTGSLSQMSRDDAKARLV ELGAKVAGSVSKKTDLVIAGEAAGSKLAKAQELGIEVIDEAE MLRLLGS |
| T7 DNA ligase | 44 | MMNIKTNPFKAVSFVESAIKKALDNAGYLIAEIKYDGVRGNI CVDNTANSYWLSRVSKTIPALEHLNGFDVRWKRLLNDDRCF YKDGFMLDGELMVKGVDFNTGSGLLRTKWTDTKNQEFHEE LFVEPIRKKDKVPFKLHTGHLHIKLYAILPLHIVESGEDCDVM TLLMQEHVKNMLPLLQEYFPEIEWQAAESYEVYDMVELQQ LYEQKRAEGHEGLIVKDPMCIYKRGKKSGWWKMKPENEAD GIIQGLVWGTKGLANEGKVIGFEVLLESGRLVNATNISRALM DEFTETVKEATLSQWGFFSPYGIGDNDACTINPYDGWACQIS YMEETPDGSLRHPSFVMFRGTEDNPQEKM |
| T3 DNA ligase | 45 | MNIFNTNPFKAVSFVESAVKKALETSGYLIADCKYDGVRGNI VVDNVAEAAWLSRVSKFIPALEHLNGFDKRWQQLLNDDRCI FPDGFMLDGELMVKGVDFNTGSGLLRTKWVKRDNMGFHL TNVPTKLTPKGREVIDGKFEFHLDPKRLSVRLYAVMPIHIAES GEDYDVQNLLMPYHVEAMRSLLVEYFPEIEWLIAETYEVYD MDSLTELYEEKRAEGHEGLIVKDPQGIYKRGKKSGWWKLK PECEADGIIQGVNWGTEGLANEGKVIGFSVLLETGRLVDAN NISRALMDEFTSNVKAHGEDFYNGWACQVNYMEATPDGSL RHPSFEKFRGTEDNPQEKM |
| T4 DNA ligase | 46 | MILKILNEIASIGSTKQKQAILEKNKDNELLKRVYRLTYSRGL QYYIKKWPKPGIATQSFGMLTLTDMLDFIEFTLATRKLTGNA AIEELTGYITDGKKDDVEVLRRVMMRDLECGASVSIANKVW PGLIPEQPQMLASSYDEKGINKNIKFPAFAQLKADGARCFAE VRGDELDDVRLLSRAGNEYLGLDLLKEELIKMTAEARQIHP EGVLIDGELVYHEQVKKEPEGLDFLFDAYPENSKAKEFAEV AESRTASNGIANKSLKGTISEKEAQCMKFQVWDYVPLVEIYS |
| | | LPAFRLKYDVRFSKLEQMTSGYDKVILIENQVVNNLDEAKVI YKKYIDQGLEGIILKNIDGLWENARSKNLYKFKEVIDVDLKI VGIYPHRKDPTKAGGFILESECGKIKVNAGSGLKDKAGVKS HELDRTRIMENQNYYIGKILECECNGWLKSDGRTDYVKLFL PIAIRLREDKTKANTFEDVFGDFHEVTGL |
| GyrA | 47 | MSDLAREITPVNIEEELKSSYLDYAMSVIVGRALPDVRDGLK PVHRRVLYAMNVLGNDWNKAYKKSARVVGDVIGKYHPHG DSAVYDTIVRMAQPFSLRYMLVDGQGNFGSIDGDSAAAMR YTEIRLAKIAHELMADLEKETVDFVDNYDGTEKIPDVMPTKI PNLLVNGSSGIAVGMATNIPPHNLTEVINGCLAYIDDEDISIE GLMEHIPGPDFPTAAIINGRRGIEEAYRTGRGKVYIRARAEVE VDAKTGRETIIVHEIPYQVNKARLIEKIAELVKEKRVEGISAL RDESDKDGMRIVIEVKRDAVGEVVLNNLYSQTQLQVSFGIN MVALHHGQPKIMNLKDIIAAFVRHRREVVTRRTIFELRKARD RAHILEALAVALANIDPIIELIRHAPTPAEAKTALVANPWQLG NVAAMLERAGDDAARPEWLEPEFGVRDGLYYLTEQQAQAI LDLRLQKLTGLEHEKLLDEYKELLDQIAELLRILGSADRLME VIREELELVREQFGDKRRTEITANSADINLEDLITQEDVVVTL SHQGYVKYQPLSEYEAQRRGGKGKSAARIKEEDFIDRLLVA NTHDHILCFSSRGRVYSMKVYQLPEATRGARGRPIVNLLPLE QDERITAILPVTEFEEGVKVFMATANGTVKKTVLTEFNRLRT AGKVAIKLVDGDELIGVDLTSGEDEVMLFSAEGKVVRFKES SVRAMGCNTTGVRGIRLGEGDKVVSLIVPRGDGAILTATQN GYGKRTAVAEYPTKSRATKGVISIKVTERNGLVVGAVQVDD CDQIMMITDAGTLVRTRVSEISIVGRNTQGVILIRTAEDENVV GLQRVAEPVDEEDLDTIDGSAAEGDDEIAPEVDVDDEPEEE |
| GyrB | 48 | MSNSYDSSSIKVLKGLDAVRKRPGMYIGDTDDGTGLHHMV FEVVDNAIDEALAGHCKEIIVTIHADNSVSVQDDGRGIPTGIH PEEGVSAAEVIMTVLHAGGKFDDNSYKVSGGLHGVGVSVV NALSQKLELVIQREGKIHRQIYEHGVPQAPLAVTGETEKTGT |
| | | MVRFWPSLETFTNVTEFEYEILAKRLRELSFLNSGVSIRLRDK RDGKEDHFHYEGGIKAFVEYLNKNKTPIHPNIFYFSTEKDGI GVEVALQWNDGFQENIYCFTNNIPQRDGGTHLAGFRAAMT RTLNAYMDKEGYSKKAKVSATGDDAREGLIAVVSVKVPDP KFSSQTKDKLVSSEVKSAVEQQMNELLAEYLLENPTDAKIV VGKIIDAARAREAARRAREMTRRKGALDLAGLPGKLADCQ ERDPALSELYLVEGDSAGGSAKQGRNRKNQAILPLKGKILN VEKARFDKMLSSQEVATLITALGCGIGRDEYNPDKLRYHSIII MTDADVDGSHIRTLLLTFFYRQMPEIVERGHVYIAQPPLYKV KKGKQEQYIKDDEAMDQYQISIALDGATLHTNASAPALAGE ALEKLVSEYNATQKMINRMERRYPKAMLKELIYQPTLTEAD LSDEQTVTRWVNALVSELNDKEQHGSQWKFDVHTNAEQNL FEPIVRVRTHGVDTDYPLDHEFITGGEYRRICTLGEKLRGLLE EDAFIERGERRQPVASFEQALDWLVKESRRGLSIQRYKGLGE MNPEQLWETTMDPESRRMLRVTVKDAIAADQLFTTLMGDA VEPRRAFIEENALKAANIDI |
| Topo III | 49 | MRLFIAEKPSLARAIADVLPKPHRKGDGFIECGNGQVVTWCI GHLLEQAQPDAYDSRYARWNLADLPIVPEKWQLQPRPSVTK QLNVIKRFLHEASEIVHAGDPDREGQLLVDEVLDYLQLAPEK RQQVQRCLINDLNPQAVERAIDRLRSNSEFVPLCVSALARAR ADWLYGINMTRAYTILGRNAGYQGVLSVGRVQTPVLGLVV RRDEEIENFVAKDFFEVKAHIVTPADERFTAIWQPSEACEPY QDEEGRLLHRPLAEHVVNRISGQPAIVTSYNDKRESESAPLP FSLSALQIEAAKRFGLSAQNVLDICQKLYETHKLITYPRSDCR YLPEEHFAGRHAVMNAISVHAPDLLPQPVVDPDIRNRCWDD KKVDAHHAIIPTARSSAINLTENEAKVYNLIARQYLMQFCPD AVFRKCVIELDIAKGKFVAKARFLAEAGWRTLLGSKERDEE NDGTPLPVVAKGDELLCEKGEVVERQTQPPRHFTDATLLSA MTGIARFVQDKDLKKILRATDGLGTEATRAGIIELLFKRGFL TKKGRYIHSTDAGKALFHSLPEMATRPDMTAHWESVLTQIS |
| | | EKQCRYQDFMQPLVGTLYQLIDQAKRTPVRQFRGIVAPGSG GSADKKKAAPRKRSAKKSPPADEVGSGAIA |
| ParE | 50 | MSDMAERLALHEFTENAYLNYSMYVIMDRALPFIGDGLKPV QRRIVYAMSELGLNASAKFKKSARTVGDVLGKYHPHGDSA CYEAMVLMAQPFSYRYPLVDGQGNWGAPDDPKSFAAMRY TESRLSKYSELLLSELGQGTADWVPNFDGTLQEPKMLPARLP NILLNGTTGIAVGMATDIPPHNLREVAQAAIALIDQPKTTLD QLLDIVQGPDYPTEAEIITSRAEIRKIYENGRGSVRMRAVWK KEDGAVVISALPHQVSGARVLEQIAAQMRNKKLPMVDDLR DESDHENPTRLVIVPRSNRVDMDQVMNHLFATTDLEKSYRI NLNMIGLDGRPAVKNLLEILSEWLVFRRDTVRRRLNYRLEK VLKRLHILEGLLVAFLNIDEVIEIIRNEDEPKPALMSRFGLTET QAEAILELKLRHLAKLEEMKIRGEQSELEKERDQLQGILASE RKMNNLLKKELQADAQAYGDDRRSPLQEREEAKAMSEHD MLPSEPVTIVLSQMGWVRSAKGHDIDAPGLNYKAGDSFKA AVKGKSNQPVVFVDSTGRSYAIDPITLPSARGQGEPLTGKLT LPPGATVDHMLMESDDQKLLMASDAGYGFVCTFNDLVARN RAGKALITLPENAHVMPPVVIEDASDMLLAITQAGRMLMFP VSDLPQLSKGKGNKIINIPSAEAARGEDGLAQLYVLPPQSTLT IHVGKRKIKLRPEELQKVTGERGRRGTLMRGLQRIDRVEIDS PRRASSGDSEE |
| ParC | 51 | MTQTYNADAIEVLTGLEPVRRRPGMYTDTTRPNHLGQEVID NSVDEALAGHAKRVDVILHADQSLEVIDDGRGMPVDIHPEE GVPAVELILCRLHAGGKFSNKNYQFSGGLHGVGISVVNALS KRVEVNVRRDGQVYNIAFENGEKVQDLQVVGTCGKRNTGT SVHFWPDETFFDSPRFSVSRLTHVLKAKAVLCPGVEITFKDEI NNTEQRWCYQDGLNDYLAEAVNGLPTLPEKPFIGNFAGDTE AVDWALLWLPEGGELLTESYVNLIPTMQGGTHVNGLRQGL LDAMREFCEYRNILPRGVKLSAEDIWDRCAYVLSVKMQDPQ FAGQTKERLSSRQCAAFVSGVVKDAFILWLNQNVQAAELLA |
| | | EMAISSAQRRMRAAKKVVRKKLTSGPALPGKLADCTAQDL NRTELFLVEGDSAGGSAKQARDREYQAIMPLKGKILNTWEV SSDEVLASQEVHDISVAIGIDPDSDDLSQLRYGKICILADADS DGLHIATLLCALFVKHFRALVKHGHVYVALPPLYRIDLGKE VYYALTEEEKEGVLEQLKRKKGKPNVQRFKGLGEMNPMQL RETTLDPNTRRLVQLTIDDEDDQRTDAMMDMLLAKKRSED RRNWLQEKGDMAEIEV |
| SHEE T4 39 | 52 | MIKNEIKILSDIEHIKKRSGMYIGSSANETHERFMFGKWESVQ YVPGLVKLIDEIIDNSVDEGIRTKFKFANKINVTIKNNQVTVE DNGRGIPQAMVKTPTGEEIPGPVAAWTIPKAGGNFGDDKER VTGGMNGVGSSLTNIFSVMFVGETGDGQNNIVVRCSNGME NKSWEDIPGKWKGTRVTFIPDFMSFETNELSQVYLDITLDRL QTLAVVYPDIQFTFNGKKVQGNFKKYARQYDEHAIVQEQEN CSIAVGRSPDGFRQLTYVNNIHTKNGGHHIDCAMDDICEDLI PQIKRKFKIDVTKARVKECLTIVMFVRDMKNMRLIRQTKER LTSPFGEIRSHIQLDAKKISRDILNNEAILMPIIEAALARKLAA EKAAETKAAKKASKAKVHKHIKANLCGKDADTTLFLTEGD SAIGYLIDVRDKELHGGYPLRGKVLNSWGMSYADMLKNKE LFDICAITGLVLGEKAFEEKEDGEWFTFELNGDTIIVNENDEV QINGKWITVGELRKNL |
| SHEE T4 52 | 53 | MQLNNRDLKSIIDNEALAYAMYTVENRAIPNMIDGFKPVQR FVIARALDLARGNKDKFHKLASIAGGVADLGYHHGENSAQ DAGALMANTWNNNFPLLDGQGNFGSRTVQKAAASRYIFAR VSKNFYNVYKDTEYAPVHQDKEHIPPAFYLPIIPTVLLNGVS GIATGYATYILPHSVSSVKKAVLQALQGKKVTKPKVEFPEFR GEVVEIDGQYEIRGTYKFTSRTQMHITEIPYKYDRETYVSKIL DPLENKGFITWDDACGEHGFGFKVKFRKEYSLSDNEEERHA KIMKDFGLIERRSQNITVINEKGKLQVYDNVVDLIKDFVEVR KTYVQKRIDNKIKETESAFRLAFAKAHFIKKVISGEIVVQGKT |
| | | RKELTEELSKIDMYSSYVDKLVGMNIFHMTSDEAKKLAEEA KAKKEENEYWKTTDVVTEYTKDLEEIK |
| SHEE T4 60 | 54 | MKFVKIDSSSVDMKKYKLQNNVRRSIKSSSMNYANVAIMT DADHDGLGSIYPSLLGFFSNWPELFEQGRIRFVKTPVIIAQVG KKQEWFYTVAEYESAKDALPKHSIRYIKGLGSLEKSEYREMI QNPVYDVVKLPENWKELFEMLMGDNADLRKEWMSQ |
| Topo I | 55 | MGKALVIVESPAKAKTINKYLGSDYVVKSSVGHIRDLPTSGS AAKKSADSTSTKTAKKPKKDERGALVNRMGVDPWHNWEA HYEVLPGKEKVVSELKQLAEKADHIYLATDLDREGEAIAWH LREVIGGDDARYSRVVFNEITKNAIRQAFNKPGELNIDRVNA QQARRFMDRVVGYMVSPLLWKKIARGLSAGRVQSVAVRL VVEREREIKAFVPEEFWEVDASTTTPSGEALALQVTHQNDKP FRPVNKEQTQAAVSLLEKARYSVLEREDKPTTSKPGAPFITS TLQQAASTRLGFGVKKTMMMAQRLYEAGYITYMRTDSTNL SQDAVNMVRGYISDNFGKKYLPESPNQYASKENSQEAHEAI RPSDVNVMAESLKDMEADAQKLYQLIWRQFVACQMTPAK YDSTTLTVGAGDFRLKARGRILRFDGWTKVMPALRKGDED RILPAVNKGDALTLVELTPAQHFTKPPARFSEASLVKELEKR GIGRPSTYASIISTIQDRGYVRVENRRFYAEKMGEIVTDRLEE NFRELMNYDFTAQMENSLDQVANHEAEWKAVLDHFFSDFT QQLDKAEKDPEEGGMRPNQMVLTSIDCPTCGRKMGIRTAST GVFLGCSGYALPPKERCKTTINLVPENEVLNVLEGEDAETNA LRAKRRCPKCGTAMDSYLIDPKRKLHVCGNNPTCDGYEIEE GEFRIKGYDGPIVECEKCGSEMHLKMGRFGKYMACTNEECK NTRKILRNGEVAPPKEDPVPLPELPCEKSDAYFVLRDGAAGV FLAANTFPKSRETRAPLVEELYRFRDRLPEKLRYLADAPQQD PEGNKTMVRFSRKTKQQYVSSEKDGKATGWSAFYVDGKW VEGKK |
| RNase H/rnhA | 56 | MLKQVEIFTDGSCLGNPGPGGYGAILRYRGREKTFSAGYTRT TNNRMELMAAIVALEALKEHCEVILSTDSQYVRQGITQWIH NWKKRGWKTADKKPVKNVDLWQRLDAALGQHQIKWEWV KGHAGHPENERCDELARAAAMNPTLEDTGYQVEV |
| phage T4 RNase H | 57 | MDLEMMLDEDYKEGICLIDFSQIALSTALVNFPDKEKINLSM VRHLILNSIKFNVKKAKTLGYTKIVLCIDNAKSGYWRRDFAY YYKKNRGKAREESTWDWEGYFESSHKVIDELKAYMPYIVM DIDKYEADDHIAVLVKKFSLEGHKILIISSDGDFTQLHKYPNV KQWSPMHKKWVKIKSGSAEIDCMTKILKGDKKDNVASVKV RSDFWFTRVEGERTPSMKTSIVEAIANDREQAKVLLTESEYN RYKENLVLIDFDYIPDNIASNIVNYYNSYKLPPRGKIYSYFVK AGLSKLTNSINEF |
| rpoE | 58 | MSEQLTDQVLVERVQKGDQKAFNLLVVRYQHKVASLVSRY VPSGDVPDVVQEAFIKAYRALDSFRGDSAFYTWLYRIAVNT AKNYLVAQGRRPPSSDVDAIEAENFESGGALKEISNPENLML SEELRQIVFRTIESLPEDLRMAITLRELDGLSYEEIAAIMDCPV GTVRSRIFRAREAIDNKVQPLIRR |
| rpoH | 59 | MTDKMQSLALAPVGNLDSYIRAANAWPMLSADEERALAEK LHYHGDLEAAKTLILSHLRFVVHIARNYAGYGLPQADLIQEG NIGLMKAVRRFNPEVGVRLVSFAVHWIKAEIHEYVLRNWRI VKVATTKAQRKLFFNLRKTKQRLGWFNQDEVEMVARELGV TSKDVREMESRMAAQDMTFDLSSDDDSDSQPMAPVLYLQD KSSNFADGIEDDNWEEQAANRLTDAMQGLDERSQDIIRARW LDEDNKSTLQELADRYGVSAERVRQLEKNAMKKLRAAIEA |
| rpoS | 60 | MSQNTLKVHDLNEDAEFDENGVEVFDEKALVEQEPSDNDL AEEELLSQGATQRVLDATQLYLGEIGYSPLLTAEEEVYFARR ALRGDVASRRRMIESNLRLVVKIARRYGNRGLALLDLIEEGN LGLIRAVEKFDPERGFRFSTYATWWIRQTIERAIMNQTRTIRL |
| | | PIHIVKELNVYLRTARELSHKLDHEPSAEEIAEQLDKPVDDVS RMLRLNERITSVDTPLGGDSEKALLDILADEKENGPEDTTQD DDMKQSIVKWLFELNAKQREVLARRFGLLGYEAATLEDVG REIGLTRERVRQIQVEGLRRLREILQTQGLNIEALFRE |
| ihfA | 61 | MALTKAEMSEYLFDKLGLSKRDAKELVELFFEEIRRALENG EQVKLSGFGNFDLRDKNQRPGRNPKTGEDIPITARRVVTFRP GQKLKSRVENASPKDE |
| ihfB | 62 | MTKSELIERLATQQSHIPAKTVEDAVKEMLEHMASTLAQGE RIEIRGFGSFSLHYRAPRTGRNPKTGDKVELEGKYVPHFKPG KELRDRANIYG |
| RecA | 63 | MAIDENKQKALAAALGQIEKQFGKGSIMRLGEDRSMDVETI STGSLSLDIALGAGGLPMGRIVEIYGPESSGKTTLTLQVIAAA QREGKTCAFIDAEHALDPIYARKLGVDIDNLLCSQPDTGEQA LEICDALARSGAVDVIVVDSVAALTPKAEIEGEIGDSHMGLA ARMMSQAMRKLAGNLKQSNTLLIFINQIRMKIGVMFGNPET TTGGNALKFYASVRLDIRRIGAVKEGENVVGSETRVKVVKN KIAAPFKQAEFQILYGEGINFYGELVDLGVKEKLIEKAGAWY SYKGEKIGQGKANATAWLKDNPETAKEIEKKVRELLLSNPN STPDFSVDDSEGVAETNEDF |
| RecB | 64 | MSDVAETLDPLRLPLQGERLIEASAGTGKTFTIAALYLRLLL GLGGSAAFPRPLTVEELLVVTFTEAATAELRGRIRSNIHELRI ACLRETTDNPLYERLLEEIDDKAQAAQWLLLAERQMDEAA VFTIHGFCQRMLNLNAFESGMLFEQQLIEDESLLRYQACADF WRRHCYPLPREIAQVVFETWKGPQALLRDINRYLQGEAPVI KAPPPDDETLASRHAQIVARIDTVKQQWRDAVGELDALIESS GIDRRKFNRSNQAKWIDKISAWAEEETNSYQLPESLEKFSQR FLEDRTKAGGETPRHPLFEAIDQLLAEPLSIRDLVITRALAEIR ETVAREKRRRGELGFDDMLSRLDSALRSESGEVLAAAIRTRF PVAMIDEFQDTDPQQYRIFRRIWHHQPETALLLIGDPKQAIY |
| | | AFRGADIFTYMKARSEVHAHYTLDTNWRSAPGMVNSVNKL FSQTDDAFMFREIPFIPVKSAGKNQALRFVFKGETQPAMKM WLMEGESCGVGDYQSTMAQVCAAQIRDWLQAGQRGEALL MNGDDARPVRASDISVLVRSRQEAAQVRDALTLLEIPSVYLS NRDSVFETLEAQEMLWLLQAVMTPERENTLRSALATSMMG LNALDIETLNNDEHAWDVVVEEFDGYRQIWRKRGVMPMLR ALMSARNIAENLLATAGGERRLTDILHISELLQEAGTQLESE HALVRWLSQHILEPDSNASSQQMRLESDKHLVQIVTIHKSKG LEYPLVWLPFITNFRVQEQAFYHDRHSFEAVLDLNAAPESVD LAEAERLAEDLRLLYVALTRSVWHCSLGVAPLVRRRGDKK GDTDVHQSALGRLLQKGEPQDAAGLRTCIEALCDDDIAWQT AQTGDNQPWQVNDVSTAELNAKTLQRLPGDNWRVTSYSGL QQRGHGIAQDLMPRLDVDAAGVASVVEEPTLTPHQFPRGAS PGTFLHSLFEDLDFTQPVDPNWVREKLELGGFESQWEPVLTE WITAVLQAPLNETGVSLSQLSARNKQVEMEFYLPISEPLIASQ LDTLIRQFDPLSAGCPPLEFMQVRGMLKGFIDLVFRHEGRYY LLDYKSNWLGEDSSAYTQQAMAAAMQAHRYDLQYQLYTL ALHRYLRHRIADYDYEHHFGGVIYLFLRGVDKEHPQQGIYT TRPNAGLIALMDEMFAGMTLEEA |
| RecC | 65 | MLRVYHSNRLDVLEALMEFIVERERLDDPFEPEMILVQSTG MAQWLQMTLSQKFGIAANIDFPLPASFIWDMFVRVLPEIPKE SAFNKQSMSWKLMTLLPQLLEREDFTLLRHYLTDDSDKRKL FQLSSKAADLFDQYLVYRPDWLAQWETGHLVEGLGEAQA WQAPLWKALVEYTHQLGQPRWHRANLYQRFIETLESATTC PPGLPSRVFICGISALPPVYLQALQALGKHIEIHLLFTNPCRYY WGDIKDPAYLAKLLTRQRRHSFEDRELPLFRDSENAGQLFN SDGEQDVGNPLLASWGKLGRDYIYLLSDLESSQELDAFVDV TPDNLLHNIQSDILELENRAVAGVNIEEFSRSDNKRPLDPLDS SITFHVCHSPQREVEVLHDRLLAMLEEDPTLTPRDIIVMVADI DSYSPFIQAVFGSAPADRYLPYAISDRRARQSHPVLEAFISLL SLPDSRFVSEDVLALLDVPVLAARFDITEEGLRYLRQWVNES |
| | | GIRWGIDDDNVRELELPATGQHTWRFGLTRMLLGYAMESA QGEWQSVLPYDESSGLIAELVGHLASLLMQLNIWRRGLAQE RPLEEWLPVCRDMLNAFFLPDAETEAAMTLIEQQWQAIIAE GLGAQYGDAVPLSLLRDELAQRLDQERISQRFLAGPVNICTL MPMRSIPFKVVCLLGMNDGVYPRQLAPLGFDLMSQKPKRG DRSRRDDDRYLFLEALISAQQKLYISYIGRSIQDNSERFPSVL VQELIDYIGQSHYLPGDEALNCDESEARVKAHLTCLHTRMPF DPQNYQPGERQSYAREWLPAASQAGKAHSEFVQPLPFTLPE TVPLETLQRFWAHPVRAFFQMRLQVNFRTEDSEIPDTEPFILE GLSRYQINQQLLNALVEQDDAERLFRRFRAAGDLPYGAFGEI FWETQCQEMQQLADRVIACRQPGQSMEIDLACNGVQITGW LPQVQPDGLLRWRPSLLSVAQGMQLWLEHLVYCASGGNGE SRLFLRKDGEWRFPPLAAEQALHYLSQLIEGYREGMSAPLLV LPESGGAWLKTCYDAQNDAMLDDDSTLQKARTKFLQAYEG NMMVRGEGDDIWYQRLWRQLTPETMEAIVEQSQRFLLPLFR FNQS |
| RecD | 66 | MKLQKQLLEAVEHKQLRPLDVQFALTVAGDEHPAVTLAAA LLSHDAGEGHVCLPLSRLENNEASHPLLATCVSEIGELQNWE ECLLASQAVSRGDEPTPMILCGDRLYLNRMWCNERTVARFF NEVNHAIEVDEALLAQTLDKLFPVSDEINWQKVAAAVALTR RISVISGGPGTGKTTTVAKLLAALIQMADGERCRIRLAAPTG KAAARLTESLGKALRQLPLTDEQKKRIPEDASTLHRLLGAQP GSQRLRHHAGNPLHLDVLVVDEASMIDLPMMSRLIDALPDH ARVIFLGDRDQLASVEAGAVLGDICAYANAGFTAERARQLS RLTGTHVPAGTGTEAASLRDSLCLLQKSYRFGSDSGIGQLAA AINRGDKTAVKTVFQQDFTDIEKRLLQSGEDYIAMLEEALA GYGRYLDLLQARAEPDLIIQAFNEYQLLCALREGPFGVAGLN ERIEQFMQQKRKIHRHPHSRWYEGRPVMIARNDSALGLFNG DIGIALDRGQGTRVWFAMPDGNIKSVQPSRLPEHETTWAMT VHKSQGSEFDHAALILPSQRTPVVTRELVYTAVTRARRRLSL YADERILSAAIATRTERRSGLAALFSSRE |
| RecF | 67 | MSLTRLLIRDFRNIETADLALSPGFNFLVGANGSGKTSVLEAI YTLGHGRAFRSLQIGRVIRHEQEAFVLHGRLQGEERETAIGL TKDKQGDSKVRIDGTDGHKVAELAHLMPMQLITPEGFTLLN GGPKYRRAFLDWGCFHNEPGFFTAWSNLKRLLKQRNAALR QVTRYEQLRPWDKELIPLAEQISTWRAEYSAGIAADMADTC KQFLPEFSLTFSFQRGWEKETEYAEVLERNFERDRQLTYTAH GPHKADLRIRADGAPVEDTLSRGQLKLLMCALRLAQGEFLT RESGRRCLYLIDDFASELDDERRGLLASRLKATQSQVFVSAI SAEHVIDMSDENSKMFTVEKGKITD |
| DiaA | 68 | MQERIKACFTESIQTQIAAAEALPDAISRAAMTLVQSLLNGN KILCCGNGTSAANAQHFAASMINRFETERPSLPAIALNTDNV VLTAIANDRLHDEVYAKQVRALGHAGDVLLAISTRGNSRDI VKAVEAAVTRDMTIVALTGYDGGELAGLLGPQDVEIRIPSH RSARIQEMHMLTVNCLCDLIDNTLFPHQDD |
| Rop | 69 | MTKQEKTALNMARFIRSQTLTLLEKLNELDADEQADICESLH DHADELYRSCLARFGDDGENL |
| T7 endonu clease | 70 | MAGYGAKGIRKVGAFRSGLEDKVSKQLESKGIKFEYEEWK VPYVIPASNHTYTPDFLLPNGIFVETKGLWESDDRKKHLLIRE QHPELDIRIVFSSSRTKLYKGSPTSYGEFCEKHGIKFADKLIPA EWIKEPKKEVPFDRLKRKGGKK |
| T7 exonucl ease | 71 | MALLDLKQFYELREGCDDKGILVMDGDWLVFQAMSAAEFD ASWEEEIWHRCCDHAKARQILEDSIKSYETRKKAWAGAPIV LAFTDSVNWRKELVDPNYKANRKAVKKPVGYFEFLDALFE REEFYCIREPMLEGDDVMGVIASNPSAFGARKAVIISCDKDF KTIPNCDFLWCTTGNILTQTEESADWWHLFQTIKGDITDGYS GIAGWGDTAEDFLNNPFITEPKTSVLKSGKNKGQEVTKWVK RDPEPHETLWDCIKSIGAKAGMTEEDIIKQGQMARILRFNEY NFIDKEIYLWRP |
| T3 endonu clease | 72 | MAGAYAARCTQGRAFRSGLEDKVSKQLESKGIKFDYELWRI PYVIPESDHLYTPDFLLPNGIFIETKGLWDSDDRKKHLLIREQ HPELDIRLVFSSSRSKLYKGSPTSYGEWCEKHGILFADKLIPV AGVKEPKKEVPFDKFKTKKGVKKNG |
| T3 exonucl ease | 73 | MALLDLKQFYELREGCDDKGILVMDGDWLVFQAMSAAEFD ASWEEEIWHRCCDHAKARQILEDSIKSYETRKKAWVGAPIV LAFTDSVNWRKELVDPNYKANRKATKKPVGYFEFLEALFER EEFYCIREPMLEGDDVMGVIASNPSAFGARKAVIISCDKDFK TIPNCDFLWCTTGNILTQTKETADWWHLFQTIKGDMTDGYS GIPGWGDTAEGFLNDPFIVEPVESVLKSGKNKGQTVTKWVK RAPDATETLWDCIKSIGAKAGMTEQEIIKQGQMARILRFEEY NYIDKEIYLWTPRS |
| EndoV | 74 | MTRINLTLVSELADQHLMAEYRELPRVFGAVRKHVANGKR VRDFKISPTFILGAGHVTFFYDKLEFLRKRQIELIAECLKRGF NIKDTTVQDISDIPQEFRGDYIPHEASIAISQARLDEKIAQRPT WYKYYGKAIYA |
| T4 endonu clease VII | 75 | MLLTGKLYKEEKQKFYDAQNGKCLICQRELNPDVQANHLD HDHELNGPKAGKVRGLLCNLCNAAEGQMKHKFNRSGLKG QGVDYLEWLENLLTYLKSDYTQNNIHPNFVGDKSKEFSRLG KEEMMAEMLQRGFEYNESDTKTQLIASFKKQLRKSLK |
| T4 endonu clease SEGA | 76 | MKRHKEKKYNYTYVITNLVNNKIYYGTHSTDDLNDGYMGS GTLLAQAKKKYGKKNFNLSILGFYKDFKSARDAERELVTID VVNDPMTYNLKIGGEGGRRIGYRVSSETKEKISKAQKGKPK HLGFSDVCRKAQLGKKQSEETKAKRKEALLNNPYGYNRNK PSHKRDPIMWDNIEKIKEIWENSGKSGAIKLKKLAIEAGFPNK SYARMLEVFRGTRTLL |
| uvsX | 77 | MSDLKSRLIKASTSKLTAELTASKFFNEKDVVRTKIPMMNIA LSGEITGGMQSGLLILAGPSKSFKSNFGLTMVSSYMRQYPDA |
| | | VCLFYDSEFGITPAYLRSMGVDPERVIHTPVQSLEQLRIDMV NQLDAIERGEKVVVFIDSLGNLASKKETEDALNEKVVSDMT RAKTMKSLFRIVTPYFSTKNIPCIAINHTYETQEMFSKTVMG GGTGPMYSADTVFIIGKRQIKDGSDLQGYQFVLNVEKSRTV KEKSKFFIDVKFDGGIDPYSGLLDMALELGFVVKPKNGWYA REFLDEETGEMIREEKSWRAKDTNCTTFWGPLFKHQPFRDAI KRAYQLGAIDSNEIVEAEVDELINSKVEKFKSPESKSKSAAD LETDLEQLSDMEEFNE |
| uvsY | 78 | MRLEDLQEELKKDVFIDSTKLQYEAANNVMLYSKWLNKHS SIKKEMLRIEAQKKVALKARLDYYSGRGDGDEFSMDRYEKS EMKTVLSADKDVLKVDTSLQYWGILLDFCSGALDAIKSRGF AIKHIQDMRAFEAGK |
| PNK | 79 | MKKIILTIGCPGSGKSTWAREFIAKNPGFYNINRDDYRQSIMA HEERDEYKYTKKKEGIVTGMQFDTAKSILYGGDSVKGVIISD TNLNPERRLAWETFAKEYGWKVEHKVFDVPWTELVKRNSK RGTKAVPIDVLRSMYKSMREYLGLPVYNGTPGKPKAVIFDV DGTLAKMNGRGPYDLEKCDTDVINPMVVELSKMYALMGY QIVVVSGRESGTKEDPTKYYRMTRKWVEDIAGVPLVMQCQ REQGDTRKDDVVKEEIFWKHIAPHFDVKLAIDDRTQVVEM WRRIGVECWQVASGDF |
| DMA | 80 | MLGAIAYTGNKQSLLPELKSHFPKYNRFVDLFCGGLSVSLN VNGPVLANDIQEPIIEMYKRLINVSWDDVLKVIKQYKLSKTS KEEFLKLREDYNKTRDPLLLYVLHFHGFSNMIRINDKGNFTT PFGKRTINKNSEKQYNHFKQNCDKIIFSSLHFKDVKILDGDF VYVDPPYLITVADYNKFWSEDEEKDLLNLLDSLNDRGIKFG QSNVLEHHGKENTLLKEWSKKYNVKHLNKKYVFNIYHSKE KNGTDEVYIFN |

### BRIEF DESCRIPTION OF THE DRAWINGS

**Figure 1** is a scheme showing the principle of strand displacement activity of Phi29 DNA polymerase, or related enzymes.
**Figures 2A-2C** is a set of photographs showing: (**Fig. 2A**) Amplification of pDNA in the presence of EcoRNAP, Phi29, rNTP and dNTP, leading to the obtention of concatemeric DNA; (**Fig. 2B**) Amplification of pDNA in the presence of either the core or the holoenzyme form of EcoRNAP. (**Fig. 2C**) Amplification of pDNA in the presence or absence of EcoRNAP, Phi29, rNTP mix, ATP + GTP, ATP alone or GTP alone.
**Figure 3** is a photograph showing: Amplification of pDNA in the presence of EcoRNAP, Phi29, RNAse H, PolI, rNTP and dNTP, leading to the obtention of concatemeric DNA. RNAse H was included in all conditions.
**Figures 4A-4B** is a set of photographs showing: (Fig. 3A) Amplification of pDNA in the presence of EcoRNAP, Phi29, rNTP and followed by digestion with a restriction enzyme. (Fig. 3B) "One pot" synthesis combining amplification and digestion reactions.
**Figure 5** is set of photographs and schemes showing amplification of 3 plasmids using Phi29 and EcoRNAP.
**Figures 6A-6B** is set of photographs showing: (**Fig. 6A**) Capillary electrophoresis analysis of a digested concatemer DNA compared to the corresponding linearized plasmid DNA. (**Fig. 6B**) Number of Illumina sequencing reads throughout the sequence of the digested concatemer DNA compared to the corresponding linearized plasmid DNA.
**Figures 7A-7D** is set of photographs and schemes showing: (**Fig. 7A**) Template pDNA used in for the experiment. The first pDNA is devoid of protelomerase site while the second pDNA harbors one protelomerase site (TelN); (**Fig. 7B**) Workflow of the experiment; and (**Fig. 7C-7D**) Agarose gel electrophoresis analysis of the amplified templates before and after TelN digestion.
**Figure 8** is a scheme showing the mechanism of Theta replication.
**Figure 9** is a scheme showing *E. coli* replisome.
**Figure 10** is a set of photographs showing the cloning of all the non-commercial but required components to reconstitute *E. coli* replisome.
**Figure 11** is a scheme showing T4 replisome.
**Figure 12A-12B** is a set of photographs showing: (**Fig. 12A**) SDS-PAGE analysis of T4 bacteriophage proteins expression using ready blue coloration and (**Fig. 12B**) SDS-PAGE analysis of T4 bacteriophage proteins expression using Lumio-tag green detection kit.
**Figure 13** is a photograph showing SDS-PAGE analysis of a selection of purified T4 bacteriophage proteins. Purification was performed using affinity chromatography.
**Figure 14** is a scheme showing T3 and T7 replisomes.
**Figure 15A-15B** is a set of photographs showing SDS-PAGE analysis of (**Fig**. **15A**) T7 bacteriophage proteins expression and (**Fig. 15B**) T7 gp2.5 and gp4 purification.
**Figure 16** is a photograph showing amplification of pDNA in the presence of EcoRNAP, T7 replication proteins, rNTP and dNTP. "1" corresponds to the control reaction that contains pDNA and EcoRNAP. "2" contains pDNA, EcoRNAP gp4, gp2.5, gp5, TopoIII and Gyrase. All reaction contains pDNA, EcoRNAP gp4, gp2.5, gp5, pol1, T7exo, T7ligase, RNAseH, TopoIII and Gyrase. For each condition, an RNAse A/T digestion was performed on the amplified product. Finally, products were heated in SDS-containing resolved on agarose gel.

### EXAMPLES

The present invention is further illustrated by the following examples.

### Example 1: Phi29 efficiently mimics replisomes

Most classical DNA production methods are based on random priming. Although these methods have been shown to efficiently amplify DNA, the lack of priming specificity increases the risk to obtain non-homogeneous and low-quality products.

To manufacture high quality DNA, it appears necessary to control the formation of the priming site. Although the use of OriC and dnaA have been previously demonstrated to be highly specific, it is worth noting that OriC-containing plasmid is not common and thus has a very narrow scope of applications. In contrast, the majority of plasmids used in biotechnology present a ColE1-like origin of replication that allows robust amplification in bacteria. Thus, targeting ColE1-like origin in combination with bacterial replisomes through a Theta mechanism is a very appealing strategy for *in vitro* DNA manufacturing.

Initiation of replication using ColE1-like origin has been previously shown to proceed through the formation of an R-loop, synthesized by EcoRNAP. Besides, to increase the specificity of such a mechanism, both RNaseH and Pol I have been shown to be involved in the processing of the R-loop to create an optimal substrate for replisome binding.

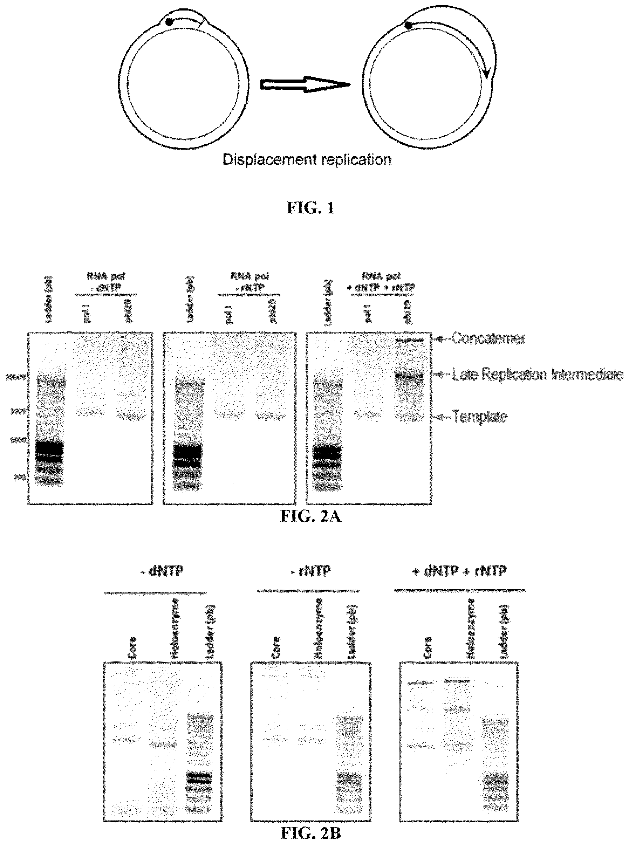

However, in the course of such a development, the possibility to specifically initiate DNA synthesis from plasmid origin using EcoRNAP followed by an amplification using non-bacterial replisomes was investigated. Although these replication enzymes are not supposed to naturally recognize the highly regulated R-loop made from the origin, their simplicity represents a profound industrial advantage over the use of bacterial replisomes. Indeed, bacterial replisomes are constituted by more than twenty polypeptides, which represents a challenge for productions at an industrial scale. In addition, to mimic replisomes, Phi29 DNA polymerase, was also tested. Indeed, this enzyme is endowed with a strand displacement activity that is similar to helicase and allows for strands separation during replication (**Figure 1**).

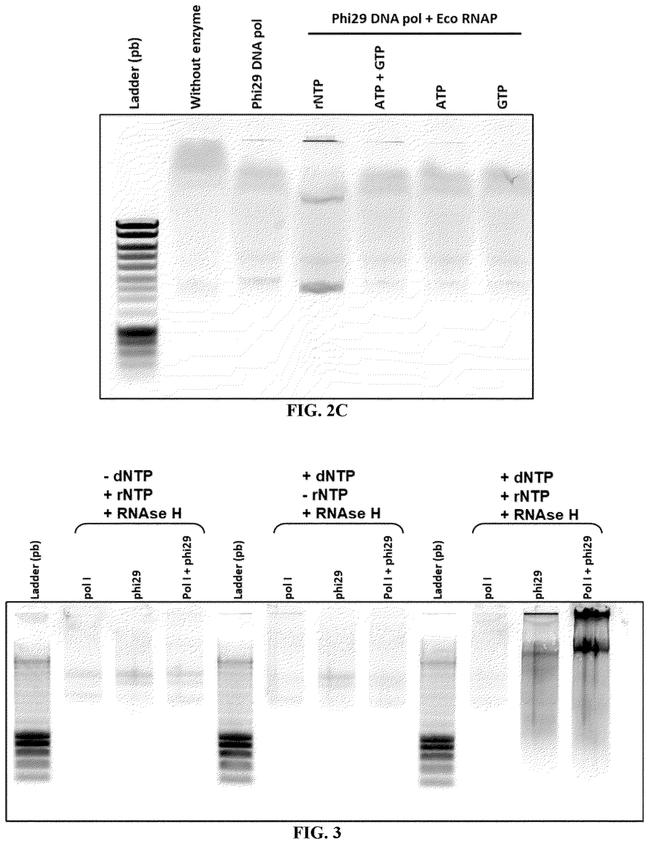

Surprisingly, Phi29 DNA polymerase was found performant at synthetizing DNA upon the addition of EcoRNAP. Indeed, in the presence of EcoRNAP, Phi29, rNTP and dNTP, the formation of a long concatemeric DNA was observed **(****Figure 2A**). Besides, this reaction was found to be highly specific since neither the core EcoRNAP alone (**Figure 2B**) nor the addition of only two rNTP could achieve similar levels of amplification (**Figure 2C**) Furthermore, the addition of both RNAse H and Pol I to the reaction mixture increased the amount of produced DNA, confirming that Phi29 amplification is specific to the R-loop formed by EcoRNAP at the origin of replication (**Figure 3**). However, as expected this process performs through a rolling circle amplification mode (RCA) instead of Theta. This leads to very large DNA concatemers superior to 70 kbp in length, which corresponds to several repetitions of the template in the same molecule.

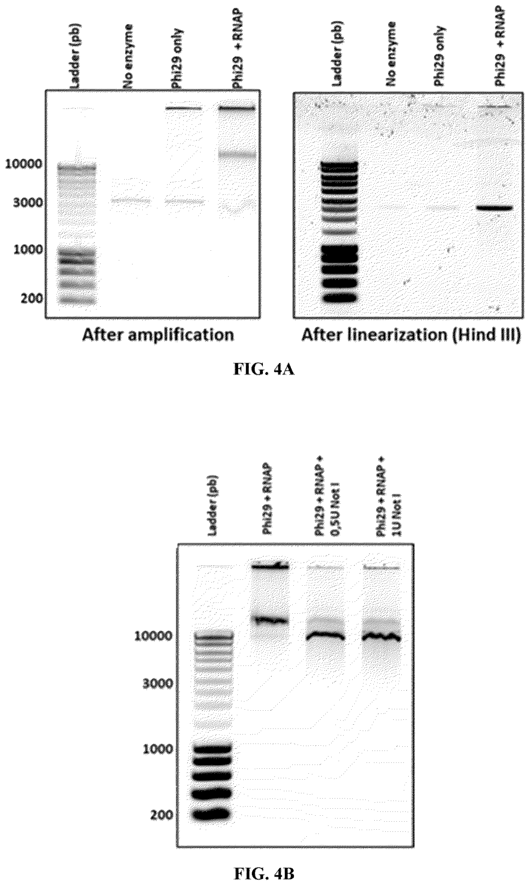

Nevertheless, such a concatemer can be easily turned into linear DNA (**Figure 4**) suitable for IVT reaction or into Linear Covalently Closed DNA (LCC DNA - **Figure X**) for AAV or CAR-T cells preparation.

Indeed, as shown in **Figure 4A****,** the concatemeric DNA obtain after amplification can be digested by a restriction enzyme to obtain linear DNA. Moreover, both amplification and digestion might be combined in a "one pot" synthesis, restriction enzymes being more active on linear DNA than on pDNA (**Figure 4B**).

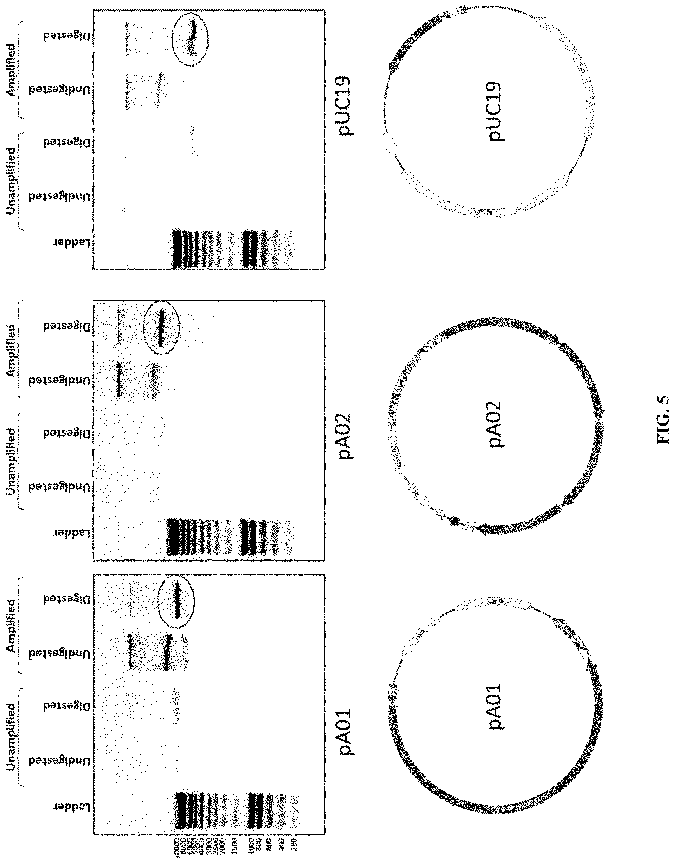

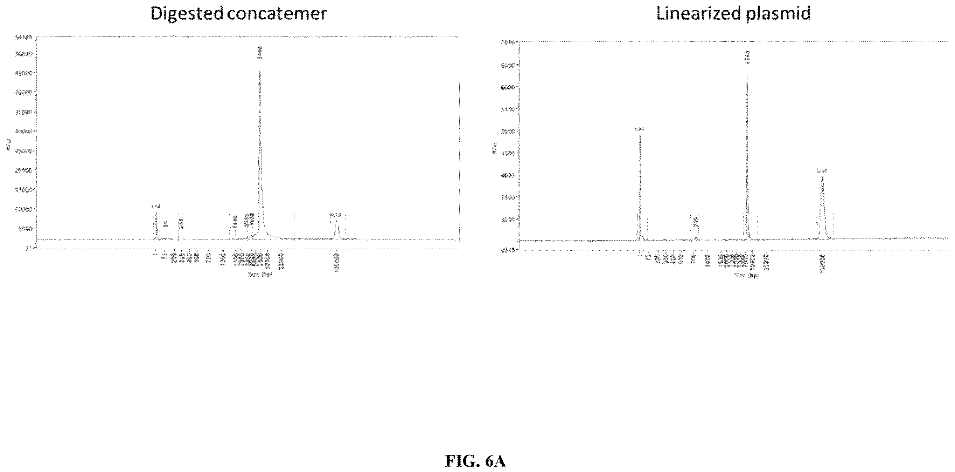

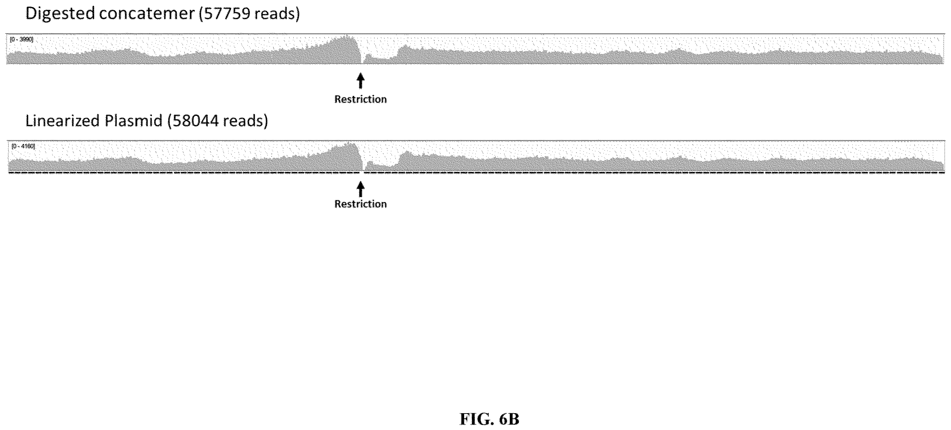

Amplification of various plasmids using this process is further illustrated on **Figure 5**, demonstrating the broad applicability of the process. Besides, analysis of the restriction digested concontemeric DNA shows a profile similar to the template pDNA (**Figure 6A**), especially regarding its sequence that is identical, as judged by Next generation sequencing (Illumina) presented in **Figure 6B****.**

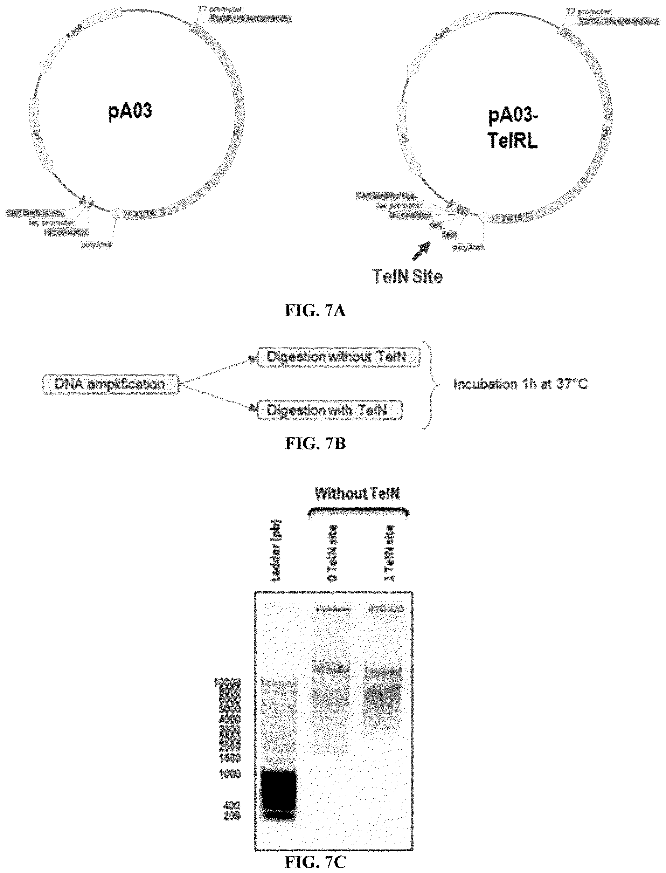

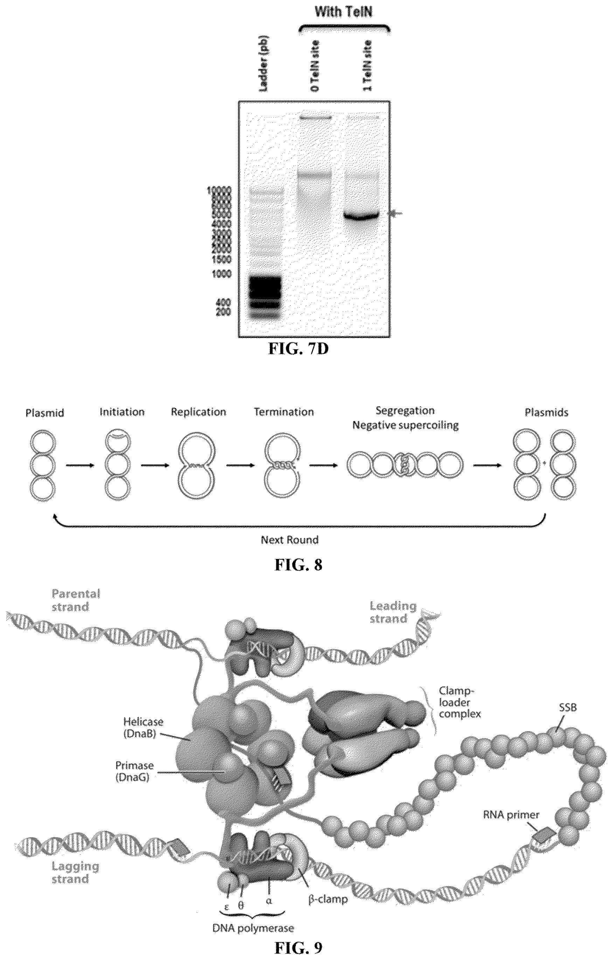

In a second round of experiments, the ability of the process to generate LCC DNA using two plasmids that harbor either one or no protelomerase site (TelN) was tested. Thus, as shown in **Figure 7**, while both templates can be amplified, only the pDNA that harbors a protelomerase site (TelN) can be digested, resulting in the obtention of LCC DNA that migrates at the expected size (4.8 kbp).

### Example 2: Theta replication process

Theta replication generates a structure that resembles the Greek letter theta (θ). In theta replication, double-stranded DNA begins to unwind at the replication origin, producing single-stranded DNA strands that then serve as templates on which new DNA can be synthesized. Replication it-self is carried out by a multi-enzymatic complex, named replisome. Thus, the unwinding of the double helix generates a replication bubble in which both strands are replicated simultaneously (Leading and lagging strands).

As the replication proceeds, a negative supercoiling is required to counteract the activity of the replisome. Finally, the action of some topoisomerase (topo III and / or Topo IV) segregates the two replicated plasmids. (**Figure 8**).

Replisome constitutions differ among organisms, some of which being very complex while some others are minimalistic. In all cases, replisomes are constituted by a DNA polymerase, a DNA helicase, an RNA primase, a sliding clamp, a DNA binding protein, and a DNA ligase.

The idea here is to associate the use of ColE1-like initiation with natural (*E. coli*) or artificial (T4 phage and T3/T7 phages) replisomes.

To secure the internalization of replisomes, three replisomes from different organisms were reconstituted:
- *E. coli* replisome,
- T4 phage replisome (less complex/simplified),
- T7 / T3 phages replisomes (minimalistic).

### E. coli replisome

The replisome of *E. coli* is the most complex and has an error rate of 10⁻⁸ to 10⁻⁹. For DNA replication (**Figure 9**), after initiation, the DnaB helicase unwinds the duplex DNA. The two separated parental strands are replicated by DNA polymerase III (Pol III) holoenzyme. The holoenzyme consists of a three-subunit catalytic core (α-polymerase, ε-exonuclease, and θ), a homodimeric β processivity clamp, and the δδ'τ2γχψ-clamp-loader complex. The τ-subunit differs from the γ-subunit in that it contains an extra 24-kDa C-terminal region, which couples the helicase and the polymerase by interacting with both the α-subunit and DnaB. DNA Pol III functions as a dimer where one core replicates the leading strand continuously and the second replicates the lagging strand in the form of Okazaki fragments. The clamp loader may consist of up to three τ-subunits, and therefore up to three polymerases can be assembled at the replication fork. Three monomers of the primase DnaG interact with DnaB and catalyze primer synthesis. The ssDNA is coated with SSB (ssDNA-binding) protein.

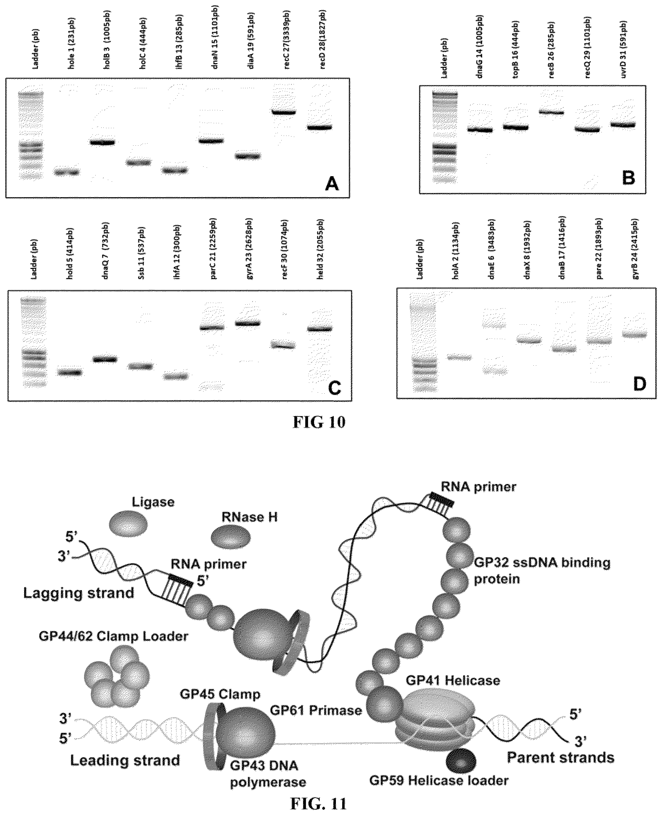

All the non-commercial but required components to reconstitute *E. coli* replisome were cloned (**Figure 10**), for iterative amplification process using EcoRNAP (ColE1-like origin).

### Simplified replisome based on T4 phage enzymes

T4 phage replisome is known to exhibit a very high fidelity of replication, with an error rate of 10⁻⁸. The replicase proteins include the gp43 DNA polymerase, responsible for leading and lagging strand synthesis, the gp45 clamp, the ring shaped processivity factor involved in polymerase fidelity, and gp44/62 clamp loader, an AAA + ATPase responsible for opening gp45 for placement and removal on duplex DNA. The primosomal proteins include the gp41 helicase, a hexameric 5' to 3' ATP dependent DNA helicase, the gp61 primase, a DNA dependent RNA polymerase responsible for synthesis of primers for lagging strand synthesis, the gp32 single stranded DNA binding protein, responsible for protection of single stranded DNA created by gp41 helicase activity, and the gp59 helicase loading protein, responsible for the loading of gp41 helicase onto gp32 protected ssDNA. Repair of Okazaki fragments is accomplished by the RNase H, a 5' to 3' exonuclease, and gp30 ligase, the ATP dependent DNA ligase.

Leading and lagging strand synthesis is coordinated by the replisome. Lagging strand primer extension and helicase progression lead to the formation of a loop of DNA extending from the replisome as proposed in the "trombone" model. (**Figure 11**).

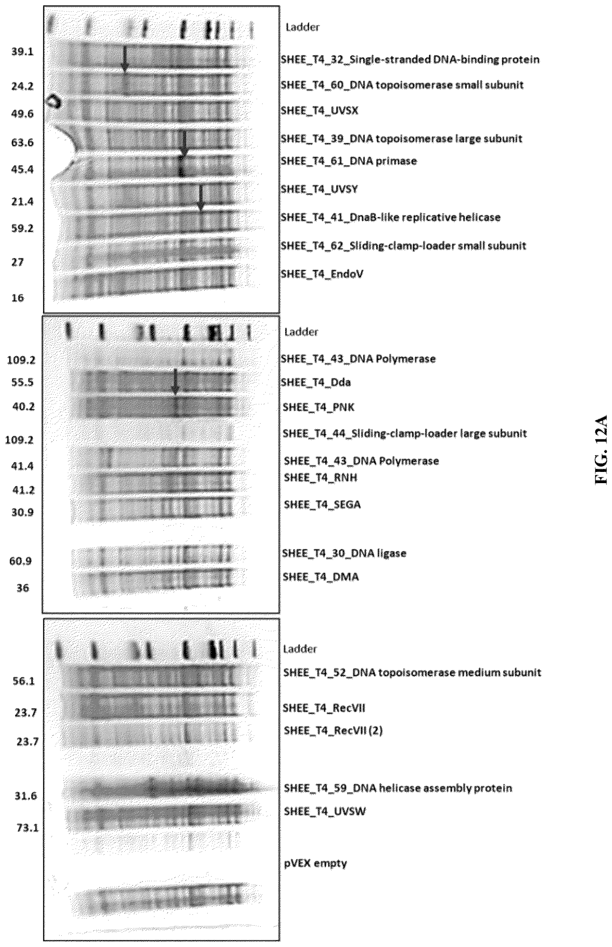

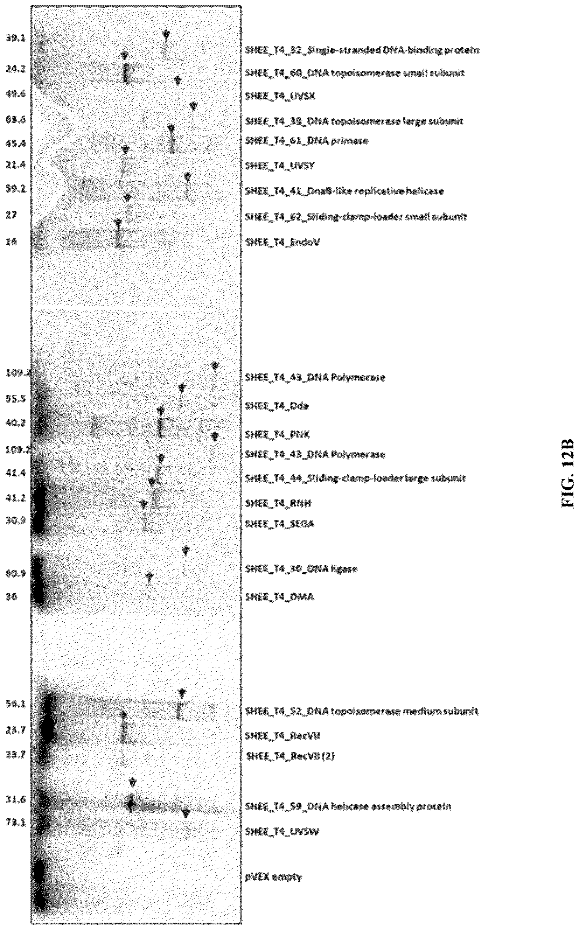

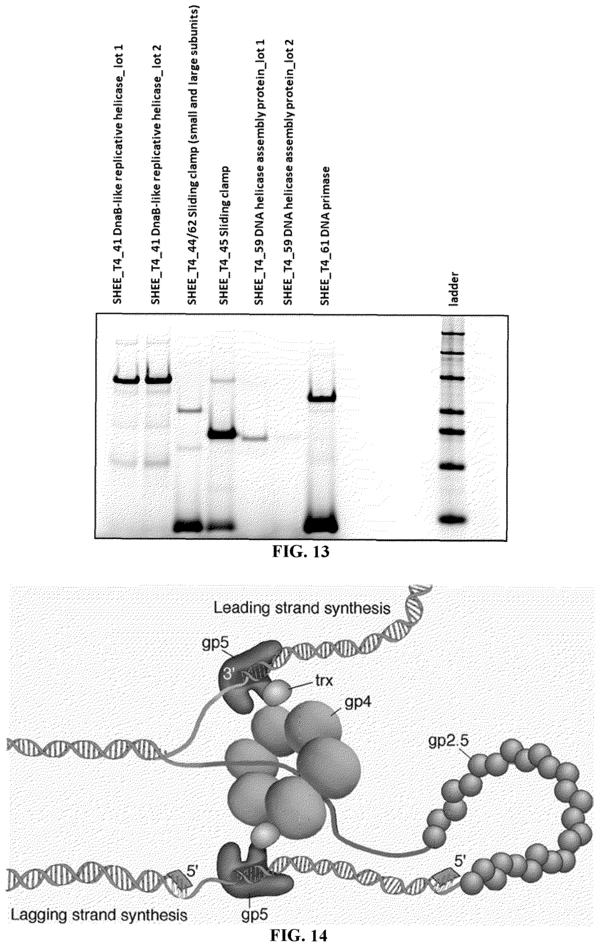

All required and accessory components to reconstitute T4 replisome have been expressed (**Figure 12A** and **Figure 12B**) and purified **(****Figure 13**), for iterative amplification process using EcoRNAP (ColE1-like origin) and T4 replisome.

### Minimal Replisome based on T3 / T7 phages enzymes.

Both T3 and T7 replisome are relatively simple and consists of only four different replication proteins: DNA polymerase (GP5), helicase-primase (GP4), ssDNA-binding protein (GP 2.5), and *E. coli* thioredoxin (trx). In vitro studies with purified T7 proteins indicate that T7 RNAP and T7 DNAP are essential for the initiation of DNA replication. Furthermore, DNA replication is stimulated in the presence of the T7 helicase-primase. (**Figure 14**).

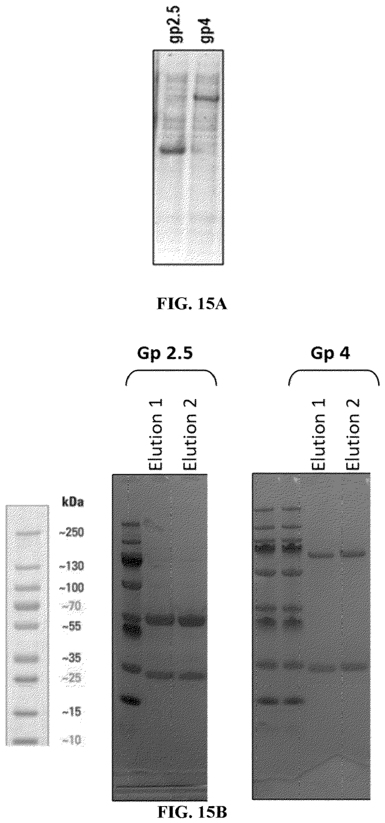

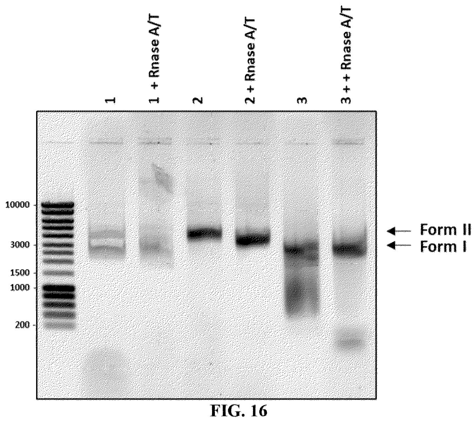

All required and accessory components to reconstitute T3/T7 replisome have been expressed and purified (**Figure 15**), for iterative amplification process using EcoRNAP (ColE1-like origin) and T3/T7 replisome. As shown in **Figure 16**, these enzymes were used in combination with EcoRNAP to perform replication of plasmid DNA in the presence of either the reconstituted replisome or in the presence of the complete system. Surprisingly, the addition of the reconstituted replisome was found performant at replicating DNA in the presence of EcoRNAP resulting in the obtention of DNA form II. Besides, upon the addition of DNA gyrase and Topoisomerase III, the complete system could produce a DNA that correspond to the supercoiled form I. To further assess the effect of RNA byproducts on the migration, all samples were digested with RNase A/T. Resulting products were found similar to the non-digested samples, confirming that DNA is the major product of reaction.

Such results were not expected since the T7 replication system is naturally endowed with its own RNA polymerase and is known to replicate from its own origin of replication. Thus, it appears that in vitro, the T7 replisome is able to associate within the ColE1-like origin to extend the R-loop formed by EcoRNAP.

## Claims

1. A method for amplifying a DNA molecule, comprising contacting said DNA molecule with a first composition comprising at least one bacterial RNA polymerase (RNAP) or a nucleic acid encoding thereof, and a second composition comprising a strand displacement DNA polymerase or a nucleic acid encoding thereof, wherein said DNA molecule comprises at least one ColE1-like origin of replication and wherein said at least one bacterial RNAP is configured to bind said at least one origin of replication.

2. The method according to claim 1, wherein said strand displacement DNA polymerase is a Phi29 DNA polymerase, and wherein optionally said Phi29 DNA polymerase has at least 75% sequence identity with SEQ ID NO: 6.

3. A method for amplifying a DNA molecule, comprising contacting said DNA molecule with a first composition comprising at least one bacterial RNA polymerase (RNAP) or a nucleic acid encoding thereof, and a second composition comprising at least one non-native replisome with respect to said at least one bacterial RNAP or nucleic acids encoding thereof, wherein said DNA molecule comprises at least one ColE1-like origin of replication and wherein said at least one bacterial RNAP is configured to bind said at least one origin of replication.

4. The method according to claim **3**, wherein said first composition further comprises one or more proteins selected from the group comprising DNA polymerase I having at least 75% sequence identity with SEQ ID NO: 16, *E. coli* Single-stranded DNA-binding protein having at least 75% sequence identity with SEQ ID NO: 39, *E. coli* RNase H having at least 75% sequence identity with SEQ ID NO: 56, and variants thereof, or nucleic acids encoding thereof.

5. The method according to claim **3** or **4**, wherein the non-native replisome is a phage T4-based replisome, a phage T3-based replisome, or a phage T7-based replisome.

6. The method according to any one of claims **3** to **5**, wherein the non-native replisome is a phage T4-based replisome comprising GP43 DNA polymerase having at least 75% sequence identity with SEQ ID NO: 19, and optionally one or more of the proteins selected from the group comprising gp41 Helicase having at least 75% sequence identity with SEQ ID NO: 25, gp59 Helicase loader having at least 75% sequence identity with SEQ ID NO: 26, *E. coli* DNA Gyrase, GP32 SSB having at least 75% sequence identity with SEQ ID NO: 40, gp61 Primase having at least 75% sequence identity with SEQ ID NO: 35, gp45 clamp having at least 75% sequence identity with SEQ ID NO: 36, gp44/62 Clamp loader having at least 75% sequence identity with SEQ ID NO: 37 or SEQ ID NO: 38, and variants thereof, or nucleic acids encoding thereof.

7. The method according to any one of claims **3** to **5**, wherein the non-native replisome is a phage T3-based replisome comprising GP5 DNA polymerase having at least 75% sequence identity with SEQ ID NO: 20, Thioredoxin 1 having at least 75% sequence identity with SEQ ID NO: 22, and optionally one or more of the proteins selected from the group comprising phage T3 DNA helicase/primase having at least 75% sequence identity with SEQ ID NO: 28, *E. coli* DNA Gyrase, and phage T3 Single-stranded DNA-binding protein having at least 75% sequence identity with SEQ ID NO: 42, and variants thereof, or nucleic acids encoding thereof.

8. The method according to any one of claims **3** to **5**, wherein the non-native replisome is a phage T7-based replisome comprising GP5 DNA polymerase having at least 75% sequence identity with SEQ ID NO: 21, Thioredoxin 1 having at least 75% sequence identity with SEQ ID NO: 22, and optionally one or more of the proteins selected from the group comprising phage T7 DNA helicase/primase having at least 75% sequence identity with SEQ ID NO: 27, *E. coli* DNA Gyrase, and phage T7 Single-stranded DNA-binding protein having at least 75% sequence identity with SEQ ID NO: 41, and variants thereof, or nucleic acids encoding thereof.

9. The method according to any one of claims **1** to **8**, wherein said DNA molecule is selected from the group comprising plasmid DNA (pDNA), linear DNA, minicircle DNA, and linear covalently closed DNA (LCC DNA), optionally wherein said DNA molecule is a pDNA or linear DNA.

10. The method according to any one of claims **1** to **9**, wherein amplification is initiated by the binding of said at least one RNAP to said at least one ColE1-like origin of replication.

11. The method according to any one of claims **1** to **10**, wherein said at least one bacterial RNA polymerase is *Escherichia coli* RNA polymerase holoenzyme.

12. The method according to any one of claims **1** to **11**, wherein the amplification is isothermal.

13. A composition comprising a phage T4-based replisome comprising GP43 DNA Polymerase, and one or more of the proteins selected from the group comprising or consisting of gp41 Helicase, gp59 Helicase loader, E. coli DNA Gyrase, GP32 SSB, gp61 Primase, gp45 clamp, gp44/62 Clamp loader, phage T4 RNaseH, phage T4 Ligase, E. coli DNA topoisomerase III, E. coli DNA topoisomerase IV, E. coli Protein DNA ligase, phage T4 DNA topoisomerase, phage T4 ATP-dependent DNA helicase Dda, phage T4 RecVII, phage T4 uvsW, phage T4 uvsX, phage T4 uvsY, phage T4 EndoV, phage T4 PNK, phage T4 SEGA, phage T4 DMA, E. coli DNA topoisomerase I, E. coli RpoE, E. coli RpoH, E. coli RpoS, E. coli ihfA, E. coli ihfB, E. coli diaA, E. coli RecA, E. coli RecBCD, E. coli RecQ, E. coli RecF, E. coli uvrD, and E. coli HelD, or nucleic acids encoding thereof.

14. A composition comprising a phage T3-based replisome comprising T3 GP5 and Thioredoxin 1, and one or more of the proteins selected from the group comprising or consisting of phage T3 DNA helicase/primase, *E. coli* DNA Gyrase, phage T3 Single-stranded DNA-binding protein, phage T3 DNA ligase, *E. coli* DNA topoisomerase III, *E. coli* DNA topoisomerase IV, T3 endonuclease, T3 exonuclease, *E. coli* DNA ligase, *E. coli* DNA topoisomerase I, *E. coli* RpoE, *E*. *coli* RpoH, *E. coli* RpoS, *E. coli* ihfA, *E. coli* ihfB, *E. coli* diaA, *E. coli* RecA, *E*. *coli* RecBCD, *E. coli* RecQ, *E. coli* RecF, *E. coli* uvrD, and *E. coli* HelD, or nucleic acids encoding thereof.

15. A composition comprising a phage T7-based replisome comprising T7 GP5 and Thioredoxin 1, and one or more of the proteins selected from the group comprising or consisting of phage T7 DNA helicase/primase, *E. coli* DNA Gyrase, phage T7 Single-stranded DNA-binding protein, phage T7 DNA ligase, *E. coli* DNA topoisomerase III, *E. coli* DNA topoisomerase IV, T7 endonuclease, T7 exonuclease, *E. coli* DNA ligase, *E. coli* DNA topoisomerase I, *E. coli* RpoE, *E*. *coli* RpoH, *E. coli* RpoS, *E. coli* ihfA, *E. coli* ihfB, *E. coli* diaA, *E. coli* RecA, *E*. *coli* RecBCD, *E. coli* RecQ, *E. coli* RecF, *E. coli* uvrD, and *E. coli* HelD, or nucleic acids encoding thereof.
